# EUROPEAN PATENT APPLICATION

(11) **EP 4 538 268 A1**
(43) Date of publication of application: **16.04.2025**
(21) Application number: 23819110.0
(22) Date of filing: 06.06.2023
(51) Int. Cl.: C07D 403/12, C07D 403/14, C07D 213/74, C07D 401/04, C07D 401/12, A61K 31/455, A61P 29/00

(54) **PYRIDAZIN-3-CARBOXAMIDE COMPOUND AS TYK2 INHIBITOR**

(30) Priority: 07.06.2022 CN 202210652678
(71) Applicant: Guangzhou Fermion Technology Co., Ltd., Guangzhou, Guangdong 510005 (CN)
(72) Inventor: DENG, Daiguo, Guangzhou, Guangdong 510005 (CN); ZHONG, Wenhe, Guangzhou, Guangdong 510005 (CN); LU, Jielian, Guangzhou, Guangdong 510005 (CN); LEI, Zengrong, Guangzhou, Guangdong 510005 (CN)
(74) Representative: Mewburn Ellis LLP
(86) International application number: PCT/CN2023/098582
(87) International publication number: WO 2023/236945

(57) **Abstract**

The present invention provides a pyridazine-3-carboxamide compound represented by general formula (I), or a pharmaceutically acceptable salt, enantiomer, diastereomer, racemate, solvate, hydrate, polymorph, prodrug or isotopic variant thereof. The present invention further provides a pharmaceutical composition including the compound, a preparation method therefor and a use thereof in treating or preventing a TYK2 kinase-mediated disease.

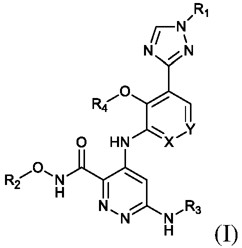

## Description

### FIELD OF THE INVENTION

The present invention relates to the field of medicinal chemistry, and in particular to a pyridazine-3-carboxamide compound, a composition containing the pyridazine-3-carboxamide compound, a preparation method therefor and a use thereof as a TYK2 inhibitor.

### BACKGROUND

The Janus kinase (JAK) family is made of intracellular non-receptor tyrosine kinases that mediate the signaling and activation of various cytokines. JAK gain-of-function expressions or mutations are associated with many autoimmune diseases, inflammation conditions and cancers. This family includes JAK1, JAK2, JAK3 and TYK2, among which JAK1, JAK2 and TYK2 are widely present in various tissues and cells of the human body, and JAK3 is mainly present in bone marrow cells, thymus cells, NK cells and activated B cells and T cells.

A JAK-mediated signaling pathway includes three key parts: a cytokine receptor on a cell surface, a JAK, and a downstream protein. Cytokines such as various interferons (IFNs) and interleukins (ILs) bind to the cytokine receptor on the cell surface, bringing into proximity the JAK that binds to an intracellular domain of the receptor. Then, a tyrosine residue of the JAK is phosphorylated, increasing the activity of the kinase domain. Next, the activated JAK phosphorylates a tyrosine residue of the receptor, creating a binding site for a protein having an SH2 domain. STAT (signal transducer and activator of transcription) binds to the phosphorylated tyrosine on the receptor through its SH2 domain, and is phosphorylated by the JAK and produces a phosphorylated STAT dimer, which then translocates to the nucleus to induce transcription of a target gene. In addition, other proteins having SH2 domains can also bind to the activated JAK, thereby cross-linking with other signaling pathways, such as PI3K/AKT, MAPK/ERK, etc.

TYK2 is a non-receptor tyrosine kinase that mediates immune signals. TYK2 mainly regulates signaling pathways driven by IL-23, IL-12 and type I interferon (IFNα), and is used as an IL-12, IL-23 and/or IFNa regulator by inhibiting TYK2-mediated signal transduction. TYK2 plays an important role in transmitting inflammatory and immune response signals, and is involved in pathophysiological processes of a variety of immune-related diseases, such as psoriasis (PS), rheumatoid arthritis (RA), systemic lupus erythematosus (SLE), inflammatory bowel disease (IBD), etc. TYK2 does not mediate cytokine responses driven by other kinases (such as IL-6, hematopoietic growth factor and IL-2). Therefore, TYK2 inhibitors can avoid adverse reactions associated with currently marketed JAK inhibitors by not acting on other subtypes.

Common small molecule JAK inhibitors are active site-directed inhibitors that bind to adenosine triphosphate (ATP) sites of the catalytic domains (JH1) of JAK proteins. Due to the high homology of the ATP sites of JAK family kinases and the similarity to the ATP-binding regions of the human kinase group, there generally exists an issue of low selectivity.

Studies have shown that the pseudokinase domain (JH2) in the JAK family, which exhibits significant catalytic activity, can provide an ideal allosteric site for the discovery of TYK2 selective inhibitors. Compound BMS-986165 is a currently known compound that can selectively bind to the JH2 of TYK2 and inhibit TYK2 kinase functions through an allosteric effect.

There is currently a need for drugs with improved activity in selectively inhibiting TYK2 by binding to JH2, thereby providing therapeutic benefits in the treatment of diseases.

### SUMMARY

In view of the foregoing, the present invention provides a pyridazine-3-carboxamide compound, which has excellent activity for selective inhibition of TYK2 and can treat a variety of TYK2-mediated diseases.

The present invention provides a compound represented by general formula (I), or a pharmaceutically acceptable salt, enantiomer, diastereomer, racemate, solvate, hydrate, polymorph, prodrug or isotopic variant thereof: 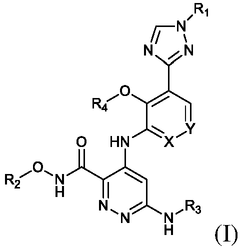 wherein each substituent is as defined in the present invention.

In one embodiment, the present invention provides a pharmaceutical composition comprising the compound, or the pharmaceutically acceptable salt, enantiomer, diastereomer, racemate, solvate, hydrate, polymorph, prodrug or isotopic variant thereof as defined herein, and a mixture thereof, and a pharmaceutically acceptable excipient; wherein preferably, the pharmaceutical composition further contains other therapeutic agents.

In one embodiment, the present invention provides a use of the compound or the pharmaceutically acceptable salt, enantiomer, diastereomer, racemate, solvate, hydrate, polymorph, prodrug or isotopic variant thereof as defined herein, and a mixture thereof, and a pharmaceutical composition comprising the same in the preparation of a medicament for treating and/or preventing a TYK2-mediated disease.

In one embodiment, the present invention provides a use of the compound or the pharmaceutically acceptable salt, enantiomer, diastereomer, racemate, solvate, hydrate, polymorph, prodrug or isotopic variant thereof as defined herein, and a mixture thereof, or a pharmaceutical composition comprising the compound in the treatment and/or prevention of a TYK2 kinase-mediated disease.

In one embodiment, the present invention provides a method for treating and/or preventing a TYK2 kinase-mediated disease in a subject by using the compound as defined herein, the method comprising administering to the subject the compound or the pharmaceutically acceptable salt, enantiomer, diastereomer, racemate, solvate, hydrate, polymorph, prodrug or isotopic variant thereof, and a mixture thereof, or a pharmaceutical composition comprising the same.

The TYK2 kinase-mediated disease recited in the present invention is selected from an autoimmune disease, a skin disease, an allergic disease, organ rejection, cancer, dry eye disease, myelofibrosis and polycythemia vera. Further, the autoimmune disease is lupus, multiple sclerosis, rheumatoid arthritis, juvenile arthritis, psoriasis, ulcerative colitis, Crohn's disease or autoimmune thyroid disease; the skin disease is psoriasis, a rash or atopic dermatitis; the allergic disease is asthma or rhinitis; the organ transplant rejection is allograft rejection or graft-versus-host disease; and the cancer is renal cancer, liver cancer, pancreatic cancer, gastric cancer, breast cancer, prostate cancer, head and neck cancer, thyroid cancer, lung cancer, glioblastoma, melanoma, lymphoma or leukemia.

In some embodiments, the TYK2 kinase-mediated disease relating to the method is selected from rheumatoid arthritis, psoriasis, ulcerative colitis and Crohn's disease.

### DETAILED DESCRIPTION

### Definitions

The compound, the preparation method therefor, and the use thereof of the present invention will be described in further detail below with reference to specific examples. The present invention may be implemented in many different forms and is not limited to the embodiments described herein. On the contrary, these embodiments are provided for the purpose of providing a thorough and complete understanding of the disclosure of the present invention.

Unless otherwise defined, all technical and scientific terms used herein have the same meanings as those commonly understood by a person skilled in the technical field of the present invention. The terms used herein in the description are for the purpose of describing specific examples only and are not intended to limit the present invention. As used herein, the term "and/or" means any one or more of the related listed items and a combination of all items.

The term "alkyl" refers to a saturated hydrocarbon containing a primary (normal) carbon atom, a secondary carbon atom, a tertiary carbon atom, a quaternary carbon atom or a combination thereof. The alkyl is preferably, for example, a C₁-C₆ alkyl, a C₁-C₅ alkyl, a C₁-C₄ alkyl and a C₁-C₃ alkyl. Using "C₁-C₃ alkyl" as an example, the term refers to an alkyl containing 1 to 3 carbon atoms, and each occurrence thereof can be independently a C₁ alkyl, a C₂ alkyl or a C₃ alkyl. Suitable examples include, but are not limited to: methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂).

"Alkenyl" is an alkyl as defined herein containing at least one carbon-carbon double bond. In one example, the alkenyl contains 2 to 20 carbon atoms, preferably 2 to 12 carbon atoms, more preferably 2 to 8 carbon atoms, still more preferably 2 to 6 carbon atoms. Non-limiting examples of alkenyls include substituted or unsubstituted vinyl, 2-propenyl, 3-butenyl, 2-butenyl, 4-pentenyl, 3-pentenyl, 2-hexenyl, 3-hexenyl, 2-heptenyl, 3-heptenyl, 4-heptenyl, 3-octenyl, 3-nonenyl or 4-decenyl, etc. When the alkenyl is substituted, there are preferably 1 to 5 substituents, and the substituents are independently selected from F, Cl, Br, I, =O, an alkyl, an alkenyl, an alkynyl, an alkoxy, a hydroxy, a nitro, a cyano and an amino.

"Alkynyl" is an alkyl as defined herein containing at least one carbon-carbon triple bond. In one example, the alkynyl contains 2 to 20 carbon atoms, preferably 2 to 12 carbon atoms, more preferably 2 to 8 carbon atoms, still more preferably 2 to 6 carbon atoms. Non-limiting examples of alkynyls include substituted or unsubstituted ethynyl, 1-propynyl, 2-propynyl, 1-butynyl, 2-butynyl, 3-butynyl, 4-pentynyl, 3-pentynyl, 2-hexynyl, 3-hexynyl, 3-butynyl, 2-heptynyl, 3-heptynyl, 4-heptynyl, 3-octynyl, 3-heptynyl or 4-decynyl, etc. When the alkynyl is substituted, there are preferably 1 to 5 substituents, and the substituents are independently selected from F, Cl, Br, I, =O, an alkyl, an alkenyl, an alkynyl, an alkoxy, a hydroxy, a nitro, a cyano and an amino.

"Carbocyclyl" or "cycloalkyl" refers to a saturated or partially unsaturated cyclic carbon-containing group, such as a 5- to 6-membered saturated carbocyclic ring and a 5- to 6-membered partially unsaturated carbocyclic ring. In one embodiment, the carbocyclyl is a 3- to 4-membered monocyclic, a 3- to 5-membered monocyclic, a 3- to 6-membered monocyclic, a 3- to 7-membered monocyclic, a 3- to 8-membered monocyclic, a 3- to 10-membered monocyclic, a 5- to 8-membered monocyclic, a 5- to 6-membered monocyclic, a 4- to 12-membered bicyclic or a 10- to 15-membered tricyclic ring system. The carbocycle includes bridged or spiro rings. Non-limiting examples of carbocyclyls include cyclopropyl, cyclobutyl, cyclopentyl, cyclohexyl, cyclohexenyl, cycloheptyl, cyclopentenyl, cyclohexadienyl, cycloheptatrienyl, benzocyclopentyl, bicyclo[3.2.1]octyl, bicyclo[5.2.0]nonyl, tricyclo[5.3.1.1]dodecyl, adamantyl or spiro[3.3]heptyl, etc. The carbocyclyl may be optionally substituted. When the carbocyclyl is substituted, there are preferably 1 to 5 substituents, and the substituents are independently selected from F, Cl, Br, I, =O, an alkyl, an alkenyl, an alkynyl, an alkoxy, a hydroxy, a nitro, a cyano and an amino.

The term "halogen" means -F, -Cl,-Br or -I. Further, the term "haloalkyl" refers to an alkyl substituted with a halogen group, wherein the alkyl is as defined above, preferably a C₁₋₆ haloalkyl, a C₁₋₅ haloalkyl, a C₁₋₄ haloalkyl, a C₁₋₃ haloalkyl and a C₁₋₂ haloalkyl.

The term "aryl" refers to an aromatic hydrocarbon group derived from an aromatic ring compound by removing a hydrogen atom, and may be a monocyclic aryl, a condensed-ring aryl, or a polycyclic aryl, and is preferably a 6- to 10-membered aryl. For polycyclic ring species, at least one is an aromatic ring system. Phrases containing this term, such as "5- to 6-membered aryl", means that the aromatic ring system includes 5 to 6 ring atoms. Preferably, the aryl is phenyl.

The term "heteroaryl" refers to an aryl containing a heteroatom, which may be a monocyclic or condensed ring, wherein the heteroatom is independently selected from N, O and S, and the heteroaryl is preferably a 5- to 12-membered heteroaryl, a 5- to 10-membered heteroaryl, preferably a 5- to 8-membered heteroaryl, more preferably a 5- to 6-membered heteroaryl, still more preferably a 5-membered heteroaryl. The heteroaryl includes, but is not limited to, pyrrolyl, furanyl, thienyl, imidazolyl, oxazolyl, isoxazolyl, pyrazolyl, pyridinyl, pyrimidinyl, pyrazinyl, pyridazinyl, quinolyl, isoquinolyl, triazolyl, tetrahydropyrrolyl and thiadiazolyl. In one embodiment, provided is a 5- to 6-membered monocyclic heteroaryl, typically containing one or more, preferably one to three, heteroatoms independently selected from N, O and S. Unless otherwise specified, the "5-membered heteroaryl" is as follows: exemplary 5-membered heteroaryl groups containing one heteroatom include, but are not limited to, pyrrolyl, furanyl and thienyl; exemplary 5-membered heteroaryl groups containing two heteroatoms include, but are not limited to, imidazolyl, pyrazolyl, oxazolinyl, isoxazolinyl, thiazolyl and isothiazolyl; exemplary 5-membered heteroaryl groups containing three heteroatoms include, but are not limited to, thiazolyl, oxadiazolyl and thiadiazolyl; exemplary 5-membered heteroaryl groups containing four heteroatoms include, but are not limited to, tetrazolyl.

"Heterocyclyl" or "heterocycle" refers to a substituted or unsubstituted saturated or partially unsaturated cyclic group containing a heteroatom selected from N, O and S. Furthermore, the term "heterocyclyl" refers to a group having a stable 3- to 10-membered saturated heterocyclic ring system in which one or more atoms constituting a non-aromatic ring are heteroatoms and the rest are carbon. The heteroatoms include, but are not limited to, nitrogen atoms, oxygen atoms, sulfur atoms, etc. The heterocyclyl may be a 3- to 7-membered monocyclic, a 5- to 8-membered monocyclic, a 5- to 6-membered monocyclic, a 4- to 12-membered bicyclic or a 10- to 15-membered tricyclic ring system, preferably a 3- to 10-membered heterocyclyl, and includes at least one, preferably one to four heteroatoms selected from N, O or S. Unless otherwise indicated in the present specification, a heterocycloalkyl group may be a monocyclic system ("monocyclic heterocycloalkyl"), or may be a bicyclic system, a tricyclic system or a polycyclic system with more rings, which may include a fused (condensed), bridged (bridged-ring) or spiro ring system (e.g., a bicyclic system ("bicyclic heterocycloalkyl"). The ring system of the bicyclic heterocycloalkyl may include one or more heteroatoms in one or both rings, and is saturated. Exemplary 3-membered heterocyclyl groups include, but are not limited to, aziridinyl, oxiranyl and thiiranyl, or stereoisomers thereof. Exemplary 4-membered heterocyclyl groups include, but are not limited to, azetidinyl, oxetanyl, thietanyl or isomers and stereoisomers thereof. Exemplary 5-membered heterocyclyl groups include, but are not limited to, tetrahydrofuranyl, tetrahydrothiophenyl, pyrrolidinyl, thiazolidinyl, isothiazolidinyl, oxazolidinyl, isoxazolidinyl, imidazolidinyl, pyrazolidinyl, dioxolanyl, oxathiolanyl, dithiolanyl or isomers and stereoisomers thereof. Exemplary 6-membered heterocyclyl groups include, but are not limited to, piperidinyl, tetrahydropyranyl, thioxanyl, morpholinyl, thiomorpholinyl, dithianyl, dioxanyl, piperazinyl, triazinyl or isomers and stereoisomers thereof. Exemplary 7-membered heterocyclyl groups include, but are not limited to, azepanyl, oxepanyl, thiepanyl and diazepanyl, or isomers and stereoisomers thereof. In one embodiment, a typical heterocyclyl is a 5- to 6-membered monocyclic heterocyclyl containing one or more, preferably one to four, more preferably one to three heteroatoms independently selected from N, O and S. In one embodiment, "heterocycloalkyl" is a 4- to 6-membered heterocycloalkyl, wherein heteroatoms are selected from one or more of N, O and S, and one, two or three heteroatoms are provided.

In various sections of the present invention, linking substituents are described. When the structure clearly requires a linking group, Markush variables listed for that group should be understood as linking groups. For example, if the structure requires a linking group and a Markush group definition for that variable lists "alkyl" or "aryl", it is understood that the "alkyl" or "aryl" represents a linking alkylene group or arylene group, respectively. In some specific structures, when an alkyl group is clearly indicated as a linking group, the alkyl group represents a linked alkylene group. For example, the alkyl in the group "-C₁-C₃ haloalkyl" should be understood as an alkylene.

The term "pharmaceutically acceptable salt" means that the compound can be converted into a corresponding salt by conventional methods, the salt being chemically or physically compatible with other ingredients constituting a pharmaceutical dosage form and physiologically compatible with a receptor. The salt may be an acidic and/or basic salt formed from the compound and an inorganic and/or organic acid and/or an inorganic and/or organic base, and also includes a zwitterionic salt (inner salt), and further includes a quaternary ammonium salt, such as an alkylammonium salt. These salts can be directly obtained during final isolation and purification of the compound. The salts can also be obtained by appropriately mixing the compound of the present invention or a stereoisomer or solvate thereof with a certain amount of an acid or a base. These salts may be precipitated from a solution and collected by filtration, or recovered after evaporation of a solvent, or may be obtained by reaction in an aqueous medium followed by cooling and drying. In particular, the salts are preferably water-soluble, pharmaceutically acceptable, non-toxic acid addition salts, examples of which are salts formed from an amino and an inorganic acid (such as hydrochloric acid, hydrobromic acid, phosphoric acid, sulfuric acid and perchloric acid) or an organic acid (such as acetic acid, oxalic acid, maleic acid, tartaric acid, citric acid, succinic acid or malonic acid), or salts formed using other conventional methods in the art (such as ion exchange methods). Other pharmaceutically acceptable salts include adipate, alginate, ascorbate, aspartate, benzene sulfonate, benzoate, bisulfate, borate, butyrate, camphorate, camphorsulfonate, citrate, cyclopentyl propionate, diglucosate, dodecyl sulfate, ethanesulfonate, formate, fumarate, gluconate, glycerophosphate, gluconate, hemisulfate, heptanate, hexanoate, hydroiodate, 2-hydroxy-ethanesulfonate, lactoate, lactate, laurate, lauryl sulfate, malate, maleate, malonate, methanesulfonate, 2-naphthalenesulfonate, niacin, nitrate, oleate, oxalate, palmitate, dihydroxynaphthalenate, pectinate, persulfate, 3-phenylpropionate, phosphate, picrate, pivalate, propionate, stearate, succinate, sulfate, tartrate, thiocyanate, p-toluenesulfonate, undecanoate, valerate, etc. Additional pharmaceutically acceptable salts may further include, where appropriate, salts derived from suitable bases, including alkali metal salts, alkaline earth metal salts and ammonium salts. Representative alkali metal or alkaline earth metal salts include a sodium salt, a lithium salt, a potassium salt, a calcium salt, a magnesium salt, etc. Additional pharmaceutically acceptable salts include, where appropriate, salts formed using counterions such as halides, hydroxide, carboxylate, sulfate, phosphate, nitrate, lower alkyl sulfonates and aryl sulfonates with non-toxic ammonium, quaternary ammonium and amine cations.

The term "solvate" may also be called "solvate compound" or "solvate", and refers to a compound containing solvent molecules, where the solvent molecules can bind to the compound molecules in ways including coordination bonds, covalent bonds, van der Waals forces, ionic bonds, hydrogen bonds, etc. Common solvents include water, methanol, ethanol, acetic acid, DMSO, THF, diethyl ether, etc. The compounds described herein can be prepared, e.g., in crystalline forms, and can be solvated. Suitable solvates include pharmaceutically acceptable solvates and further include stoichiometric and non-stoichiometric solvates. In some cases, the solvate will be capable of being isolated, for example, when one or more solvent molecules are incorporated into crystal lattices of a crystalline solid. The "solvate" includes solvates in solution and isolatable solvates. Representative solvates include hydrates, ethanolates and methanolates.

The term "hydrate" refers to a compound binding to water. Usually, the ratio of the number of water molecules contained in a hydrate of a compound to the number of molecules of the compound in the hydrate is determinate. Thus, a hydrate of a compound can be represented by, for example, the general formula R·x·H₂O, where R is the compound, and x is a number greater than zero. A given compound may form more than one type of hydrate, including, for example, a monohydrate (x is 1), lower hydrates (x is a number greater than 0 and less than 1, for example, a hemihydrate (R·0.5·H₂O)) and polyhydrates (x is a number greater than 1, for example, a dihydrate (R·2·H₂O) and a hexahydrate (R·6·H₂O)).

The term "prodrug" refers to any compound that, when administered to an organism, produces a drug, i.e., an active ingredient, as a result of a spontaneous chemical reaction, an enzyme-catalyzed chemical reaction, photolysis and/or a metabolic chemical reaction. Prodrugs are thus covalently modified analogs or latent forms of therapeutically active compounds. Suitable examples include, but are not limited to: carboxylates, carbonates, phosphates, nitrates, sulfates, sulfones, sulfoxides, amides, carbamates, azo compounds, phosphoramides, glucosides, ethers, acetals and other forms of the compound.

The present invention further includes isotopically labeled compounds (isotopic variants), which are equivalent to those general formula or specific compounds described herein except that one or more atoms are replaced by atoms having an atomic mass or mass number different from the atomic mass or mass number usually found in nature. Examples of isotopes that can be introduced into the compounds of the present invention include isotopes of hydrogen, carbon, nitrogen, oxygen, phosphorus, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively, preferably ²H (i.e., deuterium, D). The compounds of the present invention which contain the aforementioned isotopes and/or other isotopes of other atoms, prodrugs of the compounds, and pharmaceutically acceptable salts of the compounds or the prodrugs are all within the scope of the present invention. Certain isotopically labeled compounds of the present invention, such as those into which radioactive isotopes such ³H and ¹⁴C are incorporated, are useful in drug and/or substrate tissue distribution assays. Tritium (i.e., ³H) and carbon-14 (i.e., ¹⁴C) isotopes are particularly preferred for the ease of preparation and detectability. Additionally, substitution with heavier isotopes such as deuterium (i.e., ²H) may be preferred in some cases since substituted compounds may provide therapeutic advantages by virtue of greater metabolic stability, such as prolonged in-vivo half-life or reduced dosage requirements. Isotopically labeled compounds of the present invention and prodrugs thereof can generally be prepared by substituting readily available isotopically labeled reagents for non-isotopically labeled reagents when carrying out the processes disclosed in the following procedures and/or examples and preparative examples.

The compound of the present invention includes one or more asymmetric centers, and thus may have a plurality of stereoisomer forms, e.g., enantiomer and/or diastereomer forms. For example, the compound of the present invention may be a separate enantiomer, diastereomer or geometric isomer (e.g., *cis-* and *trans-isomers*), or may be in the form of a mixture of stereoisomers, including a mixture of racemates and a mixture rich in one or more stereoisomers. Isomers can be separated from the mixture by methods known to those skilled in the art, including: chiral high pressure liquid chromatography (HPLC) and formation and crystallization of chiral salts. Alternatively, preferred isomers may be prepared by asymmetric synthesis.

"Optional" or "optionally" means that a subsequently described event or circumstance may, but not necessarily, occur, including cases where the event or circumstance does or does not occur. For example, "an aryl is optionally substituted with an alkyl" means that the alkyl may, but not necessarily, be present, and the term includes instances where the aryl is substituted with an alkyl and instances where the aryl is not substituted with an alkyl.

"Pharmaceutically acceptable excipient" refers to a pharmaceutically acceptable material, composition or agent, such as a liquid or solid filler, a diluent, an excipient, a solvent or an encapsulating material. As used herein, the term "pharmaceutically acceptable excipient" includes buffers, sterile water for injection, solvents, dispersion media, coatings, antibacterial and antifungal agents, isotonicity agents, absorption retarders, etc., compatible with pharmaceutical administration. Each excipient needs to be "pharmaceutically acceptable" in the sense of being compatible with other ingredients in a formulation and not harmful to the patient. Suitable examples include, but are not limited to: (1) sugars, such as lactose, glucose and sucrose; (2) starches, such as corn starch, potato starch and substituted or unsubstituted β-cyclodextrin; (3) cellulose and derivatives thereof, such as sodium carboxymethyl cellulose, ethyl cellulose and cellulose acetate; (4) powdered tragacanth gum; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerol, sorbitol, mannitol and polyethylene glycol; (12) esters, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer's solution; (19) ethanol; (20) phosphate buffers; and (21) other non-toxic compatible substances used in drug formulations.

The term "polymorph" refers to a crystalline form of a compound (or a salt, hydrate or solvate thereof) of a particular crystal packing arrangement. All polymorphs have the same elemental composition. Different crystalline forms typically have different X-ray diffraction patterns, infrared spectra, melting points, densities, hardness, crystal shapes, optoelectronic properties, stability and solubility. Recrystallization solvent, rate of crystallization, storage temperature and other factors can cause one crystalline form to dominate. Various polymorphs of a compound can be prepared by crystallization under different conditions.

Unless defined otherwise, all technical and scientific terms used herein have the standard meanings in the art to which the claimed subject matter belongs. If there are multiple definitions for a term, the definition in this document shall prevail. It should be understood that the singular forms used in the present invention, such as "a" and "an", include plural references, unless otherwise specified.

In addition, the terms "comprise" and "include" are open-ended limitations rather than closed-ended ones, that is, they include the content specified in the present invention but do not exclude other aspects.

Unless otherwise specified, the present invention uses conventional methods such as mass spectrometry and nuclear magnetic resonance to identify compounds, and for steps and conditions, reference can be made to conventional operating steps and conditions in the art.

Unless otherwise indicated, the present invention uses standard nomenclature and standard laboratory procedures and techniques of analytical chemistry, synthetic organic chemistry and optics. In some cases, standard techniques are used for chemical synthesis, chemical analysis and light-emitting device performance testing.

In addition, it should be noted that, unless otherwise clearly stated, the description "... respectively independently" used in the present invention should be understood in a broad sense, meaning that each entity described is independent of each other and can independently be the same specific group or different specific groups. In more detail, the description "... respectively independently" may mean that specific options expressed by the same symbol do not affect each other among different groups, or may mean that specific options expressed by the same symbols do not affect each other in the same group.

Specifically, the present invention relates to the following technical solutions.

In one embodiment, the present invention relates to a compound represented by general formula (I), or a pharmaceutically acceptable salt, enantiomer, diastereomer, racemate, solvate, hydrate, polymorph, prodrug or isotopic variant thereof: 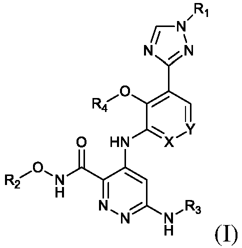 wherein
X is CR' or N;
Y is CR';
R₁ is selected from a C₁₋₆ alkyl, a C₂₋₆ alkenyl, a C₂₋₆ alkynyl, a C₃₋₇ cycloalkyl, a 3- to 7-membered heterocyclyl, a C₆₋₁₀ aryl or a 5- to 10-membered heteroaryl, and the group is optionally substituted with one or more deuteriums until fully deuterated;
R₂ is selected from a C₁₋₆ alkyl, a C₁₋₆ haloalkyl, a C₂₋₆ alkenyl, a C₂₋₆ alkynyl, a C₃₋₇ cycloalkyl, a 3- to 7-membered heterocyclyl, a C₆₋₁₀ aryl or a 5- to 10-membered heteroaryl, and the group is optionally substituted with one or more deuteriums until fully deuterated;
R₃ is selected from an -L-C₁₋₆ alkyl, an -L-C₁₋₆ haloalkyl, an -L-C₃₋₇ cycloalkyl, an -L-3-to 7-membered heterocyclyl, an -L-C₆₋₁₀ aryl or an -L-5- to 10-membered heteroaryl, and the group is optionally substituted with one or more deuteriums until fully deuterated;
R₄ is selected from H, a C₁₋₆ alkyl or a C₁₋₆ haloalkyl, and the group is optionally substituted with one or more deuteriums until fully deuterated;
each group of R1, R2, R3 and R4 is optionally substituted with 1, 2, 3 or 4 R;
wherein L is selected from a bond, -C(O)- or -C(S)-;
R is selected from H, D, a halogen, CN, a C₁₋₆ alkyl or a C₁₋₆ haloalkyl; and
R' is selected from H, D, a halogen or CN.

In a more specific embodiment, the present invention provides the compound represented by formula (I) above or the pharmaceutically acceptable salt, enantiomer, diastereomer, racemate, solvate, hydrate, polymorph, prodrug or isotopic variant thereof, wherein
X is CR' or N;
Y is CR';
R₁ is selected from a C₁₋₆ alkyl, a C₃₋₇ cycloalkyl or a 3- to 7-membered heterocyclyl, and the group is optionally substituted with one or more deuteriums until fully deuterated;
R₂ is selected from a C₁₋₆ alkyl, a C₁₋₆ haloalkyl, a C₃₋₇ cycloalkyl or a 3- to 7-membered heterocyclyl, and the group is optionally substituted with one or more deuteriums until fully deuterated;
R₃ is selected from an -L-C₃₋₇ cycloalkyl, an -L-3- to 7-membered heterocyclyl, a C₆₋₁₀ aryl or a 5- to 6-membered heteroaryl, and the group is optionally substituted with one or more deuteriums until fully deuterated;
R4 is selected from H, a C₁₋₆ alkyl or a C₁₋₆ haloalkyl, and the group is optionally substituted with one or more deuteriums until fully deuterated;
each group of R1, R2, R3 and R4 is optionally substituted with 1, 2, 3 or 4 R;
wherein L is selected from -C(O)- or -C(S)-;
R is selected from H, D, a halogen or CN; and
R' is selected from H, D, a halogen or CN.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, wherein X is CR', preferably CH.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, wherein Y is CH or CF.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, wherein R₁ is selected from a C₁₋₆ alkyl, a C₂₋₆ alkenyl, a C₂₋₆ alkynyl, a C₃₋₇ cycloalkyl or a 3- to 7-membered heterocyclyl, and the group is optionally substituted with one or more deuteriums until fully deuterated. Preferably, R₁ is selected from a C₁₋₆ alkyl or a C₃₋₇ cycloalkyl, and the group is optionally substituted by one or more deuteriums until fully deuterated. Preferably, R₁ is a C₁₋₆ alkyl, which is optionally substituted by one or more deuteriums until fully deuterated. Preferably, R₁ is a C₃₋₇ cycloalkyl.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, wherein R₂ is selected from a C₁₋₆ alkyl, a C₁₋₆ haloalkyl, a C₂₋₆ alkenyl, a C₂₋₆ alkynyl, a C₃₋₇ cycloalkyl or a 3- to 7-membered heterocyclyl, and the group is optionally substituted with one or more deuteriums until fully deuterated. Preferably, R₂ is selected from a C₁₋₆ alkyl, a C₁₋₆ haloalkyl or a C₃₋₇ cycloalkyl, and the group is optionally substituted with one or more deuteriums until fully deuterated. Preferably, R₂ is a C₁₋₆ alkyl, which is optionally substituted by one or more deuteriums until fully deuterated.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, wherein R₃ is selected from a-C(O)-C₃₋₇ cycloalkyl or a 5- to 6-membered heteroaryl, and the group is optionally substituted with one or more deuteriums until fully deuterated. Preferably, R₃ is a -C(O)-C₃₋₇ cycloalkyl.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, wherein R₄ is selected from a C₁₋₆ alkyl or a C₁₋₆ haloalkyl, and the group is optionally substituted with one or more deuteriums until fully deuterated. Preferably, R₄ is a C₁₋₆ alkyl, which is optionally substituted by one or more deuteriums until fully deuterated.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, which has the following general formula: 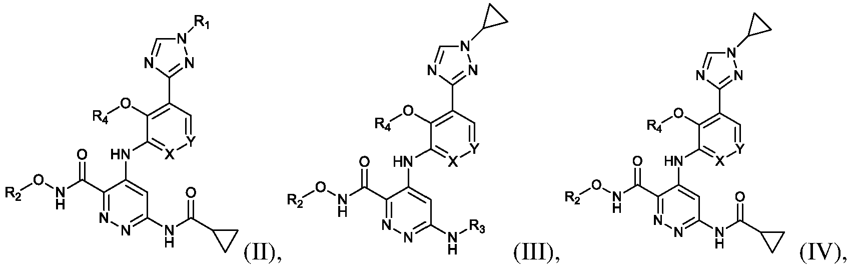 wherein each group is as defined in the present application.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, wherein the compound is selected from the following:

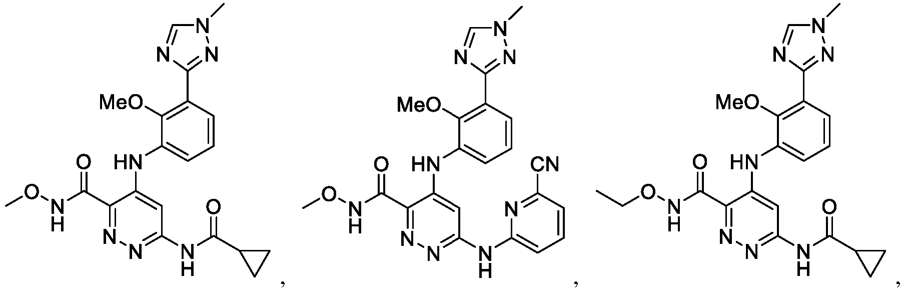

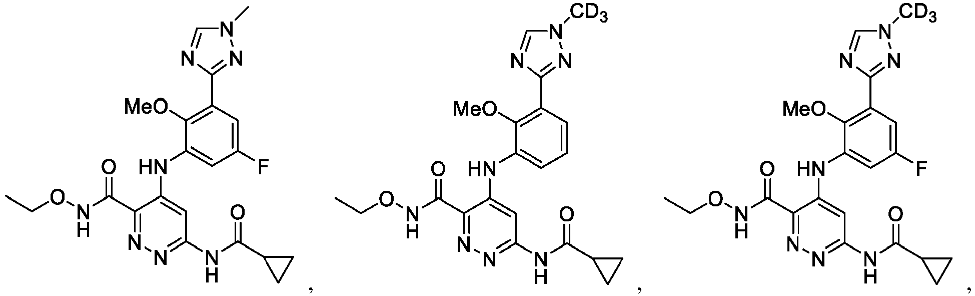

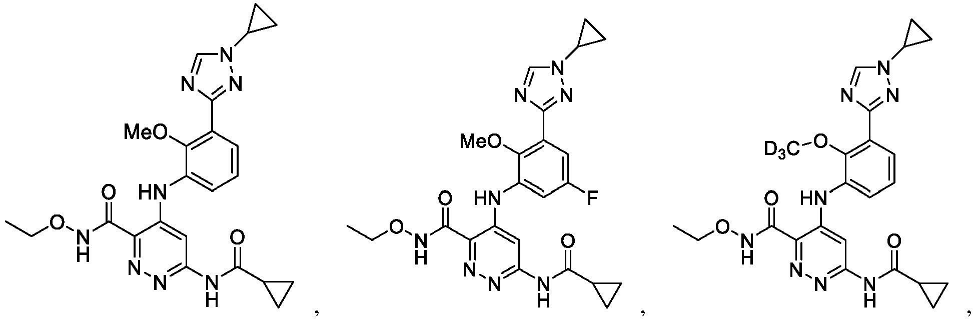

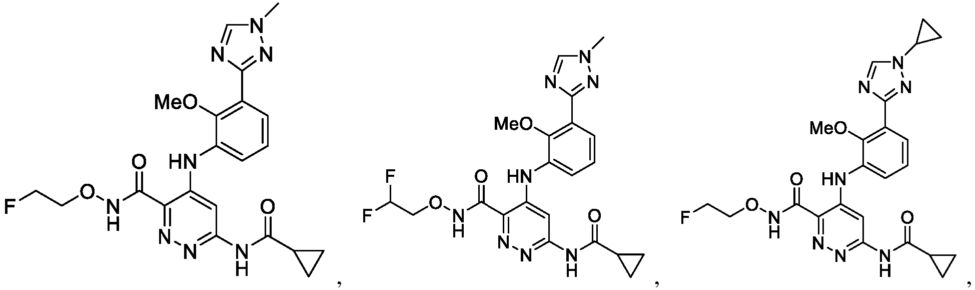

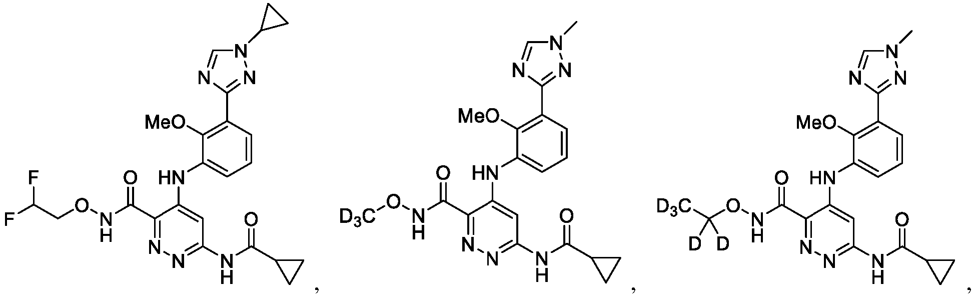

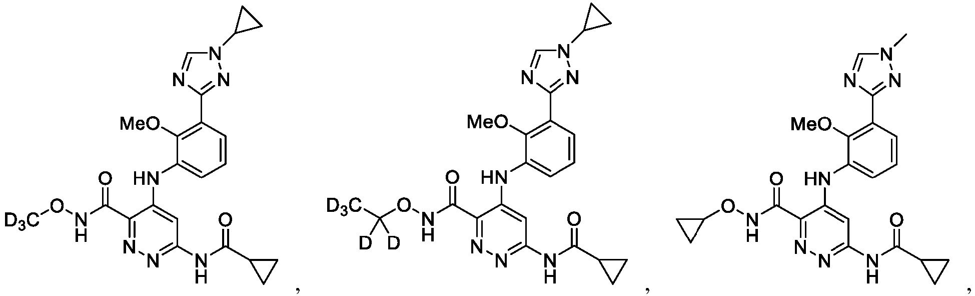

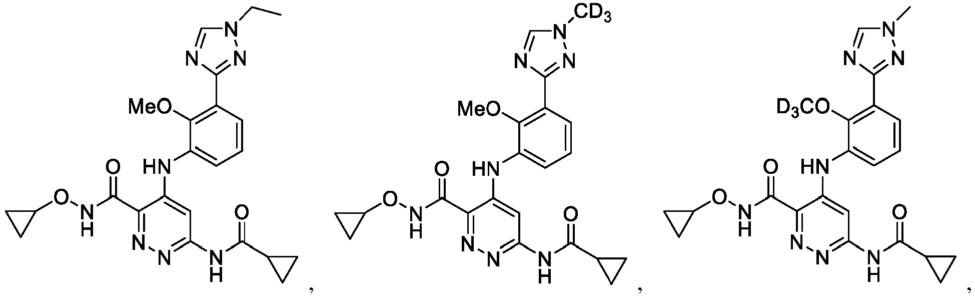

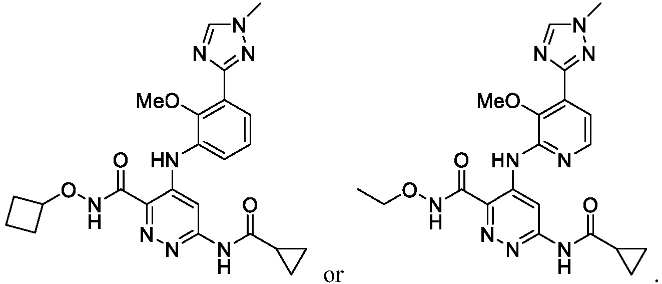

In one embodiment, the present invention provides a pharmaceutical composition comprising the compound, or the pharmaceutically acceptable salt, enantiomer, diastereomer, racemate, solvate, hydrate, polymorph, prodrug or isotopic variant thereof as defined herein, and a mixture thereof, and a pharmaceutically acceptable excipient. Preferably, the pharmaceutical composition further contains other therapeutic agents.

In one embodiment, the present invention provides a use of the compound or the pharmaceutically acceptable salt, enantiomer, diastereomer, racemate, solvate, hydrate, polymorph, prodrug or isotopic variant thereof as defined herein, and a mixture thereof, and a pharmaceutical composition comprising the same in the preparation of a medicament for treating and/or preventing a TYK2 kinase-mediated disease.

In one embodiment, the present invention provides a use of the compound or the pharmaceutically acceptable salt, enantiomer, diastereomer, racemate, solvate, hydrate, polymorph, prodrug or isotopic variant thereof as defined herein, and a mixture thereof, or a pharmaceutical composition comprising the compound in the treatment and/or prevention of a TYK2 kinase-mediated disease.

In one embodiment, the present invention provides a method for treating and/or preventing a TYK2 kinase-mediated disease in a subject by using the compound as defined herein, the method comprising administering to the subject the compound or the pharmaceutically acceptable salt, enantiomer, diastereomer, racemate, solvate, hydrate, polymorph, prodrug or isotopic variant thereof, and a mixture thereof, or a pharmaceutical composition comprising the same.

The TYK2 kinase-mediated disease recited in the present invention is selected from an autoimmune disease, a skin disease, an allergic disease, organ rejection, cancer, dry eye disease, myelofibrosis and polycythemia vera. Further, the autoimmune disease is lupus, multiple sclerosis, rheumatoid arthritis, juvenile arthritis, psoriasis, ulcerative colitis, Crohn's disease or autoimmune thyroid disease; the skin disease is psoriasis, a rash or atopic dermatitis; the allergic disease is asthma or rhinitis; the organ transplant rejection is allograft rejection or graft-versus-host disease; the cancer is renal cancer, liver cancer, pancreatic cancer, gastric cancer, breast cancer, prostate cancer, head and neck cancer, thyroid cancer, lung cancer, glioblastoma, melanoma, lymphoma or leukemia.

In some embodiments, the TYK2 kinase-mediated disease relating to the method is selected from rheumatoid arthritis, psoriasis, ulcerative colitis and Crohn's disease.

Those skilled in the art will understand that, without departing from common general knowledge in the art, the various preferred conditions described above can be arbitrarily combined to obtain various preferred embodiments of the present invention.

### Administration

The compound represented by (I) of the present invention may be administered by any means appropriate to the state of a disease to be treated. The means may depend on the need for site-specific treatment or the amount of a medicament to be delivered. Although other modes of delivery are contemplated, topical administration is generally preferred for skin-related diseases, and systemic treatment is preferred for cancerous or precancerous disease states. For example, the compound may be delivered by: oral administration, e.g., in the form of tablets, capsules, granules, powders or liquid preparations (including syrups); topical administration, e.g., in the form of solutions, suspensions, gels or ointments; sublingual administration; transbuccal administration; parenteral administration, e.g., by subcutaneous, intravenous, intramuscular or intrasternal injection or infusion techniques (e.g., in the form of sterile injectable aqueous or non-aqueous solutions or suspensions); nasal administration, e.g., by inhalation of sprays; topical administration, e.g., in the form of creams or ointments; rectal administration, e.g., in the form of suppositories; or liposomes. Unit dose formulations containing a non-toxic pharmaceutically acceptable carrier or diluent may be administered. The compounds may be administered in a form suitable for immediate release or extended release. Immediate release or extended release can be achieved using suitable pharmaceutical compositions or, particularly in the case of extended release, using devices such as subcutaneous implants or osmotic pumps.

Exemplary compositions for topical administration include topical carriers.

Exemplary compositions for oral administration include suspensions, which may contain, for example, microcrystalline cellulose to impart bulk, alginic acid or sodium alginate as a suspending agent, methylcellulose as a tackifier, and a sweetener or a flavoring agent such as those known in the art; and immediate release tablets, which may contain, for example, microcrystalline cellulose, dicalcium phosphate, starch, magnesium stearate and/or lactose and/or other excipients, binders, extenders, disintegrants, diluents and lubricants, such as those known in the art. The compound of the present invention may also be delivered orally by sublingual and/or buccal administration, for example, using molded, compressed or freeze-dried tablets. Exemplary compositions may include fast dissolving diluents, such as mannitol, lactose, sucrose and/or cyclodextrins. These formulations may also include high molecular weight excipients, such as cellulose (AVICEL^{®}) or polyethylene glycol (PEG); excipients for aiding mucoadhesion, such as hydroxypropylcellulose (HPC), hydroxypropylmethylcellulose (HPMC), sodium carboxymethylcellulose (SCMC) and/or maleic anhydride copolymers (e.g., GANTREZ^{®}); and agents for controlled release, such as polyacrylic acid copolymers (e.g., CARBOPOL 934^{®}). Lubricants, glidants, flavoring agents, coloring agents and stabilizers may also be added to facilitate preparation and use.

Exemplary compositions for nasal aerosol or inhalation administration include solutions, which may contain, for example, benzyl alcohol or other suitable preservatives, absorption promoters for enhancing absorption and/or bioavailability and/or other solubilizing or dispersing agents such as those known in the art.

Exemplary compositions for parenteral administration include injectable solutions or suspensions, which may contain, for example, suitable non-toxic parenterally acceptable diluents or solvents, such as mannitol, 1,3-butanediol, water, Ringer's solution, isotonic sodium chloride solution, or other suitable dispersing or wetting agents and suspending agents, including synthetic mono- or diglycerides and fatty acids, including oleic acid.

Exemplary compositions for rectal administration include suppositories, which may contain, for example, suitable non-irritating excipients such as cocoa butter, synthetic glycerides or polyethylene glycols, these excipients being solid at normal temperature but liquefying and/or dissolving in the rectal cavity to release a medicament.

The therapeutically effective amount of the compound of the present invention can be determined by those skilled in the art, and include exemplary doses of about 0.05-1000 mg/kg, 1-1000 mg/kg, 1-50 mg/kg, 5-250 mg/kg and 250-1000 mg/kg body weight of active compounds per day for mammals, which can be administered as a single dose or as individual divided doses (e.g., 1 to 4 times per day). It should be understood that the specific dosage level and administration frequency for any particular subject may vary and will depend on a variety of factors, including the activity of a specific compound used, the metabolic stability and duration of action of the compound, the species, age, weight, general health, sex and diet of the subject, the mode and time of administration, the rate of excretion, drug combinations and the severity of a particular disease state. Preferred subjects for treatment include animals, most preferably mammalian species, such as humans and domestic animals like dogs, cats, horses, etc. Thus, when the term "patient" is used herein, the term is meant to include all subjects, most preferably mammalian species suffering from TYK2 kinase-mediated diseases.

### Examples

The materials and reagents used herein are either commercially available or prepared by synthetic methods generally known in the art.

### Example 1

### 6-(Cyclopropylcarboxamido)-N-methoxy-4-((2-methoxy-3-(1-methyl-1H-1,2,4-triazol-3-yl)phenyl)amino)pyridazine-3-carboxamide (Compound 1)

### Synthesis of intermediate 1C

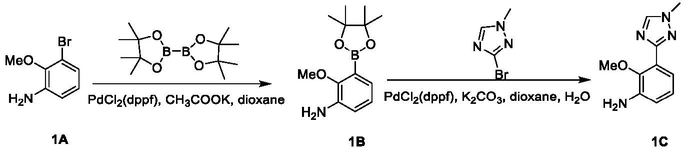

### Step 1: 2-Methoxy-3-(4,4,5,5-tetramethyl-1,3,2-dioxaborolan-2-yl)aniline

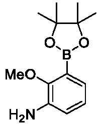

3-Bromo-2-methoxyaniline (10 g, 49.5 mmol) was dissolved in 1,4-dioxane (150 mL), and then diboric acid pinacol ester (25.1 g, 98.8 mmol), [1,1'-bis(diphenylphosphino)ferrocene] dichloropalladium (3.62 g, 4.95 mmol) and potassium acetate (9.72 g, 99 mmol) were added. After replacement with nitrogen, the mixture was allowed to react in an oil bath at 110°C for 3 hours. The reaction solution was poured into water (80 mL), the aqueous phase was extracted using ethyl acetate (100 mL × 3), and the merged organic phase was washed with saturated brine (80 mL × 3), dried over anhydrous sodium sulfate, and spun dry in vacuo to obtain a crude product. The crude product was separated and purified by column chromatography (silica gel, petroleum ether:ethyl acetate = 5:1) to obtain the title compound (7.8 g, yield: 63.4%, yellow solid).

MS (ESI): m/z 249.9 [M+H]⁺.

### Step 2: 2-Methoxy-3-(1-methyl-1H-1,2,4-triazol-3-yl)aniline

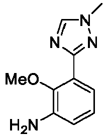

2-Methoxy-3-(4,4,5,5-tetramethyl-1,3,2-dioxaborolan-2-yl)aniline (5.4 g, 21.7 mmol) was dissolved in 1,4-dioxane (80 mL), and water (16 mL), 3-bromo-1-methyl-1*H*-1,2,4-triazole (5.27 g, 32.5 mmol), [1,1'-bis(diphenylphosphino)ferrocene]dichloropalladium (1.59 g, 2.17 mmol) and potassium carbonate (9.0 g, 65.1 mmol) were added. After replacement with nitrogen, the mixture was allowed to react in an oil bath at 110°C for 16 hours. The reaction solution was poured into water (50 mL), the aqueous phase was extracted using ethyl acetate (60 mL × 3), and the merged organic phase was washed with saturated brine (50 mL × 3), dried over anhydrous sodium sulfate, and spun dry in vacuo to obtain a crude product. The crude product was separated and purified by column chromatography (silica gel, petroleum ether:ethyl acetate = 1:1) to obtain the title compound (3.1 g, yield: 70.1%, brown solid).

MS (ESI): m/z 205.1 [M+H]⁺.

### Synthesis of compound 1

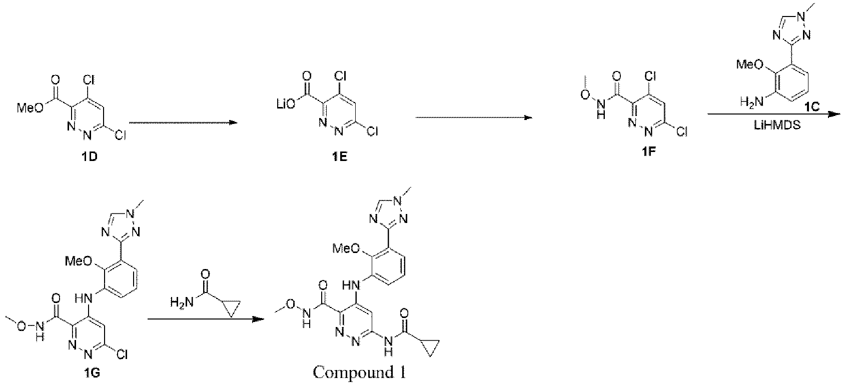

### Step 1: Lithium 4,6-dichloropyridazine-3-carboxylate

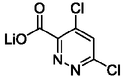

Methyl 4,6-dichloropyridazine-3-carboxylate (10 g, 48.31 mmol) was dissolved in water (7.5 mL) and acetonitrile (50 mL), and lithium bromide (12.58 g, 144.93 mmol) and DIEA (18.7 g, 144.93 mmol) were added for reaction at room temperature for 4 hours. The reaction solution was filtered by suction, and the filter cake was rinsed using acetonitrile to obtain the title compound (6.84 g, yield: 71.3%, white solid).

MS (ESI): m/z 192.9 [M+H]⁺.

### Step 2: 4,6-Dichloro-N-methoxypyridazine-3-carboxamide

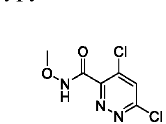

Lithium 4,6-dichloropyridazine-3-carboxylate (2 g, 10.4 mmol) was dissolved in thionyl chloride (20 mL), and DMF (0.5 mL) was added for reaction at 90°C for 1 hour. The reaction solution was spun dry, DCM (20 mL) was added, the temperature was lowered to 0°C, and methoxyamine (910.4 mg, 10.5 mmol) and triethylamine (5.26 g, 52.0 mmol) were added at 0°C. The reaction solution was allowed to react at room temperature for 1 hour, and was spun in vacuo to remove the solvent to obtain a crude product. The crude product was separated and purified by column chromatography (silica gel, petroleum ether:ethyl acetate = 3:1) to obtain the title compound (613 mg, yield: 27.5%, yellow solid).

MS (ESI): m/z 208.9 [M+H]⁺.

### Step 3: 6-Chloro-N-methoxy-4-((2-methoxy-3-(1-methyl-1H-1,2,4-triazol-3-yl)phenyl)amino)pyridazine-3-carboxamide

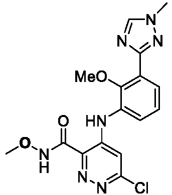

2-Methoxy-3-(1-methyl-1*H*-1,2,4-triazol-3-yl)aniline (1.0 g, 4.90 mmol) was dissolved in THF (40 mL). After replacement with nitrogen three times, the reaction system was cooled to 0°C, and lithium bis(trimethylsilyl)amide (2.46 g, 14.71 mmol) was slowly added dropwise at 0°C using a syringe. The reaction solution was allowed to react at 0°C for 1 hour. Then, a solution of 4,6-dichloro-*N*-methoxypyridazine-3-carboxamide (1.08 g, 4.90 mmol) in tetrahydrofuran was injected into the reaction solution at 0°C using a syringe. After the reaction solution naturally warmed to room temperature, the mixture was allowed to react at room temperature for 16 hours. A saturated aqueous ammonium chloride solution (10 mL) was added to the reaction solution for quenching. After quenching, the reaction solution was poured into water (10 mL). The aqueous phase was extracted three times using ethyl acetate (30 mL), and the merged organic phase was washed with saturated brine (30 mL), dried over anhydrous sodium sulfate, and spun dry in vacuo to obtain a crude product. The crude product was separated and purified by column chromatography (silica gel, dichloromethane:methanol = 15:1) to obtain the title compound (880 mg, yield: 46.3%, yellow solid).

MS (ESI): m/z 390.1 [M+H]⁺.

### Step 4: 6-(Cyclopropylcarboxamido)-N-methoxy-4-((2-methoxy-3-(1-methyl-1H-1,2,4-triazol-3-yl)phenyl)amino)pyridazine-3-carboxamide (Compound 1)

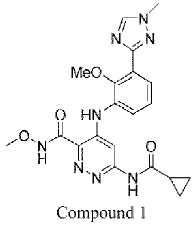

6-Chloro-*N*-methoxy-4-((2-methoxy-3-(1-methyl-1*H*-1,2,4-triazol-3-yl)phenyl)amino)pyridazine-3-carboxamide (200 mg, 0.51 mmol) was dissolved in 1,4-dioxane (8.5 mL), and cyclopropylcarboxamide (434 mg, 5.10 mmol), tris(dibenzylideneacetone)dipalladium (186.8 mg, 0.204 mmol), 4,5-bis(diphenylphosphino-9,9-dimethylxanthene) (235.8 mg, 0.408 mmol) and cesium carbonate (498.5 mg, 1.53 mmol) were added. After replacement with nitrogen, the mixture was allowed to react in an oil bath at 110°C for 16 hours. The reaction solution was spun in vacuo to remove the solvent, and then filtered by suction using dichloromethane:methanol = 15:1 to obtain a crude product. The crude product was purified by reverse phase preparative chromatography to obtain the title compound (5.9 mg, yield: 2.6%, white solid).

MS (ESI): m/z 439.1 [M+H]⁺.

¹H NMR (400 MHz, CD₃OD) δ 8.47 (s, 1H), 8.25 (s, 1H), 7.67 (d, *J =* 7.5 Hz, 1H), 7.59 (d, *J* = 7.8 Hz, 1H), 7.29 (t, *J =* 7.9 Hz, 1H), 4.01 (s, 3H), 3.87 (s, 3H), 3.74 (s, 3H), 1.29 (s, 1H), 0.99 - 0.87 (m, 4H).

### Example 2

### 6-((6-cyanopyridin-2-yl)amino)-N-methoxy-4-((2-methoxy-3-(1-methyl-1H-1,2,4-triazol-3-yl)phenyl)amino)pyridazine-3-carboxamide (Compound 2)

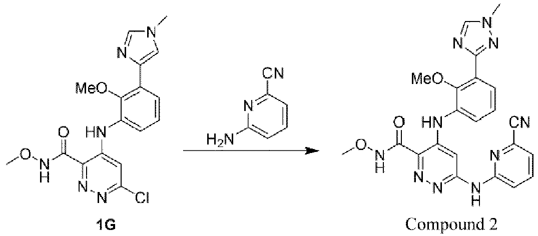

6-Chloro-*N*-methoxy-4-((2-methoxy-3-(1-methyl-1*H*-1,2,4-triazol-3-yl)phenyl)amino)pyridazine-3-carboxamide (200 mg, 0.51 mmol) was dissolved in 1,4-dioxane (3 mL), and 6-aminopyridinecarbonitrile (303.8 mg, 2.55 mmol), tris(dibenzylideneacetone)dipalladium (140.1 mg, 0.153 mmol), 4,5-bis(diphenylphosphino-9,9-dimethylxanthene) (147.4 mg, 0.255 mmol) and cesium carbonate (830.8 mg, 2.55 mmol) were added. After replacement with nitrogen, the mixture was allowed to react in an oil bath at 130°C for 16 hours. The reaction solution was spun in vacuo to remove the solvent, and then slurried and filtered by suction using a mixed solvent of dichloromethane:methanol = 15:1 to obtain a crude product. The crude product was purified by reverse phase preparative chromatography to obtain the title compound (9.72 mg, yield: 4.0%, yellow powder).

MS (ESI): m/z 473.2 [M+H]⁺.

¹H NMR (400 MHz, DMSO-*d*₆) δ 8.57 (s, 1H), 8.38 (s, 2H), 7.92 (d, *J =* 7.6 Hz, 1H), 7.72 (s, 2H), 7.62-7.59 (m, 3H), 7.38 (s, 1H), 3.96 (s, 3H), 3.77 (s, 3H), 3.76 (s, 3H).

### Example 3

### 6-(Cyclopropylcarboxamido)-N-ethoxy-4-((2-methoxy-3-(1-methyl-1H-1,2,4-triazol-3-yl)phenyl)amino)pyridazine-3-carboxamide (Compound 3)

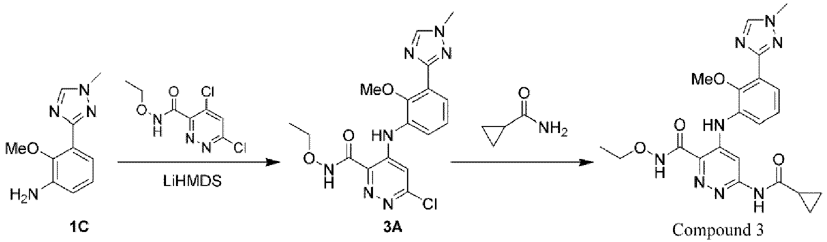

### Step 1: 6-Chloro-N-ethoxy-4-((2-methoxy-3-(1-methyl-1H-1,2,4-triazol-3-yl)phenyl)amino)pyridazine-3-carboxamide

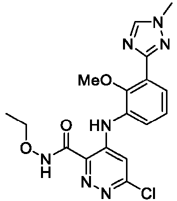

2-Methoxy-3-(1-methyl-1*H*-1,2,4-triazol-3-yl)aniline (420 mg, 2.06 mmol) was dissolved in tetrahydrofuran (10 mL). After replacement with nitrogen, the reaction solution was cooled to 0°C. Lithium bis(trimethylsilyl)amide (1.03 g, 6.16 mmol) was slowly added dropwise at 0°C, and the reaction solution was allowed to react at 0°C for 1 hour. Then, a solution (2 mL) of 4,6-dichloro-*N*-ethoxypyridazine-3-carboxamide (484 mg, 2.06 mmol) in tetrahydrofuran was added at 0°C. The reaction solution was allowed to naturally warm to room temperature and then was allowed to react at room temperature for 16 hours. Methanol (2 mL) was added to the reaction solution for quenching, and the reaction solution was spun in vacuo to remove the solvent to obtain a crude product. The crude product was separated and purified by column chromatography (silica gel, dichloromethane:methanol = 15:1) to obtain the title compound (270 mg, yield: 32.5%, white solid).

MS (ESI): m/z 404.4 [M+H]⁺.

### Step 2: 6-(Cyclopropylcarboxamido)-N-ethoxy-4-((2-methoxy-3-(1-methyl-1H-1,2,4-triazol-3-yl)phenyl)amino)pyridazine-3-carboxamide (Compound 3)

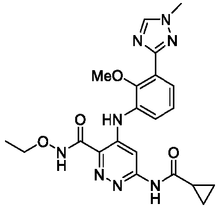

6-Chloro-*N*-ethoxy-4-((2-methoxy-3-(1-methyl-1*H*-1,2,4-triazol-3-yl)phenyl)amino)pyridazine-3-carboxamide (140 mg, 0.347 mmol) was dissolved in 1,4-dioxane (3 mL), and cyclopropylcarboxamide (295 mg, 3.47 mmol), tris(dibenzylideneacetone)dipalladium (127 mg, 0.139 mmol), 4,5-bis(diphenylphosphino-9,9-dimethylxanthene) (166 mg, 0.287 mmol) and cesium carbonate (339 mg, 1.04 mmol) were added. After replacement with nitrogen, the mixture was allowed to react in an oil bath at 110°C for 16 hours. The reaction solution was filtered by suction and spun in vacuo to remove the solvent to obtain a crude product. The crude product was separated and purified by high-performance liquid chromatography (elution system: formic acid, water and acetonitrile) to obtain the title compound (4 mg, yield: 2.55%, white solid).

MS (ESI): m/z 453.1 [M+H]⁺.

¹H NMR (400 MHz, CD₃OD) δ 8.47 (s, 1H), 8.26 (s, 1H), 7.68 (d, *J =* 7.7 Hz, 1H), 7.60 (d, *J =* 8.0 Hz, 1H), 7.30 (t, *J =* 7.8 Hz, 1H), 4.12 - 4.06 (m, 2H), 4.02 (s, 3H), 3.74 (s, 3H), 1.37 (brs, 1H), 1.29 (brs, 3H), 0.97 (d, *J =* 4.4 Hz, 2H), 0.92 (d, *J =* 8.0 Hz, 2H).

### Example 4

### 6-(Cyclopropylcarboxamido)-N-ethoxy-4-((5-fluoro-2-methoxy-3-(1-methyl-1H-1,2,4-triazol-3-yl)phenyl)amino)pyridazine-3-carboxamide (Compound 4)

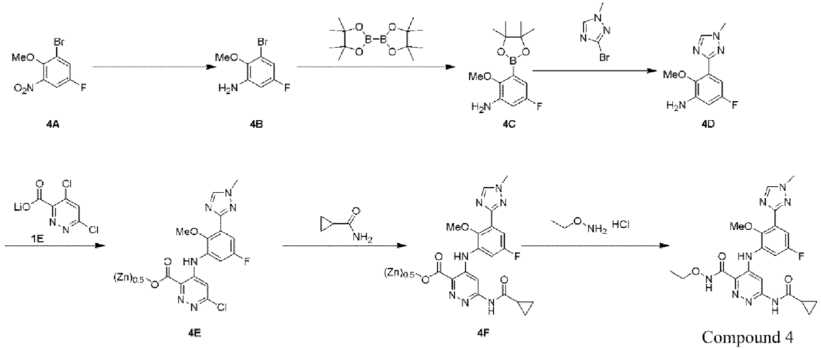

### Step 1: 3-Bromo-5-fluoro-2-methoxyaniline

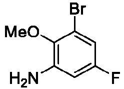

1-Bromo-5-fluoro-2-methoxy-3-nitrobenzene (2.539 g, 10.15 mmol) was dissolved in a mixed solution of isopropanol and water (12 ml), ammonium chloride (2.714 g, 50.74 mmol) was added, and then iron powder (2.79 g, 50.74 mmol) was added in batches. After replacement with nitrogen, the mixture was allowed to react in an oil bath at 80°C for 2 hours. The reaction solution was filtered by suction, and the filtrate was concentrated under reduced pressure to obtain a crude product. The crude product was separated and purified by silica gel column chromatography (petroleum ether:ethyl acetate = 10:1) to obtain the title compound (1.68 g, yield: 75.3%, yellow solid).

MS (ESI): m/z 221.9 [M+H]⁺.

### Step 2: 5-Fluoro-2-methoxy-3-(4,4,5,5-tetramethyl-1,3,2-dioxaborolan-2-yl)aniline

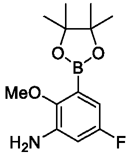

3-Bromo-5-fluoro-2-methoxyaniline (1.582 g, 7.19 mmol) was dissolved in a 1,4-dioxane solution (12 mL), and diboric acid pinacol ester (3.62 g, 14.25 mmol), potassium acetate (1.398 g, 14.25 mmol) and [1,1'-bis(diphenylphosphino)ferrocene]palladium dichloride (521.7 mg, 0.713 mmol) were added. After replacement with nitrogen, the mixture was allowed to react in an oil bath at 110°C for two hours. Water (50 mL) was added to the reaction solution, the aqueous phase was extracted using ethyl acetate (60 mL × 3), and the merged organic phase was washed with saturated brine (60 mL × 3), dried over anhydrous sodium sulfate, and spun in vacuo to remove the solvent to obtain a crude product. The crude product was separated and purified by silica gel column chromatography (petroleum ether:ethyl acetate = 5:1) to obtain the title compound (1.05 g, yield: 54.7%, yellow solid).

MS (ESI): m/z 268.0 [M+H]⁺.

### Step 3: 5-Fluoro-2-methoxy-3-(1-methyl-1H-1,2,4-triazol-3-yl)aniline

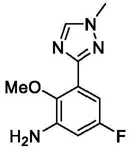

5-Fluoro-2-methoxy-3-(4,4,5,5-tetramethyl-1,3,2-dioxaborolan-2-yl)aniline (1.05 g, 3.93 mmol) was dissolved in a mixed solution of 1,4-dioxane and water (8 mL), and 3-bromo-1-methyl-1*H*-1,2,4-triazole (955 mg, 5.90 mmol), potassium carbonate (1.63 g, 11.8 mmol) and [1,1'-bis(diphenylphosphino)ferrocene]palladium dichloride (288 mg, 0.394 mmol) were added. After replacement with nitrogen, the mixture was allowed to react in an oil bath at 110°C for 16 hours. The reaction was stopped, and water (40 mL) was added to the reaction solution after cooling to room temperature. The aqueous phase was extracted using ethyl acetate (50 mL × 3), and the merged organic phase was washed with saturated brine (50 mL × 3), dried over anhydrous sodium sulfate, and spun in vacuo to remove the solvent to obtain a crude product. The crude product was purified by silica gel column chromatography (dichloromethane:methanol = 15:1) to obtain the title compound (600 mg, yield: 68.6%, yellow solid).

MS (ESI): m/z 223.1 [M+H]⁺.

### Step 4: ((6-Chloro-4-((5-fluoro-2-methoxy-3-(1-methyl-1H-1,2,4-triazol-3-yl)phenyl)amino)pyridazine-3-carbonyl)oxy)zinc (0.5)

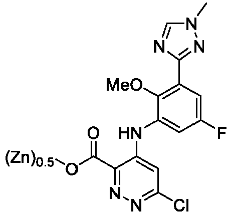

5-Fluoro-2-methoxy-3-(1-methyl-1H-1,2,4-triazol-3-yl)aniline (410 mg, 1.80 mmol) was dissolved in water (7 mL) and isopropanol (1 mL), and lithium 4,6-dichloropyridazine-3-carboxylate (414.7 mg, 2.16 mmol) and zinc acetate (396.4 mg, 2.16 mmol) were added. After replacement with nitrogen three times, the mixture was allowed to react in an oil bath at 65°C for 16 hours. The reaction was stopped, and water (10 mL) was added to the reaction solution. The solution was stirred at room temperature for 1 hour and then filtered by suction. The filter cake was rinsed using water (10 mL × 2) and tetrahydrofuran (3 mL) to obtain the title compound (140 mg, yield: 20.1%, yellow solid).

MS (ESI): m/z 379.0 [M+H]⁺.

### Step 5: ((6-(Cyclopropylcarboxamido)-4-((5-fluoro-2-methoxy-3-(1-methyl-1H-1,2,4-triazol-3-yl)phenyl)amino)pyridazine-3-carbonyl)oxy)zinc (0.5)

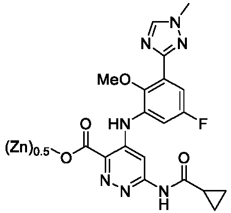

((6-Chloro-4-((5-fluoro-2-methoxy-3-(1-methyl-1*H*-1,2,4-triazol-3-yl)phenyl)amino)pyridazine-3-carbonyl)oxy)zinc (0.5) (140 mg, 0.371 mmol) was dissolved in toluene (2 mL) and acetonitrile (1 mL), and cyclopropylcarboxamide (78.9 mg, 0.927 mmol), (R)-(-)-1-[(S)-2-(dicyclohexylphosphino)ferrocene]ethyl di-tert-butylphosphine (41.1 mg, 0.074 mmol), 1,8-diazabicycloundec-7-ene (56.5 mg, 0.371 mmol), cesium carbonate (242 mg, 0.742 mmol) and palladium acetate (8.33 mg, 0.0371 mmol) were added. After replacement with nitrogen, the mixture was allowed to react in an oil bath at 100°C for 16 hours. After the reaction was stopped, the reaction solution was cooled to room temperature. Water (6 mL) and acetic acid (3 mL) were added, and the mixed system was washed using petroleum ether (10 mL × 3). The aqueous phase was extracted using dichloromethane (20 mL × 3), and the merged organic phase was washed with saturated brine (20 mL × 3), dried over anhydrous sodium sulfate, and spun in vacuo to remove the solvent to obtain the title compound (110 mg, yield: 69.6%, brown solid).

MS (ESI): m/z 428.1 [M+H]⁺.

### Step 6: 6-(Cyclopropylcarboxamido)-N-ethoxy-4-((5-fluoro-2-methoxy-3-(1-methyl-1H-1,2,4-triazol-3-yl)phenyl)amino)pyridazine-3-carboxamide (Compound 4)

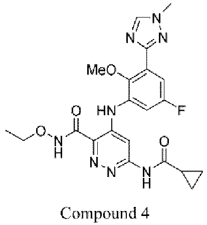

N-Methylpyrrolidone (3 mL) and acetonitrile (3 mL) were added to a reaction flask, and then ((6-(cyclopropylcarboxamido)-4-((5-fluoro-2-methoxy-3-(1-methyl-1*H*-1,2,4-triazol-3-yl)phenyl)amino)pyridazine-3-carbonyl)oxy)zinc (0.5) (110 mg, 0.258 mmol), O-ethylhydroxylamine hydrochloride 12 (37.7 mg, 0.387 mmol) and *N*-methylimidazole (63.5 mg, 0.773 mmol) were added. The reaction solution was stirred in an oil bath at 65°C for 15 minutes, and then 1-hydroxybenzotriazole (69.7 mg, 0.516 mmol) and 1-ethyl-(3-dimethylaminopropyl)carbodiimide hydrochloride (98.9 mg, 0.516 mmol) were added. The reaction solution was stirred in an oil bath at 65°C for 16 hours. The reaction was stopped, and water (10 mL) was added to the reaction solution for quenching. The aqueous phase was extracted using ethyl acetate (15 mL × 3), and the merged organic phase was washed with saturated brine (15 mL × 3), dried over anhydrous sodium sulfate, and spun in vacuo to remove the solvent to obtain a crude product. The crude product was separated and purified by high-performance liquid chromatography (elution system: formic acid, water and acetonitrile) to obtain the title compound (6.01 mg, yield: 5.0%, yellow solid).

MS (ESI): m/z 471.0 [M+H]⁺.

¹H NMR (400 MHz, DMSO-*d*₆) δ 10.73 (s, 1H), 8.60 (s, 1H), 8.39 (s, 1H), 8.25 (s, 1H), 7.45 - 7.39 (m, 2H), 4.02 - 3.98 (m, 2H), 3.96 (s, 3H), 3.74 (s, 3H), 2.11 (dd, *J* = 12.1, 5.7 Hz, 1H), 1.23 (t, *J =* 7.0 Hz, 3H), 0.85 (d, *J* = 5.7 Hz, 4H).

### Example 5

### 6-(Cyclopropylcarboxamido)-N-ethoxy-4-((2-methoxy-3-(1-(methyl-d3)-1H-1,2,4-triazol-3-yl)phenyl)amino)pyridazine-3-carboxamide (Compound 5)

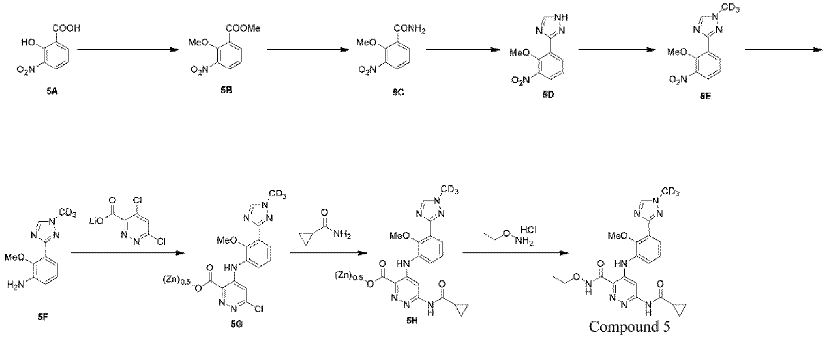

### Step 1: Methyl 2-methoxy-3-nitrobenzoate

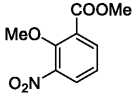

2-Hydroxy-3-nitrobenzoic acid (2.5 g, 13.65 mmol) was dissolved in *N,N-*dimethylacetamide (50 mL), and iodomethane (9.7 g, 68.3 mmol) and cesium carbonate (17.8 g, 54.61 mmol) were added for reaction at room temperature for 16 hours. Water (250 mL) was added to the reaction solution, and the solution was stirred for 1 hour and then filtered by suction. The filter cake was taken out and washed using acetonitrile (10 mL × 3), and spun in vacuo to remove the solvent. The crude product was separated and purified by column chromatography (silica gel, petroleum ether:ethyl acetate = 4:1) to obtain the title compound (2.36 g, yield: 82.1%, yellow solid).

MS (ESI): m/z 212.1 [M+H]⁺.

### Step 2: 2-Methoxy-3-nitrobenzamide

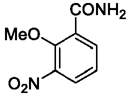

Methyl 2-methoxy-3-nitrobenzoate (2.26 g, 10.7 mmol) was dissolved in a methanolic ammonia solution (12 mL, 48 mmol), and aqueous ammonia (9 mL) was added. The mixture was sealed and allowed to react at room temperature for 16 hours. The reaction solution was spun in vacuo to remove the solvent, and was separated and purified by column chromatography (silica gel, petroleum ether:ethyl = 1:1) to obtain the title compound (1.545 g, yield: 73.7%, yellow solid).

MS (ESI): m/z 197.0 [M+H]⁺.

### Step 3: 3-(2-Methoxy-3-nitrophenyl)-1H-1,2,4-triazole

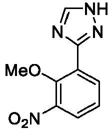

2-Methoxy-3-nitrobenzamide (1.62 g, 8.26 mmol) was dissolved in *N,N-*dimethylformamide dimethyl acetal (22 mL) for reaction in an oil bath at 95°C for 1 hour, followed by spinning in vacuo to remove the solvent, and ethanol (6 mL) was added for dissolution to obtain a crude solution. In an ice bath, ethanol (16 mL) and acetic acid (6 mL) were added to a reaction flask and stirred for 5 minutes, then hydrazine hydrate (12 mL) was added dropwise and continued to be stirred for 15 minutes, then an ethanol solution of the crude product was added dropwise. The solution was slowly warmed to room temperature, and stirred for 4 hours. After the reaction solution was spun in vacuo to remove the solvent, ethyl acetate (200 mL) was added for dilution, and a saturated sodium bicarbonate aqueous solution was used for washing (200 mL × 2). The organic phase was separated and washed with saturated brine (50 mL × 3), dried over anhydrous sodium sulfate, and separated and purified by column chromatography (silica gel, petroleum ether:ethyl acetate = 1:1) to obtain the title compound (830 mg, yield: 45.7%, yellow solid).

MS (ESI): m/z 221.0 [M+H]⁺.

### Step 4: 3-(2-Methoxy-3-nitrophenyl)-1-(methyl-d₃)-1H-1,2,4-triazole

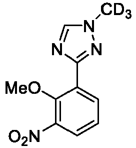

3-(2-Methoxy-3-nitrophenyl)-1H-1,2,4-triazole (1 g, 4.55 mmol) was dissolved in *N,N-*dimethylacetamide (16 mL), cesium carbonate (2.96 g, 9.1 mmol) was added, and deuterated iodomethane (988.4 mg, 6.82 mmol) was added at 0°C. The solution was slowly warmed to room temperature for reaction for 2 hours. A saturated ammonium chloride solution (15 mL) was added to the reaction solution for quenching, and extraction was performed using ethyl acetate (30 mL × 3). The organic phase was washed with saturated brine (20 mL × 3), dried over anhydrous sodium sulfate, and separated and purified by column chromatography (silica gel, petroleum ether:ethyl acetate = 2:3) to obtain the title compound (866 mg, yield: 80.3%, yellow solid).

MS (ESI): m/z 238.3 [M+H]⁺.

### Step 5: 2-Methoxy-3-(1-(methyl-d₃)-1H-1,2,4-triazol-3-yl)aniline

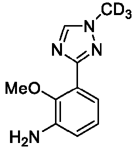

3-(2-Methoxy-3-nitrophenyl)-1-(methyl-*d*₃)-1*H*-1,2,4-triazole (866 mg, 3.65 mmol) was dissolved in methanol (20 mL), and aqueous ammonia (1 mL) and palladium/carbon (155.6 mg) were added. After replacement with hydrogen, the mixture was allowed to react at room temperature for 2 hours. The reaction solution was filtered by suction, washed with methanol (20 mL × 3), and spun in vacuo to remove the solvent to obtain the title compound (622 mg, yield: 82%, yellow solid).

MS (ESI): m/z 208.1 [M+H]⁺.

### Step 6: ((6-Chloro-4-((2-methoxy-3-(1-(methyl-d₃)-1H-1,2,4-triazol-3-yl)phenyl)amino)pyridazine-3-carbonyl)oxy)zinc (0.5)

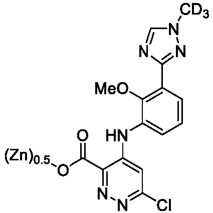

2-Methoxy-3-(1-(methyl-*d*₃)-1*H*-1,2,4-triazol-3-yl)aniline (320 mg, 1.55 mmol) and lithium 4,6-dichloropyridazine-3-carboxylate (357.1 mg, 1.86 mmol) were dissolved in a mixed solvent of isopropanol (2 mL) and water (14 mL), zinc acetate (340 mg, 1.86 mmol) was added, and the mixture was allowed to react in an oil bath at 65°C for 16 hours. After the reaction stopped, water (15 mL) was added to the reaction solution and stirring was performed at room temperature for 1 hour. The reaction solution was filtered by suction, and the filter cake was washed using water (10 mL × 2) and tetrahydrofuran (5 mL × 3), and spun in vacuo to remove the solvent to obtain the title compound (420 mg, yield: 74.6%, yellow solid).

MS (ESI): m/z 364.0 [M+H]⁺.

### Step 7: ((6-(Cyclopropylcarboxamido)-4-((2-methoxy-3-(1-(methyl-d₃)-1H-1,2,4-triazol-3-yl)phenyl)amino)pyridazine-3-carbonyl)oxy)zinc (0.5)

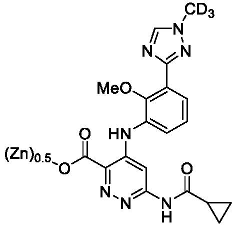

((6-Chloro-4-((2-methoxy-3-(1-(methyl-*d*₃)-1*H*-1,2,4-triazol-3-yl)phenyl)amino)pyridazine-3-carbonyl)oxy)zinc (0.5) (400 mg, 1.02 mmol) was dissolved in toluene (7 mL) and acetonitrile (14 mL), and cyclopropylcarboxamide (216 mg, 2.454 mmol), (R)-1-[(SP)-2-(dicyclohexylphosphino)ferrocene]ethyl di-*tert*-butylphosphine (112.6 mg, 0.203 mmol), diazabicyclo (154.6 mg, 1.02 mmol), cesium carbonate (661 mg, 2.03 mmol) and palladium acetate (45.6 mg, 0.203 mmol) were added. After replacement with nitrogen, the mixture was allowed to react in an oil bath at 100°C for 16 hours. After the reaction stopped, water (20 mL) and acetic acid (10 mL) were added to the reaction solution, and stirring was performed at room temperature for 0.5 hours, followed by washing using petroleum ether (15 mL × 3). The aqueous phase was extracted using dichloromethane (20 mL × 3), washed with saturated brine (15 mL×3), dried over anhydrous sodium sulfate, and spun in vacuo to remove the solvent to obtain the title compound (400 mg, yield: 88.5%, brown solid).

MS (ESI): m/z 413.1 [M+H]⁺.

### Step 8: 6-(Cyclopropylcarboxamido)-N-ethoxy-4-((2-methoxy-3-(1-(methyl-d3)-1H-1,2,4-triazol-3-yl)phenyl)amino)pyridazine-3-carboxamide (Compound 5)

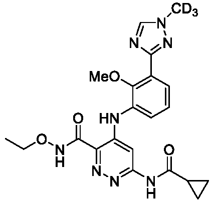

*N*-Methylpyrrolidone (15 mL) and acetonitrile (15 mL) were added to a reaction flask and stirred at room temperature for 10 minutes, and then ((6-(cyclopropylcarboxamido)-4-((2-methoxy-3-(1-(methyl-*d*₃)-1*H*-1,2,4-triazol-3-yl)phenyl)amino)pyridazine-3-carbonyl)oxy)zinc (0.5) (400 mg, 0.9 mmol), O-ethylhydroxylamine hydrochloride (132 mg, 1.35 mmol) and *N-*methylimidazole (222.3 mg, 2.71 mmol) were added. After replacement with nitrogen, the mixture was stirred in an oil bath at 65°C for 15 minutes, and then 1-hydroxybenzotriazole (244 mg, 1.81 mmol) and 1-ethyl-(3-dimethylaminopropyl)carbodiimide hydrochloride (346.1 mg, 1.81 mmol) were added. After replacement with nitrogen, the mixture was allowed to react in an oil bath at 65°C for 16 hours. The reaction was stopped, and water (30 mL) was added to the reaction solution for quenching, and extraction was performed using ethyl acetate (30 mL × 3). The organic phase was washed with saturated brine (20 mL × 3), dried over anhydrous sodium sulfate, and spun in vacuo to remove the solvent. A crude product was prepared and purified by high-performance liquid chromatography (elution system: ammonia, water and acetonitrile) to obtain the title compound (11.4 mg, yield: 2.8%, white solid)

MS (ESI): m/z 456.2 [M+H]⁺.

¹H NMR (400 MHz, DMSO-*d*₆) δ 12.22 (s, 1H), 11.31 (s, 1H), 10.55 (s, 1H), 8.56 (s, 1H), 8.15 (s, 1H), 7.66 (d, *J =* 6.9 Hz, 1H), 7.51 (d, *J =* 5.8 Hz, 1H), 7.28 (d, *J =* 6.6 Hz, 1H), 3.99 (d, *J* = 5.4 Hz, 2H), 3.73 (s, 3H), 2.09 (brs, 1H), 1.23 (brs, 3H), 0.82 (brs, 4H).

### Example 6

### 6-(Cyclopropylcarboxamido)-N-ethoxy-4-((5-fluoro-2-methoxy-3-(1-(methyl-d₃)-1H-1,2,4-triazol-3-yl)phenyl)amino)pyridazine-3-carboxamide (Compound 6)

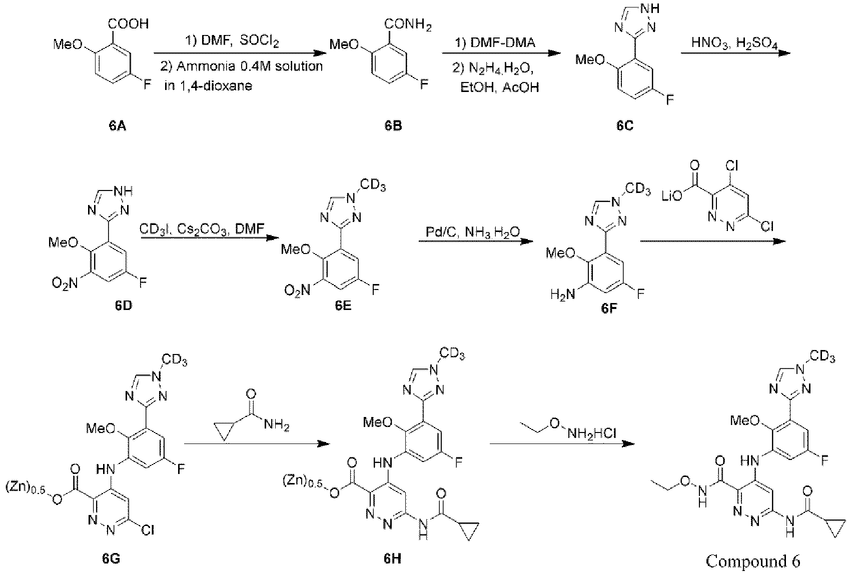

### Step 1: 5-Fluoro-2-methoxybenzamide

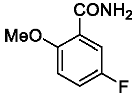

5-Fluoro-2-methoxybenzoic acid (7.2 g, 42.32 mmol) was dissolved in thionyl chloride (20 mL), and N,N-dimethylformamide (0.5 mL) was added. The reaction solution was allowed to react in an oil bath at 85°C for 2 hours. The reaction solution was spun in vacuo to remove the solvent, dichloromethane (50 mL × 3) was added in portions and spinning was performed to remove the residual thionyl chloride to obtain a crude product, and the crude product was dissolved in dichloromethane (20 mL) for later use. A 500 mL three-necked flask was taken, into which an ammonia 1,4-dioxane solution (0.4 M, 170 mL) was added. The solution was cooled to 0°C, and the reserved dichloromethane solution was slowly added dropwise. The reaction solution continued to be stirred for 30 minutes. The reaction solution was poured into water (100 mL), the aqueous phase was extracted using ethyl acetate (100 mL × 3), and the merged organic phase was washed with saturated brine (70 mL × 3), dried over anhydrous sodium sulfate, and spun in vacuo to remove the solvent to obtain the title compound (6.25 g, yield: 87.3%, white solid).

MS (ESI): m/z 170.1 [M+H]⁺.

### Step 2: 3-(5-Fluoro-2-methoxyphenyl)-1H-1,2,4-triazole

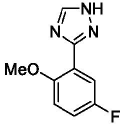

5-Fluoro-2-methoxybenzamide (6.25 g, 36.95 mmol) was dissolved in *N,N-*dimethylformamide dimethyl acetal (50 mL). After replacement with nitrogen, the mixture was allowed to react in an oil bath at 95°C for 1 hour. The reaction solution was spun in vacuo to remove the solvent to obtain a crude DMF-DMA addition product, which was dissolved in ethanol (30 mL) for later use. In an ice bath, ethanol (80 mL) and acetic acid (30 mL) were added to the reaction flask, and hydrazine hydrate (98 wt%, 15 mL) was added dropwise. The mixture continued to be stirred for 15 minutes, and then an ethanol solution of the crude DMF-DMA addition product was added dropwise. After the reaction solution naturally warmed to room temperature, the reaction solution was stirred at room temperature for 4 hours. The reaction solution was concentrated under reduced pressure, then diluted using ethyl acetate (500 mL), and washed with a saturated sodium bicarbonate aqueous solution (400 mL × 3). The organic phase was washed with saturated brine (300 mL × 3), dried over anhydrous sodium sulfate, and spun in vacuo to remove the solvent to obtain the title compound (5.46 g, yield: 76.5%, white solid).

MS (ESI): m/z 194.1 [M+H]⁺.

### Step 3: 3-(5-Fluoro-2-methoxy-3-nitrophenyl)-1H-1,2,4-triazole

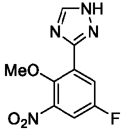

3-(5-Fluoro-2-methoxyphenyl)-*1H*-1,2,4-triazole (5.46 g, 28.26 mmol) was dissolved in concentrated sulfuric acid (50 mL). Nitric acid (68 wt%, 5.24 g, 56.55 mmol) was added dropwise in an ice bath. After the addition was complete, the solution continued to be stirred in the ice bath for 2 hours. The reaction was stopped, the reaction solution was poured into ice water (50 mL), and ammonia water was slowly added dropwise to adjust the pH to 9.0. The aqueous phase was extracted using ethyl acetate (100 mL × 3), and the merged organic phase was washed with saturated brine (80 mL × 3), dried over anhydrous sodium sulfate, and spun in vacuo to remove the solvent to obtain the title compound (6.4 g, yield: 95.1%, yellow solid).

MS (ESI): m/z 239.1 [M+H]⁺.

### Step 4: 3-(5-Fluoro-2-methoxy-3-nitrophenyl)-1-(methyl-d₃)-1H-1,2,4-triazole

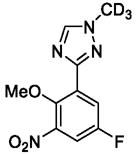

3-(5-Fluoro-2-methoxy-3-nitrophenyl)-1*H*-1,2,4-triazole (3.4 g, 14.3 mmol) and cesium carbonate (5.59 g, 17.16 mmol) were added to *N,N*-dimethylformamide (60 mL). After replacement with nitrogen, the reaction system was cooled to 0°C, then deuterated iodomethane (3.11 g, 21.5 mmol) was added, and the reaction solution was stirred at room temperature for 4 hours. The reaction was stopped, water (70 mL) was added to the reaction solution for quenching, and extraction was performed three times using ethyl acetate (80 mL). The merged organic phase was washed with saturated brine, dried over anhydrous sodium sulfate, and spun in vacuo to remove the solvent to obtain the title compound (1.831 g, yield: 49%, yellow solid).

MS (ESI): m/z 256.1 [M+H]⁺.

### Step 5: 5-Fluoro-2-methoxy-3-(1-(methyl-d₃)-1H-1,2,4-triazol-3-yl)aniline

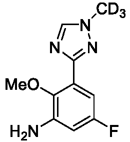

3-(5-Fluoro-2-methoxy-3-nitrophenyl)-1-(methyl-*d*₃)-1*H*-1,2,4-triazole (1.831 g, 7.18 mmol) was dissolved in methanol (50 mL), and aqueous ammonia (25 wt%, 1 mL) and palladium/carbon (305.39 mg) were added. After replacement with hydrogen, the mixture was allowed to react at room temperature for 16 hours. The reaction was stopped, the reaction solution was filtered by suction and washed using methanol, and the filtrates were merged and concentrated under reduced pressure, and then separated and purified by column chromatography (silica gel, dichloromethane:methanol = 92:8) to obtain the title compound (1.493 g, yield: 92.39%, yellow solid).

MS (ESI): m/z 226.0 [M+H]⁺.

### Step 6: ((6-Chloro-4-((5-fluoro-2-methoxy-3-(1-(methyl-d₃)-1H-1,2,4-triazol-3-yl)phenyl)amino)pyridazine-3-carbonyl)oxy)zinc (0.5)

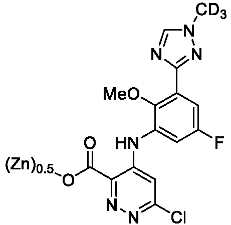

5-Fluoro-2-methoxy-3-(1-(methyl-*d*₃)-1*H*-1,2,4-triazol-3-yl)aniline (300 mg, 1.33 mmol), lithium 4,6-dichloropyridazine-3-carboxylate (318.06 mg, 1.60 mmol) and zinc acetate (293.29 mg, 1.60 mmol) were dissolved in a 4.8 mL solvent of water:toluene = 7:1. After replacement with nitrogen, the mixture was allowed to react at 100°C for 16 hours. The reaction was stopped. 6 mL of water was added to the reaction solution, and stirring was performed for one hour, followed by filtration by suction and washing using tetrahydrofuran (2 mL). The filter cake was dried to obtain the title compound (276 mg, yield: 50.12%, white solid).

MS (ESI): m/z 382.0 [M+H]⁺.

### Step 7: ((6-(Cyclopropylcarboxamido)-4-((5-fluoro-2-methoxy-3-(1-(methyl-d₃)-1H-1,2,4-triazol-3-yl)phenyl)amino)pyridazine-3-carbonyl)oxy)zinc (0.5)

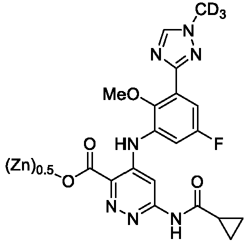

((6-Chloro-4-((5-fluoro-2-methoxy-3-(1-(methyl-*d*₃)-1*H*-1,2,4-triazol-3-yl)phenyl)amino)pyridazine-3-carbonyl)oxy)zinc (0.5) (200 mg, 0.48 mmol), cyclopropylcarboxamide (102.92 mg, 1.21 mmol), (*R*)-(S)-Cy₂PF-PtBu₂ (554.55 mg, 0.097 mmol), 1,8-diazabicyclo[5.4.0]undec-7-ene (73.64 mg, 0.48 mmol), cesium carbonate (315.22 mg, 0.97 mmol) and palladium acetate (21.72 mg, 0.097 mmol) were dissolved in a 1.8 mL solvent of toluene:acetonitrile = 2:1. After replacement with nitrogen, the mixture was allowed to react at 100°C for 16 hours. The reaction was stopped, and a 6 mL solution of water:acetic acid = 2:1 was added to the reaction solution, and extraction was performed using petroleum ether (8 mL). The lower aqueous phase was extracted three times using dichloromethane (10 mL), and the merged dichloromethane organic phase was washed with saturated brine, dried over anhydrous sodium sulfate, and spun in vacuo to remove the solvent to obtain the title compound (250 mg, purity: 77%, brown solid).

MS (ESI): m/z 431.0 [M+H]⁺.

### Step 8: 6-(Cyclopropylcarboxamido)-N-ethoxy-4-((5-fluoro-2-methoxy-3-(1-(methyl-d₃)-1H-1,2,4-triazol-3-yl)phenyl)amino)pyridazine-3-carboxamide (Compound 6)

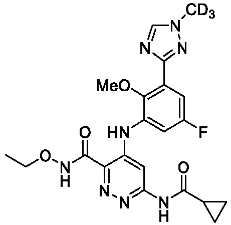

*N*-Methylpyrrolidone (6.25 mL) and acetonitrile (6.25 mL) were added to the reaction flask, followed by the addition of ((6-(cyclopropylcarboxamido)-4-((5-fluoro-2-methoxy-3-(1-(methyl-*d*₃)-1*H*-1,2,4-triazol-3-yl)phenyl)amino)pyridazine-3-carbonyl)oxy)zinc (0.5) (250 mg, 0.54 mmol), O-ethylhydroxylamine hydrochloride (79.16 mg, 0.81 mmol) and N-methylimidazole (133.25 mg, 1.62 mmol). After replacement with nitrogen, the mixture was allowed to react in an oil bath at 65°C for 15 minutes, and then 1-hydroxybenzotriazole (146.20 mg, 1.08 mmol) and 1-(3-dimethylaminopropyl)-3-ethylcarbodiimide hydrochloride (207.42 mg, 1.08 mmol) were added. After replacement with nitrogen, the reaction was continued in the oil bath at 65°C for 16 hours. The reaction was stopped, and ethyl acetate (30 mL) was added to the reaction solution, which was then washed using saturated brine. The organic phase was dried over anhydrous sodium sulfate and spun in vacuo to remove the solvent to obtain a crude product. The crude product was separated and purified by reverse phase chromatography (elution system: hydrochloric acid, water and acetonitrile) to obtain the title compound (26.59 mg, yield: 10.38%, white solid).

MS (ESI): m/z 474.0 [M+H]⁺.

### Example 7

### 6-(Cyclopropylcarboxamido)-4-((3-(1-cyclopropyl-1H-1,2,4-triazol-3-yl)-2-methoxyphenyl)amino)-N-ethoxypyridazine-3-carboxamide (Compound 7)

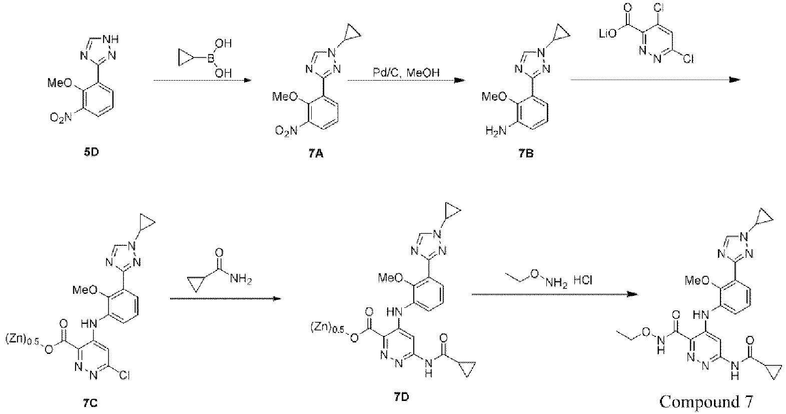

### Step 1: 1-Cyclopropyl-3-(2-methoxy-3-nitrophenyl)-1H-1,2,4-triazole

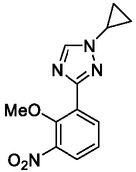

3-(2-Methoxy-3-nitrophenyl)-1*H*-1,2,4-triazole (420 mg, 1.909 mmol) was dissolved in 1,4-dioxane (15 mL), and cyclopropylboric acid (246 mg, 2.864 mmol), sodium carbonate (405 mg, 3.821 mmol), 2,2-bipyridine (298 mg, 1.908 mmol) and copper acetate (347 mg, 1.91 mmol) were added. After replacement with oxygen, the mixture was allowed to react in an oil bath at 85°C for 16 hours. The reaction was stopped, and the reaction solution was poured into water (30 mL). The aqueous phase was extracted using ethyl acetate (30 mL × 3). The merged organic phase was washed with saturated brine (30 mL × 3), dried over anhydrous sodium sulfate, and spun in vacuo to remove the solvent to obtain a crude product. The crude product was separated and purified by silica gel column chromatography (petroleum ether:ethyl acetate = 3:1) to obtain the title compound (270 mg, yield: 62.5%, yellow oily matter).

MS (ESI): m/z 261.0 [M+H]⁺.

### Step 2: 3-(1-Cyclopropyl-1H-1,2,4-triazol-3-yl)-2-methoxyaniline

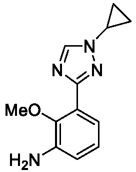

1-Cyclopropyl-3-(2-methoxy-3-nitrophenyl)-1*H*-1,2,4-triazole (270 mg, 1.037 mmol) was dissolved in methanol (10 mL), and a palladium/carbon catalyst (27 mg, 0.254 mmol) was added. After replacement with hydrogen three times, the mixture was allowed to react at room temperature for 16 hours. The reaction was stopped, filtering was performed to remove the palladium/carbon, and the filtrate was spun in vacuo to remove the solvent to obtain a crude product. The crude product was purified by silica gel column chromatography (dichloromethane:methanol = 15:1) to obtain the title compound (200 mg, yield: 83.6%, colorless and transparent oily matter).

MS (ESI): m/z 231.1 [M+H]⁺.

### Step 3: ((6-Chloro-4-((3-(1-cyclopropyl-1H-1,2,4-triazol-3-yl)-2-methoxyphenyl)amino)pyridazine-3-carbonyl)oxy)zinc (0.5)

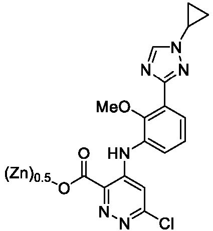

3-(1-Cyclopropyl-1*H*-1,2,4-triazol-3-yl)-2-methoxyaniline (200 mg, 0.869 mmol) was dissolved in water (7 mL) and isopropanol (1 mL), and lithium 4,6-dichloropyridazine-3-carboxylate (200 mg, 1.042 mmol) and zinc acetate (191 mg, 1.041 mmol) were added. After replacement with nitrogen three times, the mixture was allowed to react in an oil bath at 65°C for 16 hours. The reaction was stopped, and water (5 mL) was added to the reaction solution. The solution was stirred at room temperature for 1 hour and then filtered by suction. The filter cake was rinsed using water (6 mL × 2) and tetrahydrofuran (1 mL), and dried to obtain the title compound (170 mg, yield: 46.8%, yellow solid).

MS (ESI): m/z 387.0 [M+H]⁺.

### Step 4: ((6-(Cyclopropylcarboxamido)-4-((3-(1-cyclopropyl-1H-1,2,4-triazol-3-yl)-2-methoxyphenyl)amino)pyridazine-3-carbonyl)oxy)zinc (0.5)

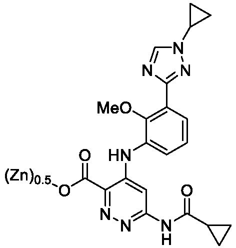

((6-Chloro-4-((3-(1-cyclopropyl-1*H*-1,2,4-triazol-3-yl)-2-methoxyphenyl)amino)pyridazine-3-carbonyl)oxy)zinc (0.5) (170 mg, 0.406 mmol) was dissolved in toluene (2 mL) and acetonitrile (1 mL), and then cyclopropylcarboxamide (86.4 mg, 1.015 mmol), (*R*)-(-)-1-[(S)-2-(dicyclohexylphosphino)ferrocene]ethyl di-tert-butylphosphine (45 mg, 0.0811 mmol), 1,8-diazabicycloundec-7-ene (61.8 mg, 0.406 mmol), cesium carbonate (265 mg, 0.813 mmol) and palladium acetate (9.12 mg, 0.0406 mmol) were added. After replacement with nitrogen, the mixture was allowed to react in an oil bath at 100°C for 16 hours. The reaction was stopped, and the reaction solution was cooled to room temperature. Water (6 mL) and acetic acid (3 mL) were added, and the mixed system was extracted using petroleum ether (10 mL × 3), and then the aqueous phase was extracted using dichloromethane (20 mL × 3), and the merged dichloromethane organic phase was washed with saturated brine (20 mL × 3), dried over anhydrous sodium sulfate, and spun in vacuo to remove the solvent to obtain the title compound (195 mg, brown solid).

MS (ESI): m/z 436.2 [M+H]⁺.

### Step 5: 6-(Cyclopropylcarboxamido)-4-((3-(1-cyclopropyl-1H-1,2,4-triazol-3-yl)-2-methoxyphenyl)amino)-N-ethoxypyridazine-3-carboxamide (Compound 7)

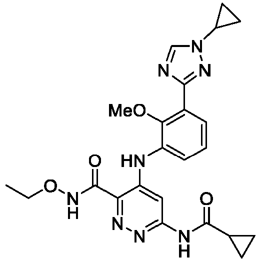

A 50 mL reaction flask was taken, into which N-methylpyrrolidone (4 mL) and acetonitrile (4 mL) were added. The mixed solvent was stirred at room temperature for 10 minutes, and then ((6-(cyclopropylcarboxamido)-4-((3-(1-cyclopropyl-1*H*-1,2,4-triazol-3-yl)-2-methoxyphenyl)amino)pyridazine-3-carbonyl)oxy)zinc (0.5) (195 mg, 0.417 mmol), O-ethylhydroxylamine hydrochloride (61 mg, 0.625 mmol) and N-methylimidazole (103 mg, 1.255 mmol) were added. The reaction solution was stirred in an oil bath at 65°C for 15 minutes, and then 1-hydroxybenzotriazole (113 mg, 0.836 mmol) and 1-ethyl-(3-dimethylaminopropyl)carbodiimide hydrochloride (160 mg, 0.836 mmol) were added. The reaction solution continued to be stirred and allowed to react in the oil bath at 65°C for 16 hours. The reaction was stopped, and water (15 mL) was added to the reaction solution for quenching. The aqueous phase was extracted using ethyl acetate (20 mL × 3), and the merged organic phase was washed with saturated brine (20 mL × 3), dried over anhydrous sodium sulfate, and spun in vacuo to remove the solvent to obtain a crude product. The crude product was separated and purified by high-performance liquid chromatography (elution system: formic acid, water and acetonitrile) to obtain the title compound (16.03 mg, yield: 8.02%, yellow solid).

MS (ESI): m/z 479.2 [M+H]⁺.

¹H NMR (400 MHz, DMSO-*d* ₆) δ 11.32 (s, 1H), 10.55 (s, 1H), 8.69 (s, 1H), 8.40 (s, 1H), 8.14 (s, 1H), 7.66 (d, *J =* 7.6 Hz, 1H), 7.51 (d, *J =* 8.0 Hz, 1H), 7.27 (t, *J =* 7.9 Hz, 1H), 3.99 (q, *J* = 7.0 Hz, 2H), 3.90 - 3.84 (m, 1H), 3.71 (s, 3H), 2.12 - 2.05 (m, 1H), 1.23 (t, *J* = 7.0 Hz, 3H), 1.17 - 1.16 (m, 2H), 1.07 - 1.06 (m, 2H), 0.86 - 0.80 (m, 4H).

### Example 8

### 6-(Cyclopropylcarboxamido)-4-((3-(1-cyclopropyl-1H-1,2,4-triazol-3-yl)-5-fluoro-2-methoxyphenyl)amino)-N-ethoxypyridazine-3-carboxamide (Compound 8)

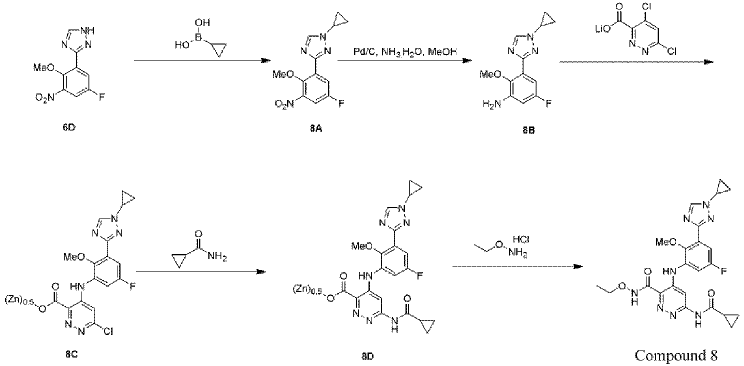

### Step 1: 1-Cyclopropyl-3-(5-fluoro-2-methoxy-3-nitrophenyl)-1H-1,2,4-triazole

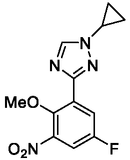

3-(5-Fluoro-2-methoxy-3-nitrophenyl)-1*H*-1,2,4-triazole (3.1 g, 13.02 mmol) was dissolved in a 1,4-dioxane solution (50 mL), and cyclopropylboric acid (1.68 g, 19.56 mmol), sodium carbonate (2.76 g, 26.04 mmol), 2,2-bipyridine (2.03 g, 13.0 mmol) and copper acetate (2.36 g, 13.0 mmol) were added. After replacement with oxygen, the mixture was allowed to react in an oil bath at 85°C for 16 hours. The reaction was stopped, and the reaction solution was poured into water (60 mL). The aqueous phase was extracted using ethyl acetate (60 mL × 3). The merged organic phase was washed with saturated brine (50 mL × 3), dried over anhydrous sodium sulfate, and spun in vacuo to remove the solvent to obtain a crude product. The crude product was separated and purified by silica gel column chromatography (petroleum ether:ethyl acetate = 3:1 to obtain the title compound (2.1 g, yield: 58.0%, yellow oily matter).

MS (ESI): m/z 279.0 [M+H]⁺.

### Step 2: 3-(1-Cyclopropyl-1H-1,2,4-triazol-3-yl)-5-fluoro-2-methoxyaniline

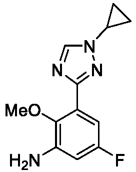

1-Cyclopropyl-3-(5-fluoro-2-methoxy-3-nitrophenyl)-1*H*-1,2,4-triazole (2.1 g, 7.55 mmol) was dissolved in methanol (50 mL), and a palladium/carbon catalyst (321 mg, 3.02 mmol) and ammonia water (1 mL) were added. After replacement with hydrogen three times, the mixture was allowed to react at room temperature for 16 hours. The reaction was stopped, filtering was performed to remove the palladium/carbon, and the filtrate was spun in vacuo to remove the solvent to obtain a crude product. The crude product was purified by silica gel column chromatography (dichloromethane:methanol = 15:1) to obtain the title compound (1.5 g, yield: 80.2%, colorless and transparent oily matter).

MS (ESI): m/z 249.2 [M+H]⁺.

### Step 3: ((6-Chloro-4-((3-(1-cyclopropyl-1H-1,2,4-triazol-3-yl)-5-fluoro-2-methoxyphenyl)amino)pyridazine-3-carbonyl)oxy)zinc (0.5)

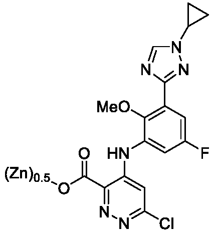

3-(1-Cyclopropyl-1*H*-1,2,4-triazol-3-yl)-5-fluoro-2-methoxyaniline (400 mg, 1.61 mmol) was dissolved in water (7 mL) and isopropanol (1 mL), and lithium 4,6-dichloropyridazine-3-carboxylate (371 mg, 1.93 mmol) and zinc acetate (355 mg, 1.93 mmol) were added. After replacement with nitrogen three times, the mixture was allowed to react in an oil bath at 100°C for 16 hours. The reaction was stopped, and water (10 mL) was added to the reaction solution. The solution was stirred at room temperature for 1 hour and then filtered by suction. The filter cake was rinsed using water (10 mL × 2) and tetrahydrofuran (3 mL) and dried to obtain the title compound (310 mg, yield: 44.1%, yellow solid).

MS (ESI): m/z 405.2 [M+H]⁺.

### Step 4: ((6-(Cyclopropylcarboxamido)-4-((3-(1-cyclopropyl-1H-1,2,4-triazol-3-yl)-5-fluoro-2-methoxyphenyl)amino)pyridazine-3-carbonyl)oxy)zinc (0.5)

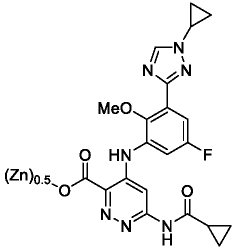

((6-Chloro-4-((3-(1-cyclopropyl-1*H*-1,2,4-triazol-3-yl)-5-fluoro-2-methoxyphenyl)amino)pyridazine-3-carbonyl)oxy)zinc (0.5) (160 mg, 0.367 mmol) was dissolved in toluene (2 mL) and acetonitrile (1 mL), and cyclopropylcarboxamide (78.1 mg, 0.918 mmol), (*R*)-(-)-1-[(S)-2-(dicyclohexylphosphino)ferrocene]ethyl di-tert-butylphosphine (40.7 mg, 0.073 mmol), 1,8-diazabicycloundec-7-ene (55.9 mg, 0.367 mmol), cesium carbonate (239 mg, 0.736 mmol) and palladium acetate (8.24 mg, 0.0367 mmol) were added. After replacement with nitrogen, the mixture was allowed to react in an oil bath at 100°C for 16 hours. The reaction was stopped, the reaction solution was cooled to room temperature, and water (6 mL) and acetic acid (3 mL) were added. The mixed system was extracted using petroleum ether (10 mL × 3), and then the aqueous phase was extracted using dichloromethane (20 mL × 3), and the merged dichloromethane organic phase was washed with saturated brine (20 mL × 3), dried over anhydrous sodium sulfate, and spun in vacuo to remove the solvent to obtain the title compound (168 mg, yield: 94.5%, brown solid).

MS (ESI): m/z 454.3 [M+H]⁺.

### Step 5: 6-(Cyclopropylcarboxamido)-4-((3-(1-cyclopropyl-1H-1,2,4-triazol-3-yl)-5-fluoro-2-methoxyphenyl)amino)-N-ethoxypyridazine-3-carboxamide (Compound 8)

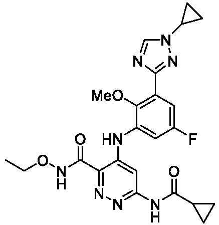

A 50 mL reaction flask was taken, into which *N*-methylpyrrolidone (3 mL) and acetonitrile (3 mL) were added. The mixed solvent was stirred at room temperature for 10 minutes, and then ((6-(cyclopropylcarboxamido)-4-((3-(1-cyclopropyl-1*H*-1,2,4-triazol-3-yl)-5-fluoro-2-methoxyphenyl)amino)pyridazine-3-carbonyl)oxy)zinc (0.5) (130 mg, 0.268 mmol), O-ethylhydroxylamine hydrochloride 12 (39.2 mg, 0.402 mmol) and *N*-methylimidazole (66 mg, 0.804 mmol) were added. The reaction solution was stirred in an oil bath at 65°C for 15 minutes, and then 1-hydroxybenzotriazole (72.4 mg, 0.536 mmol) and 1-ethyl-(3-dimethylaminopropyl)carbodiimide hydrochloride (103 mg, 0.537 mmol) were added. The reaction solution was stirred and allowed to react in an oil bath at 65°C for 16 hours. The reaction was stopped, and water (10 mL) was added to the reaction solution for quenching. The aqueous phase was extracted using ethyl acetate (15 mL × 3), and the merged organic phase was washed with saturated brine (15 mL × 3), dried over anhydrous sodium sulfate, and spun in vacuo to remove the solvent to obtain a crude product. The crude product was separated and purified by high-performance liquid chromatography (elution system: formic acid, water and acetonitrile) to obtain the title compound (7.54 mg, yield: 5.7%, yellow solid).

MS (ESI): m/z 497.2 [M+H]⁺.

### Example 9

### 6-(Cyclopropylcarboxamido)-4-((3-(1-cyclopropyl-1H-1,2,4-triazol-3-yl)-2-(methoxy-d3)phenyl)amino)-N-ethoxypyridazine-3-carboxamide (Compound 9)

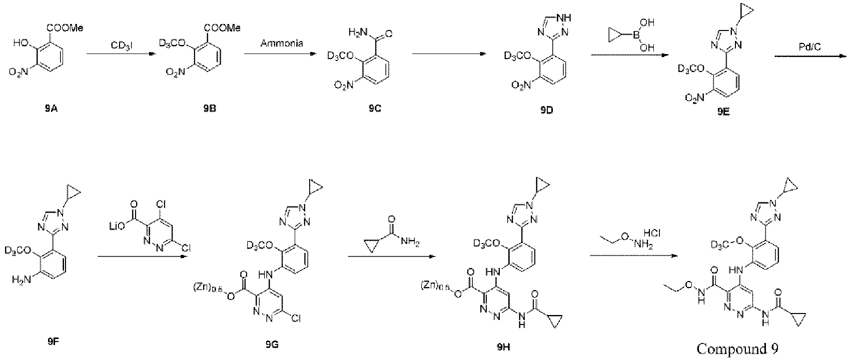

### Step 1: Methyl 2-(methoxy-d₃)-3-nitrobenzoate

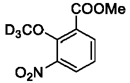

Methyl 2-hydroxy-3-nitrobenzoate (1.7 g, 8.623 mmol) was dissolved in *N,N-*dimethylformamide (20 mL), and deuterated iodomethane (1.87 g, 12.9 mmol) and potassium carbonate (2.38 g, 17.221 mmol) were added. After replacement with nitrogen, the mixture was allowed to react in an oil bath at 60°C for 2 hours. The reaction was stopped, and the reaction solution was poured into water (50 mL). The aqueous phase was extracted using ethyl acetate (50 mL × 3). The merged organic phase was washed with saturated brine (30 mL × 3), dried over anhydrous sodium sulfate, and spun in vacuo to remove the solvent to obtain a crude product. The crude product was separated and purified by silica gel column chromatography (petroleum ether:ethyl acetate = 5:1) to obtain the title compound (1.69 g, yield: 91.5%, yellow oily matter).

MS (ESI): m/z 215.1 [M+H]⁺.

### Step 2: 2-(Methoxy-d₃)-3-nitrobenzamide

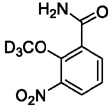

Methyl 2-(methoxy-*d*₃)-3-nitrobenzoate (1.69 g, 7.89 mmol) was dissolved in a methanolic ammonia solution (30 mL), and aqueous ammonia (15 mL) was added. After replacement with nitrogen, the mixture was allowed to react at room temperature for 16 hours. The reaction was stopped, and the reaction solution was spun in vacuo to remove the solvent to obtain a crude product. The crude product was separated and purified by silica gel column chromatography (dichloromethane:methanol = 20:1) to obtain the title compound (1.45 g, yield: 92.2%, yellow solid).

MS (ESI): m/z 200.1 [M+H]⁺.

### Step 3: 3-(2-(Methoxy-d₃)-3-nitrophenyl)-1H-1,2,4-triazole

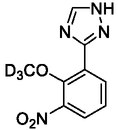

2-(Methoxy-*d*₃)-3-nitrobenzamide (1.45 g, 7.28 mmol) was dissolved in *N,N-*dimethylformamide dimethyl acetal (10 mL). After replacement with nitrogen, the mixture was allowed to react in an oil bath at 95°C for 1 hour. The reaction solution was concentrated under reduced pressure to obtain a crude product, and the crude product was dissolved in ethanol (10 mL) for later use. A 250 mL single-necked flask was taken, into which ethanol (40 mL) and acetic acid (10 mL) were added. The system was cooled to 0°C, and hydrazine hydrate (4 mL) was added dropwise, and stirring was performed at 0°C for 1 hour. The crude product ethanol solution was added dropwise to the system, which continued to be stirred for 2 hours. The reaction was stopped, and the reaction solution was concentrated under reduced pressure to obtain a crude product. The crude product was dissolved in ethyl acetate (200 mL). The organic phase was washed with a saturated aqueous sodium bicarbonate solution (50 mL × 3), dried over anhydrous sodium sulfate, and concentrated under reduced pressure to obtain a crude product. The crude product was purified by silica gel column chromatography (dichloromethane:methanol = 10:1) to obtain the title compound (1.1 g, yield: 67.7%, light yellow solid).

MS (ESI): m/z 224.1 [M+H]⁺.

### Step 4: 1-Cyclopropyl-3-(2-(methoxy-d₃)-3-nitrophenyl)-1H-1,2,4-triazole

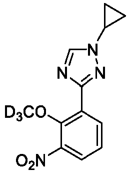

3-(2-(Methoxy-*d*₃)-3-nitrophenyl)-1*H*-1,2,4-triazole (1.1 g, 4.928 mmol) was dissolved in 1,4-dioxane (40 mL), and cyclopropylboronic acid (635 mg, 7.392 mmol), sodium carbonate (1.04 g, 9.812 mmol), 2,2-bipyridine (770 mg, 4.93 mmol) and copper acetate (895 mg, 4.928 mmol) were added. After replacement with oxygen, the mixture was allowed to react in an oil bath at 85°C for 16 hours. The reaction was stopped, and the reaction solution was poured into water (50 mL). The aqueous phase was extracted using ethyl acetate (50 mL × 3). The merged organic phase was washed with saturated brine (50 mL × 3), dried over anhydrous sodium sulfate, and spun in vacuo to remove the solvent to obtain a crude product. The crude product was separated and purified by silica gel column chromatography (petroleum ether:ethyl acetate = 1:1) to obtain the title compound (900 mg, yield: 69.2%, yellow oily matter).

MS (ESI): m/z 264.0 [M+H]⁺.

### Step 5: 3-(1-Cyclopropyl-1H-1,2,4-triazol-3-yl)-2-(methoxy-d₃)aniline

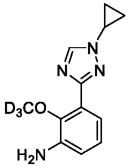

1-Cyclopropyl-3-(2-(methoxy-*d*₃)-3-nitrophenyl)-1*H*-1,2,4-triazole (900 mg, 3.419 mmol) was dissolved in methanol (25 mL), and a palladium/carbon catalyst (90 mg) was added. After replacement with hydrogen three times, the mixture was allowed to react at room temperature for 16 hours. The reaction was stopped, the reaction solution was filtered, and the filtrate was spun in vacuo to remove the solvent to obtain a crude product. The crude product was purified by silica gel column chromatography (dichloromethane:methanol = 15:1) to obtain the title compound (520 mg, yield: 65.2%, colorless and transparent oily matter).

MS (ESI): m/z 234.2 [M+H]⁺.

### Step 6: ((6-Chloro-4-((3-(1-cyclopropyl-1H-1,2,4-triazol-3-yl)-2-(methoxy-d₃)phenyl)amino)pyridazine-3-carbonyl)oxy)zinc (0.5)

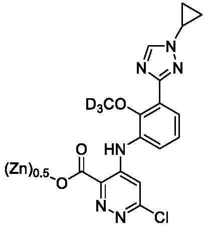

3-(1-Cyclopropyl-1*H*-1,2,4-triazol-3-yl)-2-(methoxy-*d*₃)aniline (520 mg, 2.229 mmol) was dissolved in water (14 mL) and isopropanol (2 mL), and lithium 4,6-dichloropyridazine-3-carboxylate (530 mg, 2.677 mmol) and zinc acetate (491 mg, 2.676 mmol) were added. After replacement with nitrogen three times, the mixture was allowed to react in an oil bath at 65°C for 16 hours. The reaction was stopped, and water (10 mL) was added to the reaction solution. The solution was stirred at room temperature for 1 hour and then filtered by suction. The filter cake was rinsed using water (15 mL × 3) and tetrahydrofuran (4 mL) and then dried to obtain the title compound (680 mg, yield: 72.3%, white solid).

MS (ESI): m/z 390.2 [M+H]⁺.

### Step 7: ((6-(Cyclopropylcarboxamido)-4-((3-(1-cyclopropyl-1H-1,2,4-triazol-3-yl)-2-(methoxy-d₃)phenyl)amino)pyridazine-3-carbonyl)oxy)zinc (0.5)

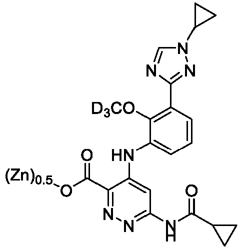

((6-Chloro-4-((3-(1-cyclopropyl-1*H*-1,2,4-triazol-3-yl)-2-(methoxy-*d*₃)phenyl)amino)pyridazine-3-carbonyl)oxy)zinc (0.5) (680 mg, 1.613 mmol) was dissolved in toluene (8 mL) and acetonitrile (4 mL), and cyclopropylcarboxamide (343 mg, 4.031 mmol), (R)-(-)-1-[(S)-2-(dicyclohexylphosphino)ferrocene]ethyl di-tert-butylphosphine (179 mg, 0.323 mmol), 1,8-diazabicycloundec-7-ene (246 mg, 1.616 mmol), cesium carbonate (1.05 g, 3.223 mmol) and palladium acetate (72.4 mg, 0.322 mmol) were added. After replacement with nitrogen, the mixture was allowed to react in an oil bath at 100°C for 16 hours. The reaction was stopped, the reaction solution was cooled to room temperature, and water (20 mL) and acetic acid (3 mL) were added. The mixed system was extracted using petroleum ether (30 mL × 3), then the aqueous phase was extracted using dichloromethane (50 mL × 3), and the merged dichloromethane organic phase was washed with saturated brine (50 mL × 3), dried over anhydrous sodium sulfate, and spun in vacuo to remove the solvent to obtain the title compound (720 mg, yield: 94.9%, brown solid).

MS (ESI): m/z 439.3 [M+H]⁺.

### Step 8: 6-(Cyclopropylcarboxamido)-4-((3-(1-cyclopropyl-1H-1,2,4-triazol-3-yl)-2-(methoxy-d₃)phenyl)amino)-N-ethoxypyridazine-3-carboxamide (Compound 9)

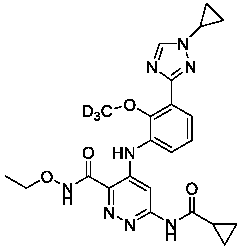

A 50 mL reaction flask was taken, into which N-methylpyrrolidone (4 mL) and acetonitrile (4 mL) were added. The mixed solvent was stirred at room temperature for 10 minutes, and then ((6-(cyclopropylcarboxamido)-4-((3-(1-cyclopropyl-1*H*-1,2,4-triazol-3-yl)-2-(methoxy-*d*₃)phenyl)amino)pyridazine-3-carbonyl)oxy)zinc (0.5) (370 mg, 0.787 mmol), *O-*ethylhydroxylamine hydrochloride (153.5 mg, 1.574 mmol) and N-methylimidazole (193.8 mg, 2.361 mmol) were added. The reaction solution was stirred in an oil bath at 65°C for 15 minutes, and then 1-hydroxybenzotriazole (212.7 mg, 1.574 mmol) and 1-ethyl-(3-dimethylaminopropyl)carbodiimide hydrochloride (301.7 mg, 1.574 mmol) were added. The reaction solution continued to be stirred and allowed to react in the oil bath at 65°C for 3 hours. The reaction was stopped, and water (30 mL) was added to the reaction solution. The aqueous phase was extracted using ethyl acetate (40 mL × 3), and the merged organic phase was washed with saturated brine (40 mL×3), dried over anhydrous sodium sulfate, and spun in vacuo to remove the solvent to obtain a crude product. The crude product was separated and purified by high-performance liquid chromatography (elution system: formic acid, water and acetonitrile) to obtain the title compound (88.36 mg, yield: 23.3%, pink solid).

MS (ESI): m/z 482.1 [M+H]⁺.

¹H NMR (400 MHz, DMSO-*d*₆) δ 11.32 (s, 1H), 10.55 (s, 1H), 8.69 (s, 1H), 8.38 (s, 1H), 8.14 (s, 1H), 7.66 (d, *J =* 7.8 Hz, 1H), 7.51 (d, *J =* 7.9 Hz, 1H), 7.27 (t, *J =* 7.9 Hz, 1H), 4.02 - 3.96 (m, 2H), 3.89 - 3.85 (m, 1H), 2.11 - 2.07 (m, 1H), 1.23 (t, *J* = 7.0 Hz, 3H), 1.17 - 1.15 (m, 2H), 1.09 - 1.05 (m, 2H), 0.84 - 0.80 (m, 4H).

### Example 10

### 6-(Cyclopropylcarboxamido)-N-(2-fluoroethoxy)-4-((2-methoxy-3-(1-methyl-1H-1,2,4-triazol-3-yl)phenyl)amino)pyridazine-3-carboxamide (Compound 10)

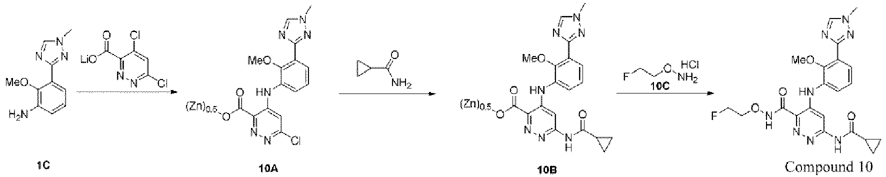

### Synthesis of intermediate 10C

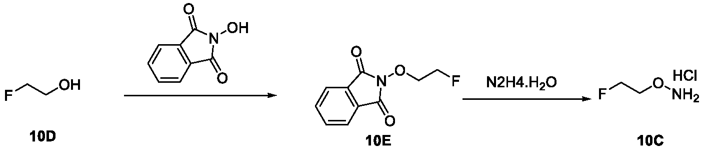

### Step 1: 2-(2-Fluoroethoxy)isoindoline-1,3-dione

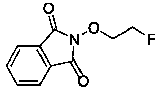

2-Fluoroethane-1-ol (5.0 g, 78.052 mmol) was dissolved in an ultra-dry tetrahydrofuran solution (50 mL), and 2-hydroxyisoindoline-1,3-dione (12733 mg, 78.052 mmol) and triphenylphosphine (26.614 g, 101.468 mmol) were added. After replacement with nitrogen, diethyl azodicarboxylate (17.671 g, 101.468 mmol) was added dropwise at 0°C. After the addition was complete, the mixture was allowed to react at room temperature for 16 hours. The reaction was stopped, and the reaction solution was filtered and then spun dry. The crude product was separated and purified by silica gel column chromatography (petroleum ether:ethyl acetate = 3:1) to obtain the title compound (8.4 g, yield: 51.4%, white solid).

¹H NMR (400 MHz, DMSO-*d*₆) δ 7.89 - 7.83 (m, 4H), 4.78 - 4.74 (m, 1H), 4.66 - 4.62 (m, 1H), 4.46 - 4.41 (m, 1H), 4.38 - 4.34 (m, 1H).

### Step 2: O-(2-Fluoroethyl)hydroxylamine hydrochloride

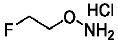

2-(2-Fluoroethoxy)isoindoline-1,3-dione (8.4 g, 40.157 mmol) was dissolved in a dichloromethane solution (50 mL), and hydrazine hydrate (4.016 g, 80.314 mmol) was added dropwise. After replacement with nitrogen, the mixture was allowed to react at room temperature for 16 hours. The reaction was stopped, the reaction solution was filtered, the filtrate was washed using water (20 mL × 2), and the aqueous phase was collected. The aqueous phase was extracted using a mixed solution of chloroform and isopropanol (3:1) (30 mL × 6). The organic phases were merged and then a solution of hydrogen chloride in dioxane (30 mL) was added. The resulting solution was stirred at room temperature for 30 minutes and then spun dry to obtain the title compound (1.7 g, yield: 36.6%, white solid).

### Synthesis of compound 10

### Step 1: ((6-Chloro-4-((2-methoxy-3-(1-methyl-1H-1,2,4-triazol-3-yl)phenyl)amino)pyridazine-3-carbonyl)oxy)zinc (0.5)

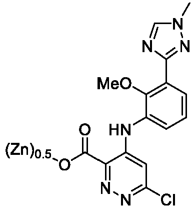

2-Methoxy-3-(1-methyl-1*H*-1,2,4-triazol-3-yl)aniline (500 mg, 2.448 mmol) was dissolved in water (35 mL) and isopropanol (5 mL), and lithium 4,6-dichloropyridazine-3-carboxylate (974 mg, 4.896 mmol) and zinc acetate (898 mg, 4.896 mmol) were added. After replacement with nitrogen three times, the mixture was allowed to react in an oil bath at 65°C for 16 hours. The reaction was stopped, and water (15 mL) was added to the reaction solution. The solution was stirred at room temperature for 1 hour and then filtered by suction. The filter cake was rinsed using water (10 mL × 2) and tetrahydrofuran (1 mL) to obtain the title compound (530 mg, yield: 55.2%, brown solid).

MS (ESI): m/z 361.0 [M+H]⁺.

### Step 2: ((6-(Cyclopropylcarboxamido)-4-((2-methoxy-3-(1-methyl-1H-1,2,4-triazol-3-yl)phenyl)amino)pyridazine-3-carbonyl)oxy)zinc (0.5)

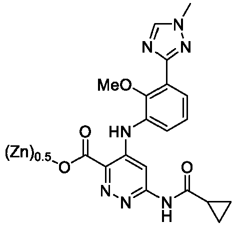

((6-Chloro-4-((2-methoxy-3-(1-methyl-1*H*-1,2,4-triazol-3-yl)phenyl)amino)pyridazine-3-carbonyl)oxy)zinc (0.5) (480 mg, 1.223 mmol) was dissolved in toluene (8 mL) and acetonitrile (4 mL), and cyclopropylcarboxamide (260 mg, 3.058 mmol), (*R*)-(-)-1-[(S)-2-(dicyclohexylphosphino)ferrocene]ethyl di-*tert*-butylphosphine (136 mg, 0.245 mmol), 1,8-diazabicycloundec-7-ene (186 mg, 1.223 mmol), potassium carbonate (338 mg, 2.446 mmol) and palladium acetate (55 mg, 0.245 mmol) were added. After replacement with nitrogen, the mixture was allowed to react in an oil bath at 75°C for 16 hours. The reaction was stopped, and the reaction solution was cooled to room temperature. Water (15 mL) and acetic acid (7 mL) were added, and the mixed system was extracted using petroleum ether (30 mL × 2), and then the aqueous phase was extracted using dichloromethane (30 mL × 2), and the merged dichloromethane organic phase was washed with saturated brine (15 mL × 3), dried over anhydrous sodium sulfate, and spun in vacuo to remove the solvent to obtain the title compound (500 mg, yield: 81.8%, brown solid).

MS (ESI): m/z 410.1 [M+H]⁺.

### Step 3: 6-(Cyclopropylcarboxamido)-N-(2-fluoroethoxy)-4-((2-methoxy-3-(1-methyl-1H-1,2,4-triazol-3-yl)phenyl)amino)pyridazine-3-carboxamide (Compound 10)

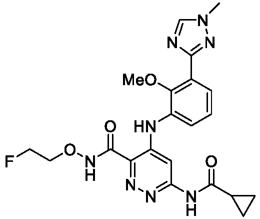

A 50 mL reaction flask was taken, into which *N*-methylpyrrolidone (2 mL) and acetonitrile (2 mL) were added. The mixed solvent was stirred at room temperature for 10 minutes, and then ((6-(cyclopropylcarboxamido)-4-((2-methoxy-3-(1-methyl-1*H*-1,2,4-triazol-3-yl)phenyl)amino)pyridazine-3-carbonyl)oxy)zinc (0.5) (200 mg, 0.453 mmol), *O*-(2-fluoroethyl)hydroxylamine hydrochloride (79 mg, 0.680 mmol) and *N*-methylimidazole (111 mg, 1.359 mmol) were added. The reaction solution was stirred in an oil bath at 65°C for 15 minutes, and then 1-hydroxybenzotriazole (122 mg, 0.906 mmol) and 1-ethyl-(3-dimethylaminopropyl)carbodiimide hydrochloride (174 mg, 0.906 mmol) were added. The reaction solution was stirred in an oil bath at 65°C for 16 hours. The reaction was stopped, and water (10 mL) was added to the reaction solution, and extraction was performed using ethyl acetate (15 mL × 3). The organic phases were merged, dried over anhydrous sodium sulfate, filtered, and spun dry. The crude product was separated and purified by high-performance liquid chromatography (elution system: formic acid, water and acetonitrile) to obtain the title compound (7.64 mg, yield: 3.6%, grey solid).

MS (ESI): m/z 471.0 [M+H]⁺.

¹H NMR (400 MHz, DMSO-*d*₆) δ 8.56 (s, 1H), 8.40 (s, 1H), 8.15 (s, 1H), 7.66 (d, *J* = 7.7 Hz, 1H), 7.51 (d, *J* = 7.1 Hz, 1H), 7.28 (d, *J* = 7.9 Hz, 1H), 4.69 (d, *J* = 48.0 Hz, 2H), 4.29 - 4.07 (m, 2H), 3.93 (s, 3H), 3.73 (s, 3H), 2.09 (brs, 1H), 0.82 - 0.77 (m, 4H).

### Example 11

### 6-(Cyclopropylcarboxamido)-N-(2,2-difluoroethoxy)-4-((2-methoxy-3-(1-methyl-1H-1,2,4-triazol-3-yl)phenyl)amino)pyridazine-3-carboxamide (Compound 11)

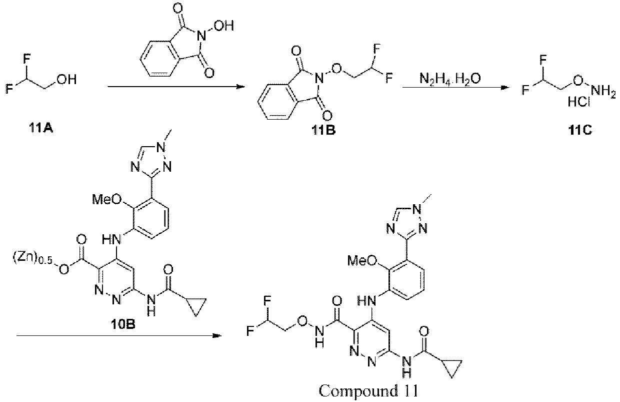

### Step 1: 2-(2,2-difluoroethoxy)isoindole-1,3-dione

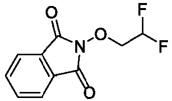

2,2-Difluoroethane-1-ol (5.0 g, 60.938 mmol) was dissolved in an ultra-dry tetrahydrofuran solution (50 mL), and 2-hydroxyisoindoline-1,3-dione (9.941 g, 60.938 mmol) and triphenylphosphine (20.778 g, 79.219 mmol) were added. After replacement with nitrogen, diethyl azodicarboxylate (13.796 g, 79.219 mmol) was added dropwise at 0°C, and the mixture was allowed to react at room temperature for 16 hours. The reaction was stopped, the reaction solution was filtered, and then the filtrate was spun dry in vacuo. The crude product was separated and purified by silica gel column chromatography (petroleum ether:ethyl acetate = 6:1) to obtain the title compound (11 g, yield: 79.5%, white solid)

¹H NMR (400 MHz, CDCl₃) δ 7.90 - 7.84 (m, 2H), 7.81 - 7.76 (m, 2H), 6.40 - 6.06 (m, 1H), 4.38 (td, *J =* 12.7, 4.2 Hz, 2H).

### Step 2: O-(2,2-Difluoroethyl)hydroxylamine hydrochloride

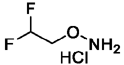

2-(2,2-Difluoroethoxy)isoindole-1,3-dione (10.0 g, 44.020 mmol) was dissolved in a dichloromethane solution (50 mL), and hydrazine hydrate (4.402 g, 88.040 mmol) was added dropwise. After replacement with nitrogen, the mixture was allowed to react at room temperature for 16 hours. The reaction was stopped, the reaction solution was filtered, the filtrate was washed using water (50 mL × 2), and the aqueous phase was collected. The aqueous phase was then extracted using a mixed solution of chloroform and isopropanol (3:1) (30 mL × 6). The organic phases were merged and then a solution of hydrogen chloride in dioxane (20 mL) was added. The resulting solution was stirred at room temperature for 1 hour and then spun dry to obtain the title compound (1.1 g, yield: 18.7%, white solid).

### Step 3: 6-(Cyclopropylcarboxamido)-N-(2,2-difluoroethoxy)-4-((2-methoxy-3-(1-methyl-1H-1,2,4-triazol-3-yl)phenyl)amino)pyridazine-3-carboxamide (Compound 11)

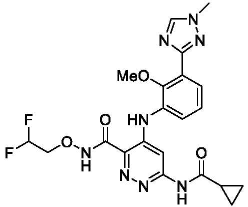

*N*-Methylpyrrolidone (2 mL) and acetonitrile (2 mL) were added to a reaction flask, and the mixed solvent was stirred at room temperature for 10 minutes. Then, ((6-(cyclopropylcarboxamido)-4-((2-methoxy-3-(1-methyl-1*H*-1,2,4-triazol-3-yl)phenyl)amino)pyridazine-3-carbonyl)oxy)zinc (0.5) (200 mg, 0.453 mmol), *O*-(2,2-difluoroethyl)hydroxylamine hydrochloride (91 mg, 0.680 mmol) and *N*-methylimidazole (111 mg, 1.359 mmol) were added. The reaction solution was stirred in an oil bath at 65°C for 15 minutes, and then 1-hydroxybenzotriazole (122 mg, 0.906 mmol) and 1-ethyl-(3-dimethylaminopropyl)carbodiimide hydrochloride (174 mg, 0.906 mmol) were added. The reaction was continued for 16 hours while stirring in an oil bath at 65°C. The reaction was stopped, water (10 mL) was added to the reaction solution, and extraction was performed using ethyl acetate (10 mL × 3). The organic phases were merged, dried over anhydrous sodium sulfate, filtered, and spun dry. The crude product was separated and purified by high-performance liquid chromatography (elution system: formic acid, water and acetonitrile) to obtain the title compound (15.64 mg, yield: 7.1%, white solid).

MS (ESI): m/z 489.2 [M+H]⁺.

¹H NMR (400 MHz, DMSO-*d*₆) δ 8.57 (s, 1H), 8.43 (s, 1H), 8.13 (s, 1H), 7.65 (d, *J* = 8.0 Hz, 1H), 7.50 (s, 1H), 7.27 (t, *J* = 8.0 Hz, 1H), 6.36 (t, *J* = 54.2 Hz, 1H), 4.18 (d, *J* = 14.3 Hz, 2H), 3.93 (s, 3H), 3.73 (s, 3H), 2.09 (brs, 1H), 0.82 (brs, 4H).

### Example 12

### 6-(Cyclopropanamido)-4-((3-(1-cyclopropyl-1H-1,2,4-triazol-3-yl)-2-methoxyphenyl)amino)-N-(2-fluoroethoxy)pyridazine-3-carboxamide (Compound 12)

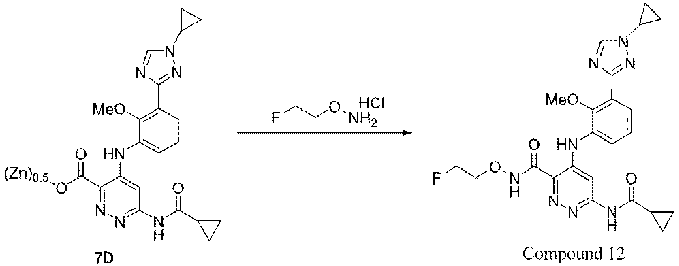

*N*-Methylpyrrolidone (4 mL) and acetonitrile (4 mL) were added to a reaction flask, and the mixed solvent was stirred at room temperature for 10 minutes. Then, ((6-(cyclopropylcarboxamido)-4-((3-(1-cyclopropyl-1*H*-1,2,4-triazol-3-yl)-2-methoxyphenyl)amino)pyridazine-3-carbonyl)oxy)zinc (0.5) (270 mg, 0.578 mmol), *O*-(2-fluoroethyl)hydroxylamine hydrochloride (133.6 mg, 1.156 mmol) and *N*-methylimidazole (142.4 mg, 1.734 mmol) were added. The reaction solution was stirred in an oil bath at 65°C for 15 minutes, and then 1-hydroxybenzotriazole (156.2 mg, 1.156 mmol) and 1-ethyl-(3-dimethylaminopropyl)carbodiimide hydrochloride (221.6 mg, 1.156 mmol) were added. The reaction solution was stirred and allowed to react in an oil bath at 65°C for 16 hours. The reaction was stopped, and the reaction solution was poured into water (30 mL). The aqueous phase was extracted using ethyl acetate (30 mL × 3), and the merged organic phase was washed with saturated brine (30 mL × 3), dried over anhydrous sodium sulfate, and spun in vacuo to remove the solvent to obtain a crude product. The crude product was separated and purified by high-performance liquid chromatography (elution system: trifluoroacetic acid, water and acetonitrile) to obtain the title compound (11.3 mg, yield: 3.94%, yellow solid).

MS (ESI): m/z 497.1 [M+H]⁺.

¹H NMR (400 MHz, DMSO-*d*₆) δ 12.46 (s, 1H), 11.34 (s, 1H), 10.50 (s, 1H), 8.69 (s, 1H), 8.14 (s, 1H), 7.68 - 7.65 (m, 1H), 7.51 (dd, *J* = 7.9, 1.3 Hz, 1H), 7.26 (d, *J* = 7.9 Hz, 1H), 4.76 - 4.74 (m, 1H), 4.64 - 4.62 (m, 1H), 4.25 - 4.23 (m, 1H), 4.17 -4.16 (m, 1H), 3.88 - 3.86 (m, 1H), 3.71 (s, 3H), 2.10 - 2.06 (m, 1H), 1.18 - 1.16 (m, 2H), 1.07 (dd, *J* = 6.9, 1.7 Hz, 2H), 0.83 - 0.81 (m, 4H).

### Example 13

### 6-(Cyclopropylcarboxamido)-4-((3-(1-cyclopropyl-1H-1,2,4-triazol-3-yl)-2-methoxyphenyl)amino)-N-(2,2-difluoroethoxy)pyridazine-3-carboxamide (Compound 13)

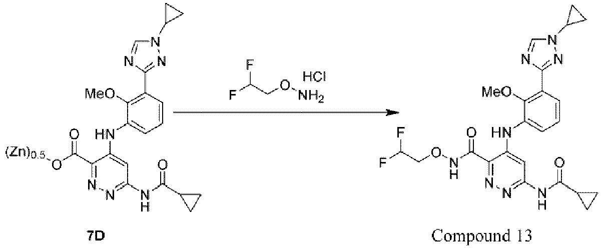

*N*-Methylpyrrolidone (4 mL) and acetonitrile (4 mL) were added to a reaction flask, and the mixed solvent was stirred at room temperature for 10 minutes. Then, ((6-(cyclopropylcarboxamido)-4-((3-(1-cyclopropyl-1*H*-1,2,4-triazol-3-yl)-2-methoxyphenyl)amino)pyridazine-3-carbonyl)oxy)zinc (0.5) (230 mg, 0.492 mmol), O-(2,2-difluoroethyl)hydroxylamine hydrochloride (194.8 mg, 1.476 mmol) and *N*-methylimidazole (121.2 mg, 1.476 mmol) were added. The reaction solution was stirred in an oil bath at 65°C for 15 minutes, and then 1-hydroxybenzotriazole (133 mg, 0.984 mmol) and 1-ethyl-(3-dimethylaminopropyl)carbodiimide hydrochloride (189 mg, 0.986 mmol) were added. The reaction solution was stirred and allowed to react in an oil bath at 65°C for 16 hours. The reaction was stopped, and the reaction solution was poured into water (30 mL). The aqueous phase was extracted using ethyl acetate (30 mL × 3), and the merged organic phase was washed with saturated brine (30 mL × 3), dried over anhydrous sodium sulfate, and spun in vacuo to remove the solvent to obtain a crude product. The crude product was separated and purified by high-performance liquid chromatography (elution system: hydrochloric acid, water and acetonitrile) to obtain the title compound (17.76 mg, yield: 7.01%, yellow solid).

MS (ESI): m/z 515.0 [M+H]⁺.

¹H NMR (400 MHz, DMSO-*d* ₆) δ 12.68 (s, 1H), 11.42 (s, 1H), 10.47 (s, 1H), 8.74 (s, 1H), 8.10 (s, 1H), 7.68 (d, *J* = 7.9 Hz, 1H), 7.52 (d, *J* = 7.6 Hz, 1H), 7.28 (t, *J* = 7.9 Hz, 1H), 6.50 - 6.21 (m, 1H), 4.26 - 4.19 (m, 2H), 3.90 - 3.86 (m, 1H), 3.72 (s, 3H), 2.11 - 2.05 (m, 1H), 1.17 (d, *J* = 3.2 Hz, 2H), 1.07 (d, *J* = 5.4 Hz, 2H), 0.82 (brs, 4H).

### Example 14

### 6-(Cyclopropylcarboxamido)-N-(methoxy-d₃)-4-((2-methoxy-3-(1-methyl-1H-1,2,4-triazol-3-yl)phenyl)amino)pyridazine-3-carboxamide (Compound 14)

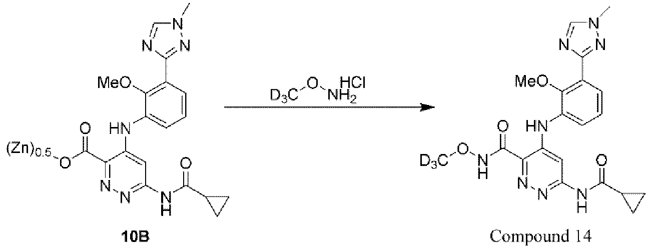

((6-(Cyclopropylcarboxamido)-4-((2-methoxy-3-(1-methyl-1*H*-1,2,4-triazol-3-yl)phenyl)amino)pyridazine-3-carbonyl)oxy)zinc (0.5) (130 mg, 0.29 mmol) and *O*-(methyl-*d*₃)hydroxylamine hydrochloride (38.26 mg, 0.44 mmol) were dissolved in *N*-methylpyrrolidone (1 mL) and acetonitrile (1 mL), and N-methylimidazole (72.34 mg, 0.88 mmol) was added. The reaction temperature was raised to 65°C and the solution was stirred for 15 minutes. Then, 1-hydroxybenzotriazole (79.67 mg, 0.59 mmol) and 1-(3-dimethylaminopropyl)-3-ethylcarbodiimide hydrochloride (113.02 mg, 0.59 mmol) were added, and the reaction was continued at that temperature for 2 hours. The reaction was stopped, and the reaction solution was separated and purified by reverse phase preparative chromatography (elution system: formic acid, acetonitrile and water) to obtain the title compound (27.15 mg, yield: 20.8%, white solid).

MS (ESI): m/z 442.3 [M+H]⁺.

¹H NMR (400 MHz, DMSO-*d*₆) δ 12.35 (s, 1H), 11.32 (s, 1H), 10.55 (s, 1H), 8.56 (s, 1H), 8.15 (s, 1H), 7.68 - 7.66 (m, 1H), 7.52 - 7.50 (m, 1H), 7.29 - 7.25 (m, 1H), 3.95 (s, 3H), 3.73 (s, 3H), 2.12 - 2.05 (m, 1H), 0.83 - 0.81 (m, 4H).

### Example 15

### 6-(Cyclopropylcarboxamido)-N-(ethoxy-d₅)-4-((2-methoxy-3-(1-methyl-1H-1,2,4-triazol-3-yl)phenyl)amino)pyridazine-3-carboxamide (Compound 15)

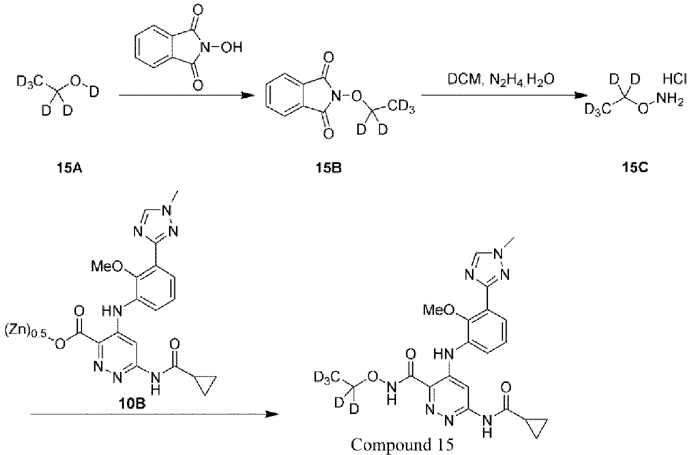

### Step 1: 2-(Ethoxy-d₅)isoindole-1,3-dione

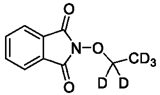

2-Hydroxyisoindoline-1,3-dione (5.5 g, 33.715 mmol) was dissolved in tetrahydrofuran (45 mL), and ethane-1,1,2,2,2-*d*₅-1-ol-*d* (1.76 g, 33.775 mmol) and triphenylphosphine (11.5 g, 43.845 mmol) were added. After replacement with nitrogen, diethyl azodicarboxylate (7.63 g, 43.813 mmol) was slowly added dropwise in an ice bath. The reaction solution was stirred and allowed to react at room temperature for 16 hours. The reaction was stopped, and the reaction solution was concentrated under reduced pressure to obtain a crude product. The crude product was separated and purified by silica gel column chromatography (petroleum ether:ethyl acetate = 3:1) to obtain the title compound (2.79 g, yield: 42.1%, white solid).

MS (ESI): m/z 197.0 [M+H]⁺.

### Step 2: O-(Ethyl-d₅)hydroxylamine hydrochloride

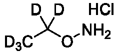

2-(Ethoxy-*d*₅)isoindole-1,3-dione (2.7 g, 13.76 mmol) was dissolved in dichloromethane (30 mL), and hydrazine hydrate (1.38 g, 27.57 mmol) was added. After replacement with nitrogen, the reaction solution was stirred and allowed to react at room temperature for 16 hours. The reaction was stopped, the reaction solution was filtered, and the filtrate was poured into water (40 mL). The aqueous phase was extracted using a mixed solvent of chloroform:isopropanol = 3:1 (40 mL × 3), and the merged organic phase was dried over anhydrous sodium sulfate and filtered. A hydrogen chloride dioxane solution (15 mL) was added to the filtrate, and the reaction solution was stirred at room temperature for 1 hour and then concentrated under reduced pressure to obtain the title compound (580 mg, yield: 41.1%, white solid).

### Step 3: 6-(Cyclopropylcarboxamido)-N-(ethoxy-d₅)-4-((2-methoxy-3-(1-methyl-1H-1,2,4-triazol-3-yl)phenyl)amino)pyridazine-3-carboxamide (Compound 15)

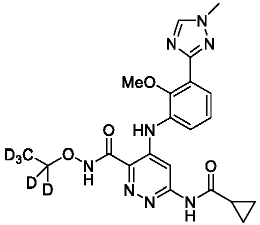

*N*-Methylpyrrolidone (10 mL) and acetonitrile (10 mL) were added to a reaction flask, and ((6-(cyclopropylcarboxamido)-4-((2-methoxy-3-(1-methyl-1*H*-1,2,4-triazol-3-yl)phenyl)amino)pyridazine-3-carbonyl)oxy)zinc (0.5) (300 mg, 0.76 mmol), *O*-(ethyl-*d*₅)hydroxylamine hydrochloride (117.6 mg, 1.15 mmol) and *N*-methylimidazole (188.3 mg, 2.29 mmol) were added. After replacement with nitrogen, the mixture was allowed to react in an oil bath at 65°C for 15 minutes, and then 1-hydroxybenzotriazole (206.6 mg, 1.53 mmol) and 1-(3-dimethylaminopropyl)-3-ethylcarbodiimide hydrochloride (293.1 mg, 1.53 mmol) were added. After replacement with nitrogen, the mixture was allowed to react in an oil bath at 65°C for 16 hours. The reaction was stopped, water (20 mL) was added to the reaction solution for quenching, and then extraction was performed using ethyl acetate (40 mL × 3). The merged organic phase was washed with saturated brine, dried over anhydrous sodium sulfate, and spun in vacuo to remove the solvent to obtain a crude product. The crude product was separated and purified by reverse phase preparative chromatography to obtain the title compound (71.89 mg, yield: 20.6%, white solid).

MS (ESI): m/z 458.2 [M+H]⁺.

¹H NMR (400 MHz, DMSO-*d*₆) δ 12.25 (s, 1H), 11.32 (s, 1H), 10.55 (s, 1H), 8.57 (s, 1H), 8.15 (s, 1H), 7.67 (d, *J* = 7.5 Hz, 1H), 7.51 (d, *J* = 7.8 Hz, 1H), 7.27 (t, *J* = 7.9 Hz, 1H), 3.95 (s, 3H), 3.73 (s, 3H), 2.10 - 2.06 (m, 1H), 0.82 (brs, 2H), 0.81 (brs, 2H).

### Example 16

### 6-(Cyclopropanamido)-4-((3-(1-cyclopropyl-1H-1,2,4-triazol-3-yl)-2-methoxyphenyl)amino)-N-(methoxy-d₃)pyridazine-3-carboxamide (Compound 16)

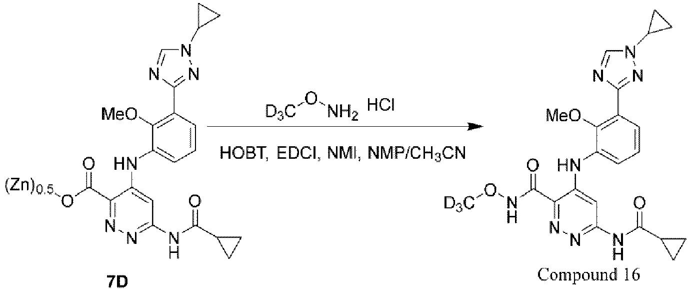

*N*-Methylpyrrolidone (6 mL) and acetonitrile (6 mL) were added to a reaction flask, and the mixed solvent was stirred at room temperature for 10 minutes. Then, ((6-(cyclopropylcarboxamido)-4-((3-(1-cyclopropyl-1*H*-1,2,4-triazol-3-yl)-2-methoxyphenyl)amino)pyridazine-3-carbonyl)oxy)zinc (0.5) (300 mg, 0.642 mmol), *O*-deuterated methylhydroxylamine hydrochloride (111.7 mg, 1.284 mmol) and N-methylimidazole (158.1 mg, 1.926 mmol) were added. The reaction solution was stirred in an oil bath at 65°C for 15 minutes, and then 1-hydroxybenzotriazole (173.5 mg, 1.284 mmol) and 1-ethyl-(3-dimethylaminopropyl)carbodiimide hydrochloride (246.1 mg, 1.284 mmol) were added. The reaction solution continued to be stirred and allowed to react in the oil bath at 65°C for 16 hours. The reaction was stopped, and the reaction solution was poured into water (30 mL). The aqueous phase was extracted using ethyl acetate (30 mL × 3). The merged organic phase was washed with saturated brine (30 mL × 3), dried over anhydrous sodium sulfate, and spun in vacuo to remove the solvent to obtain a crude product. The crude product was separated and purified by high-performance liquid chromatography (elution system: formic acid, water and acetonitrile) to obtain the title compound (55.71 mg, yield: 17.5%, yellow solid).

MS (ESI): m/z 468.1 [M+H]⁺.

¹H NMR (400 MHz, DMSO-*d*₆) δ 12.35 (s, 1H), 11.32 (s, 1H), 10.55 (s, 1H), 8.69 (s, 1H), 8.15 (s, 1H), 7.66 (d, *J =* 7.9 Hz, 1H), 7.51 (d, *J =* 7.7 Hz, 1H), 7.27 (t, *J =* 7.9 Hz, 1H), 3.89 - 3.85 (m, 1H), 3.72 (s, 3H), 2.11 - 2.06 (m, 1H), 1.19 - 1.15 (m, 2H), 1.09 - 1.05 (m, 2H), 0.84 - 0.80 (m, 4H).

### Example 17

### 6-(Cyclopropylcarboxamido)-4-((3-(1-cyclopropyl-1H-1,2,4-triazol-3-yl)-2-methoxyphenyl)amino)-N-(ethoxy-d5)pyridazine-3-carboxamide (Compound 17)

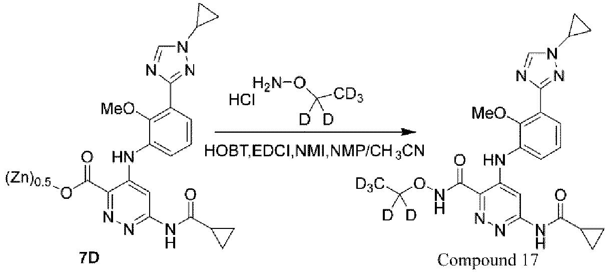

*N*-Methylpyrrolidone (6.5 mL) and acetonitrile (6.5 mL) were added to a reaction flask, and ((6-(cyclopropionamido)-4-((3-(1-cyclopropyl-1*H*-1,2,4-triazol-3-yl)-2-methoxyphenyl)amino)pyridazine-3-carbonyl)oxy)zinc (0.5) (200 mg, 0.43 mmol), *O*-(ethyl-*d*₅)hydroxylamine hydrochloride (65.2 mg, 0.64 mmol) and *N*-methylimidazole (105.45 mg, 1.28 mmol) were added. After replacement with nitrogen, the mixture was allowed to react in an oil bath at 65°C for 15 min. Then, 1-hydroxybenzotriazole (115.7 mg, 0.86 mmol) and 1-(3-dimethylaminopropyl)-3-ethylcarbodiimide hydrochloride (164.15 mg, 0.86 mmol) were added. After replacement with nitrogen, the mixture was allowed to react in the oil bath at 65°C for 16 hours. The reaction was stopped, and water (20 mL) was added to the reaction solution for quenching, then extraction was performed using ethyl acetate (20 mL × 3), and the merged organic phase was washed with saturated brine, dried over anhydrous sodium sulfate, and spun in vacuo to remove the solvent to obtain a crude product. The crude product was separated and purified by reverse phase preparative chromatography (elution system: formic acid, water and acetonitrile) to obtain the title compound (65.3 mg, yield: 31.7%, white solid).

MS (ESI): m/z 484.3 [M+H]⁺.

¹H NMR (400 MHz, DMSO-*d*₆) δ 12.26 (s, 1H), 11.32 (s, 1H), 10.56 (s, 1H), 8.69 (s, 1H), 8.15 (s, 1H), 7.66 (dd, *J* = 7.8, 1.3 Hz, 1H), 7.51 (d, *J* = 7.9 Hz, 1H), 7.27 (t, *J* = 7.9 Hz, 1H), 3.90 - 3.84 (m, 1H), 3.72 (s, 3H), 2.13 - 2.04 (m, 1H), 1.21 - 1.04 (m, 4H), 0.87 - 0.79 (m, 4H).

### Example 18

### 6-(Cyclopropanamido)-N-cyclopropoxy-4-((2-methoxy-3-(1-methyl-1H-1,2,4-triazol-3-yl)phenyl)amino)pyridazine-3-carboxamide (Compound 18)

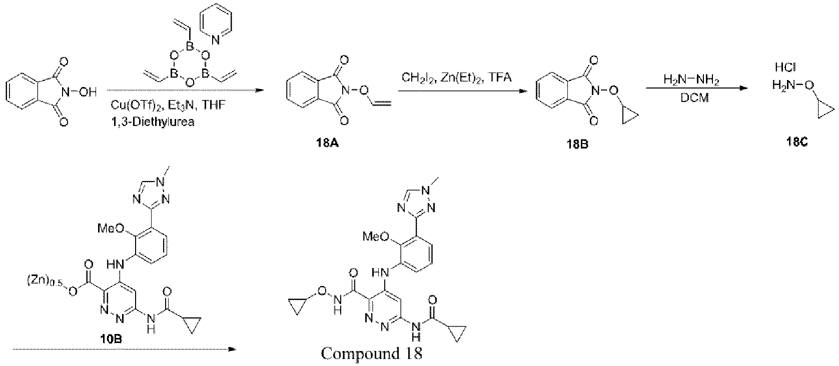

### Step 1: 2-(Vinyl)isoindoline-1,3-dione

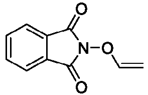

2-Hydroxyisoindoline-1,3-dione (900 mg, 5.517 mmol) was dissolved in ultra-dry tetrahydrofuran (20 mL), and an ethylene boric anhydride pyridine complex (876 mg, 3.641 mmol), 1,3-diethylurea (256 mg, 2.207 mmol), copper trifluoromethanesulfonate (798 mg, 2.207 mmol) and triethylamine (1114 mg, 11.034 mmol) were added. After replacement with oxygen, the mixture was allowed to react at 50°C for 16 hours. The reaction was stopped, the reaction solution was filtered, and then the filtrate was collected and spun dry, and a crude product was separated and purified by silica gel column chromatography (petroleum ether:ethyl acetate = 4:1) to obtain the title compound (950 mg, yield: 91.1%, white solid).

¹H NMR (400 MHz, DMSO-*d*₆) δ 7.92 - 7.86 (m, 4H), 6.92 (dd, J = 13.6, 6.4 Hz, 1H), 4.73 (dd, J = 13.6, 3.5 Hz, 1H), 4.40 (dd, J = 6.4, 3.5 Hz, 1H).

### Step 2: 2-Cyclopropyloxyisoindoline-1,3-dione

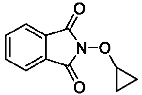

Diethylzinc (19 mL, 19.0 mmol, 1.0 M hexane solution) was added dropwise into ultra-dry dichloromethane (16 mL) at 0°C. After replacement with nitrogen, the temperature was kept at 0°C. Trifluoroacetic acid (2.172 g, 19.048 mmol) was dissolved in ultra-dry dichloromethane (8 mL), the resulting solution was slowly added dropwise into the above solution, and stirring was performed for 20 minutes. Diiodomethane (5.101 g, 19.048 mmol) was dissolved in ultra-dry dichloromethane (8 mL), the resulting solution was slowly added dropwise, and stirring was continued for 20 minutes. 2-(Vinyl)isoindoline-1,3-dione (900 mg, 4.762 mmol) was dissolved in an ultra-dry dichloromethane solution (5.5 mL), and the resulting solution was slowly added dropwise. After replacement with nitrogen, the mixture was allowed to react at room temperature for 16 hours. The reaction was stopped, and dilute hydrochloric acid (0.1 N, 16 mL) was added to the reaction solution, which was separated. The organic phase was collected and then washed using saturated sodium bicarbonate solution (17 mL) and saturated brine (10 mL) in sequence, dried over anhydrous sodium sulfate, filtered, and spun dry to obtain a crude product. The crude product was separated and purified by silica gel column chromatography (petroleum ether:ethyl acetate = 5:1) to obtain the title compound (850 mg, yield: 87.9%, white solid).

### Step 3: O-Cyclopropylhydroxylamine hydrochloride

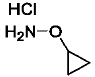

2-Cyclopropyloxyisoindoline-1,3-dione (450 mg, 2.217 mmol) was dissolved in dichloromethane (20 mL), and hydrazine hydrate (222 mg, 4.434 mmol) was added. After replacement with nitrogen, the mixture was allowed to react at room temperature for 16 hours. The reaction was stopped, the reaction solution was filtered, and the filtrate was washed using water (20 mL × 2). The aqueous phase was collected and extracted using a mixed chloroform/isopropanol solution (3:1, 15 mL × 6). The organic phases were merged and then dried, and a solution of hydrogen chloride in 1,4-dioxane (1 mL) was added. The resulting solution was stirred for 1 h and then spun dry to obtain the title compound (130 mg, yield: 53.5%, white solid).

### Step 4: 6-(Cyclopropanamido)-N-cyclopropoxy-4-((2-methoxy-3-(1-methyl-1H-1,2,4-triazol-3-yl)phenyl)amino)pyridazine-3-carboxamide (Compound 18)

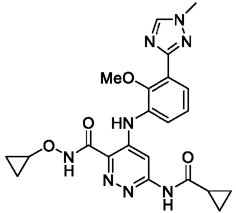

*N*-Methylpyrrolidone (4 mL) and acetonitrile (4 mL) were added to a reaction flask, and then ((6-(cyclopropionamido)-4-((3-(1-methyl-1*H*-1,2,4-triazol-3-yl)-2-methoxyphenyl)amino)pyridazine-3-carbonyl)oxy)zinc (0.5) (255 mg, 0.578 mmol), *O-*cyclopropylhydroxylamine hydrochloride (95.37 mg, 0.867 mmol) and *N*-methylimidazole (142.4 mg, 1.734 mmol) were added. The reaction solution was stirred in an oil bath at 65°C for 15 minutes, and then 1-hydroxybenzotriazole (156.2 mg, 1.156 mmol) and 1-ethyl-(3-dimethylaminopropyl)carbodiimide hydrochloride (221.6 mg, 1.156 mmol) were added. The reaction solution was stirred and allowed to react in an oil bath at 65°C for 2 hours. The reaction was stopped, and water (30 mL) was added to the reaction solution. The aqueous phase was extracted using ethyl acetate (30 mL × 3), and the merged organic phase was washed with saturated brine (30 mL×3), dried over anhydrous sodium sulfate, and spun in vacuo to remove the solvent to obtain a crude product. The crude product was separated and purified by high-performance liquid chromatography (elution system: formic acid, water and acetonitrile) to obtain the title compound (16.22 mg, yield: 6.03%, yellow solid).

MS (ESI): m/z 465.0 [M+H]⁺.

¹H NMR (400 MHz, DMSO-*d*₆) δ 12.37 (s, 1H), 11.32 (s, 1H), 10.54 (s, 1H), 8.56 (s, 1H), 8.16 (s, 1H), 7.69 - 7.65 (m, 1H), 7.52 (d, *J =* 7.7 Hz, 1H), 7.27 (t, *J =* 7.9 Hz, 1H), 4.12 - 4.07 (m, 1H), 3.95 (s, 3H), 3.73 (s, 3H), 2.13 - 2.06 (m, 1H), 0.90 (brs, 2H), 0.82 (brs, 4H), 0.61 - 0.56 (m, 2H).

### Example 19

### 6-(Cyclopropylcarboxamido)-N-cyclopropyloxy-4-((3-(1-ethyl-1H-1,2,4-triazol-3-yl)-2-methoxyphenyl)amino)pyridazine-3-carboxamide (Compound 19)

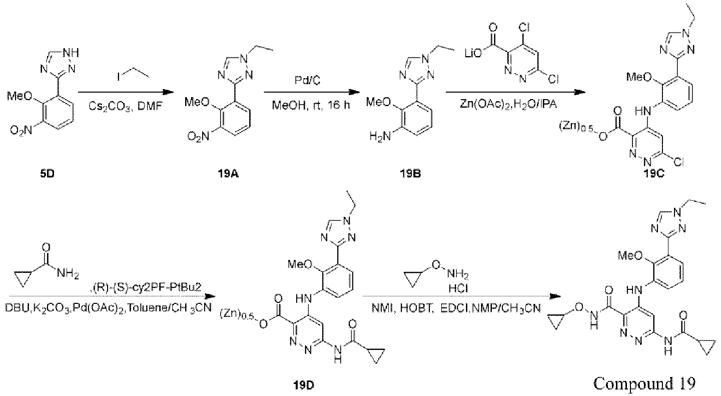

### Step 1: 1-Ethyl-3-(2-methoxy-3-nitrophenyl)-1H-1,2,4-triazole

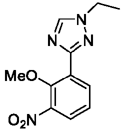

3-(2-Methoxy-3-nitrophenyl)-1H-1,2,4-triazole (450 mg, 2.044 mmol) was dissolved in ultra-dry N,N-dimethylformamide (10 mL), and cesium carbonate (1.998 g, 6.132 mmol) was added. After replacement with nitrogen, iodoethane (478 mg, 3.066 mmol) was slowly added dropwise in an ice bath at 0°C, and then the mixture was transferred to room temperature for reaction for 2 hours. The reaction was stopped, and the reaction solution was filtered and then poured into water (30 mL). The aqueous phase was extracted using ethyl acetate (15 mL × 3), and the merged organic phase was washed with saturated brine (20 mL × 2) and dried over anhydrous sodium sulfate. After filtration, the filtrate was spun in vacuo to remove the solvent to obtain the title compound (530 mg, light yellow oily matter), which was directly used for the reaction in the next step.

MS (ESI): m/z 249.0 [M+H]⁺.

### Step 2: 3-(1-ethyl-1H-1,2,4-triazol-3-yl)-2-methoxyaniline

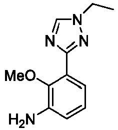

1-Ethyl-3-(2-methoxy-3-nitrophenyl)-1*H*-1,2,4-triazole 3 (530 mg, 2.135 mmol) was dissolved in methanol (20 mL), and palladium/carbon (91 mg, 0.854 mmol) was added. After replacement with hydrogen three times, the mixture was allowed to react at room temperature for 2 hours. The reaction was stopped, and the reaction solution was filtered and then spun dry under reduced pressure. A crude product was separated and purified by silica gel column chromatography (petroleum ether:ethyl acetate = 1:3) to obtain the title compound (335 mg, yield: 71.9%, light yellow solid).

MS (ESI): m/z 219.2 [M+H]⁺.

### Step 3: ((6-Chloro-4-((3-(1-ethyl-1H-1,2,4-triazol-3-yl)-2-methoxyphenyl)amino)pyridazine-3-carbonyl)oxy)zinc (0.5)

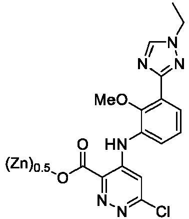

3-(1-Ethyl-1*H*-1,2,4-triazol-3-yl)-2-methoxyaniline (300 mg, 1.375 mmol) was dissolved in water (14 mL) and isopropanol (2 mL), and lithium 4,6-dichloropyridazine-3-carboxylate (410 mg, 2.063 mmol) and zinc acetate (379 mg, 2.063 mmol) were added. After replacement with nitrogen three times, the mixture was allowed to react in an oil bath at 65°C for 16 hours. The reaction was stopped, and water (10 mL) was added to the reaction solution. The solution was stirred at room temperature for 1 hour and then filtered by suction. The filter cake was rinsed using water (6 mL × 2) and tetrahydrofuran (1 mL), and dried to obtain the title compound (440 mg, yield: 79.0%, light yellow solid).

MS (ESI): m/z 375.3 [M+H]⁺.

### Step 4: ((6-(Cyclopropanamido)-4-((3-(1-ethyl-1H-1,2,4-triazol-3-yl)-2-methoxyphenyl)amino)pyridazine-3-carbonyl)oxy)zinc (0.5)

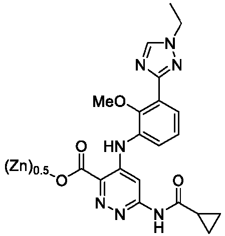

((6-Chloro-4-((3-(1-ethyl-1*H*-1,2,4-triazol-3-yl)-2-methoxyphenyl)amino)pyridazine-3-carbonyl)oxy)zinc (0.5) (400 mg, 0.984 mmol) was dissolved in toluene (8 mL) and acetonitrile (4 mL), and cyclopropylcarboxamide (209 mg, 2.460 mmol), (R)-(-)-1-[(S)-2-(dicyclohexylphosphino)ferrocene]ethyl di-tert-butylphosphine (109 mg, 0.197 mmol), 1,8-diazabicycloundec-7-ene (150 mg, 0.984 mmol), potassium carbonate (273 mg, 1.974 mmol) and palladium acetate (44 mg, 0.197 mmol) were added. After replacement with nitrogen, the mixture was allowed to react in an oil bath at 75°C for 16 hours. The reaction was stopped, and the reaction solution was cooled to room temperature. Water (15 mL) and acetic acid (7.5 mL) were added, and the mixed system was washed using petroleum ether (30 mL × 2). The aqueous phase was extracted using dichloromethane (15 mL × 3), and the merged dichloromethane organic phase was washed with saturated brine (20 mL × 2), dried over anhydrous sodium sulfate, filtered, and spun in vacuo to remove the solvent to obtain the title compound (750 mg, brown solid).

MS (ESI): m/z 424.1 [M+H]⁺.

### Step 5: 6-(Cyclopropylcarboxamido)-N-cyclopropyloxy-4-((3-(1-ethyl-1H-1,2,4-triazol-3-yl)-2-methoxyphenyl)amino)pyridazine-3-carboxamide (Compound 19)

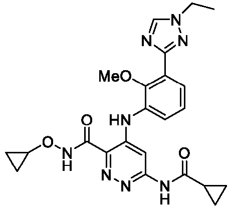

*N*-Methylpyrrolidone (8 mL) and acetonitrile (8 mL) were added to a reaction flask, and the mixed solvent was stirred at room temperature for 10 minutes. Then, ((6-(cyclopropionamido)-4-((3-(1-ethyl-1*H*-1,2,4-triazol-3-yl)-2-methoxyphenyl)amino)pyridazine-3-carbonyl)oxy)zinc (0.5) (750 mg, 1.648 mmol), *O*-cyclopropylhydroxylamine hydrochloride (271 mg, 2.472 mmol) and *N*-methylimidazole (404 mg, 4.944 mmol) were added. The reaction solution was stirred in an oil bath at 65°C for 15 minutes, and then 1-hydroxybenzotriazole (445 mg, 3.296 mmol) and 1-ethyl-(3-dimethylaminopropyl)carbodiimide hydrochloride (632 mg, 3.296 mmol) were added. The reaction solution continued to be stirred and allowed to react in the oil bath at 65°C for 16 hours. The reaction was stopped, and the reaction solution was filtered. A crude product was separated and purified by high-performance liquid chromatography (elution system: formic acid, water and acetonitrile) to obtain the title compound (21.82 mg, yield: 3.7%, light yellow solid).

MS (ESI): m/z 479.2 [M+H]⁺.

¹H NMR (400 MHz, DMSO-*d*₆) δ 12.46 (s, 1H), 11.33 (s, 1H), 10.56 (s, 1H), 8.61 (s, 1H), 8.17 (s, 1H), 7.68 (dd, *J* = 7.8, 1.5 Hz, 1H), 7.52 (dd, *J* = 7.9, 1.4 Hz, 1H), 7.28 (t, *J* = 7.9 Hz, 1H), 4.28 (q, *J* = 7.3 Hz, 2H), 4.11 - 4.08 (m, 1H), 3.73 (s, 3H), 2.14 - 2.05 (m, 1H), 1.46 (t, *J* = 7.3 Hz, 3H), 0.90 (brs, 2H), 0.85 - 0.79 (m, 4H), 0.62 - 0.55 (m, 2H).

### Example 20

### 6-(Cyclopropylcarboxamido)-N-cyclopropyloxy-4-((2-methoxy-3-(1-(methyl-d3)-1H-1,2,4-triazol-3-yl)phenyl)amino)pyridazine-3-carboxamide (Compound 20)

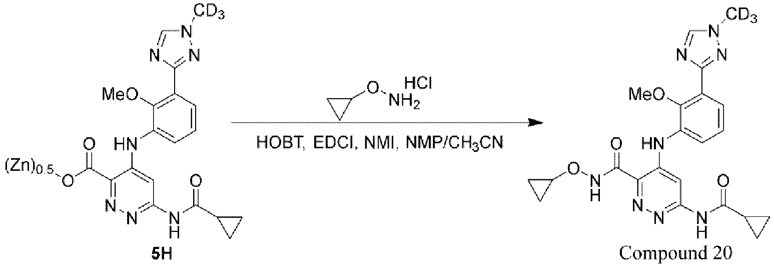

*N*-Methylpyrrolidone (1 mL) and acetonitrile (1 mL) were added to a reaction flask, and then ((6-(cyclopropionamido)-4-((2-methoxy-3-(1-(methyl-*d*₃)-1*H*-1,2,4-triazol-3-yl)phenyl)amino)pyridazine-3-carbonyl)oxy)zinc (0.5) (155 mg, 0.349 mmol), *O-*cyclopropylhydroxylamine hydrochloride (57 mg, 0.524 mmol) and *N*-methylimidazole (86 mg, 1.047 mmol) were added. The reaction solution was stirred in an oil bath at 65°C for 15 minutes, and then 1-hydroxybenzotriazole (94 mg, 0.698 mmol) and 1-ethyl-(3-dimethylaminopropyl)carbodiimide hydrochloride (137 mg, 0.698 mmol) were added. The reaction solution continued to be stirred and allowed to react in the oil bath at 65°C for 16 hours. The reaction was stopped, and the reaction solution was filtered. A crude product of the filtrate was separated and purified by high-performance liquid chromatography (elution system: formic acid, water and acetonitrile) to obtain the title compound (53.77 mg, yield: 33.0%, light yellow solid).

MS (ESI): m/z 468.4 [M+H]⁺.

¹H NMR (400 MHz, DMSO-*d*₆) δ 12.49 (s, 1H), 11.50 (s, 1H), 10.61 (s, 1H), 8.69 (s, 1H), 8.06 (s, 1H), 7.69 (dd, *J* = 7.8, 1.5 Hz, 1H), 7.53 (dd, *J* = 8.0, 1.4 Hz, 1H), 7.29 (t, *J* = 7.9 Hz, 1H), 4.12 - 4.05 (m, 1H), 3.72 (s, 3H), 2.10 - 2.02 (m, 1H), 0.91 - 0.86 (m, 2H), 0.85 - 0.82 (m, 4H), 0.61 - 0.54 (m, 2H).

### Example 21

### 6-(Cyclopropylcarboxamido)-N-cyclopropyloxy-4-((2-(methoxy-d₃)-3-(1-methyl-1H-1,2,4-triazol-3-yl)phenyl)amino)pyridazine-3-carboxamide (Compound 21)

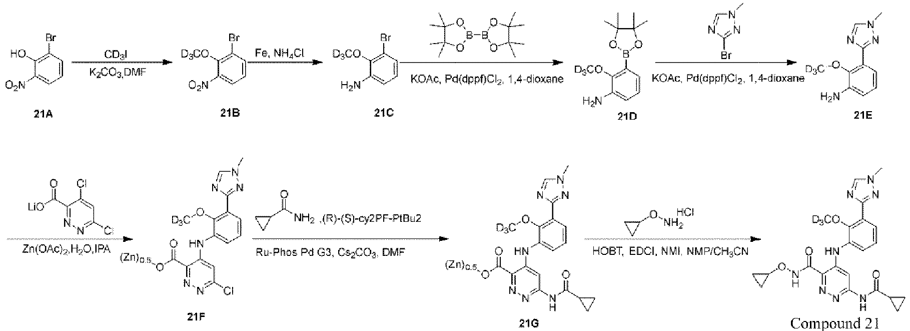

### Step 1: 1-Bromo-2-(methoxy-d₃)-3-nitrobenzene

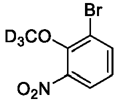

2-Bromo-6-nitrophenol (1000 mg, 4.587 mmol) was dissolved in ultra-dry *N,N-*dimethylformamide (15 mL), and potassium carbonate (1.268 g, 9.174 mmol) was added. After replacement with nitrogen, deuterated iodomethane (997 mg, 6.881 mmol) was slowly added dropwise in an ice bath at 0°C, and the mixture was then transferred to 60°C for reaction for 3 hours. The reaction was stopped, and the reaction solution was filtered and then poured into water (50 mL). The aqueous phase was extracted using ethyl acetate (20 mL × 3), and the merged organic phase was washed with saturated brine (20 mL × 3), dried over anhydrous sodium sulfate, and spun in vacuo to remove the solvent to obtain a crude title compound (1000 mg, yield: 77.5%, light yellow oily matter)

¹H NMR (400 MHz, DMSO-*d*₆) δ 8.01 (dd, *J* = 8.1, 1.5 Hz, 1H), 7.95 (dd, *J* = 8.2, 1.5 Hz, 1H), 7.31 (t, *J* = 8.1 Hz, 1H).

### Step 2: 3-Bromo-2-(methoxy-d₃)aniline

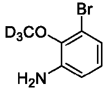

1-Bromo-2-(methoxy-*d*₃)-3-nitrobenzene (1000 mg, 4.254 mmol) was dissolved in isopropanol (9 mL) and water (0.9 mL), and iron powder (1188 mg, 21.270 mmol) and ammonium chloride (1138 mg, 21.270 mmol) were added. After replacement with nitrogen, the mixture was allowed to react at 80°C for 2 hours. The reaction was stopped, and the reaction solution was filtered and then spun dry, so as to obtain a crude product, which was separated and purified by silica gel column chromatography (petroleum ether:ethyl acetate = 1:1) to obtain the title compound (730 mg, yield: 83.7%, light yellow solid).

MS (ESI): m/z 205.2 [M+H]⁺.

### Step 3: 2-(Methoxy-d₃)-3-(4,4,5,5-tetramethyl-1,3,2-dioxaborolan-2-yl)aniline

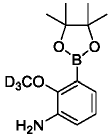

3-Bromo-2-(methoxy-*d*₃)aniline (730 mg, 3.560 mmol) and diboronic acid pinacol ester (1808 mg, 7.12 mmol) were dissolved in ultra-dry dioxane (20 mL), and potassium acetate (699 mg, 7.12 mmol) and [1,1'-bis(diphenylphosphino)ferrocene]dichloropalladium(II) (260 mg, 0.356 mmol) were added. After replacement with nitrogen three times, the mixture was allowed to react in an oil bath at 110°C for 3 hours. The reaction was stopped, and the reaction solution was filtered and then spun dry. A crude product was separated and purified by silica gel column chromatography (petroleum ether:ethyl acetate = 1:1) to obtain the title compound (783 mg, yield: 87.2%, light yellow solid).

MS (ESI): m/z 254.2 [M+H]⁺.

### Step 4: 2-(Methoxy-d₃)-3-(1-methyl-1H-1,2,4-triazol-3-yl)aniline

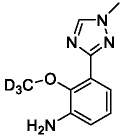

2-(Methoxy-*d*₃)-3-(4,4,5,5-tetramethyl-1,3,2-dioxaborolan-2-yl)aniline (783 mg, 3.105 mmol) and 3-bromo-1-methyl-1*H*-1,2,4-triazole (755 mg, 4.658 mmol) were dissolved in a dioxane solution (20 mL) and water (4 mL), and potassium carbonate (1287 mg, 9.315 mmol) and [1,1'-bis(diphenylphosphino)ferrocene]dichloropalladium(II) (228 mg, 0.311 mmol) were added. After replacement with nitrogen three times, the mixture was allowed to react in an oil bath at 110°C for 16 hours. The reaction was stopped, and the reaction solution was filtered and then spun dry. A crude product was separated and purified by silica gel column chromatography (petroleum ether:ethyl acetate = 1:1) to obtain the title compound (550 mg, yield: 85.5%, light yellow solid).

MS (ESI): m/z 208.2 [M+H]⁺.

### Step 5: ((6-Chloro-4-((2-(methoxy-d₃)-3-(1-methyl-1H-1,2,4-triazol-3-yl)phenyl)amino)pyridazine-3-carbonyl)oxy)zinc (0.5)

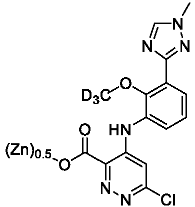

2-(Methoxy-*d*₃)-3-(1-methyl-1*H*-1,2,4-triazol-3-yl)aniline (550 mg, 2.654 mmol) was dissolved in water (21 mL) and isopropanol (3 mL), and lithium 4,6-dichloropyridazine-3-carboxylate 5 (612 mg, 3.185 mmol) and zinc acetate (854 mg, 3.185 mmol) were added. After replacement with nitrogen three times, the mixture was allowed to react in an oil bath at 65°C for 16 hours. The reaction was stopped, and water (20 mL) was added to the reaction solution. The solution was stirred at room temperature for 1 hour and then filtered by suction. The filter cake was rinsed using water (6 mL × 2) and tetrahydrofuran (1 mL), and dried to obtain the title compound (400 mg, yield: 38.1%, yellow solid).

MS (ESI): m/z 364.2 [M+H]⁺.

### Step 6: ((6-(Cyclopropylcarboxamido)-4-((2-(methoxy-d₃)-3-(1-methyl-1H-1,2,4-triazol-3-yl)phenyl)amino)pyridazine-3-carbonyl)oxy)zinc (0.5)

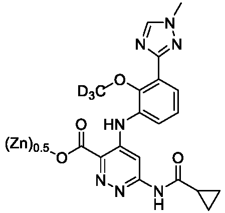

((6-Chloro-4-((2-(methoxy-*d*₃)-3-(1-methyl-1*H*-1,2,4-triazol-3-yl)phenyl)amino)pyridazine-3-carbonyl)oxy)zinc (0.5) (180 mg, 0.455 mmol) was dissolved in ultra-dry *N*,*N*-dimethylformamide (5 mL), and cyclopropylcarboxamide (97 mg, 1.138 mmol), cesium carbonate (296 mg, 0.91 mmol) and methanesulfonic acid (2-dicyclohexylphosphino-2',6'-diisopropoxy-1,1'-biphenyl)(2-amino-1,1'-biphenyl-2-yl)palladium(II) (115 mg, 0.137 mmol) were added. After replacement with nitrogen, the mixture was allowed to react at 100°C for 16 hours. The reaction was stopped, and the reaction solution was filtered and then separated and purified by reverse column chromatography (elution system: formic acid, water and acetonitrile) to obtain the title compound (50 mg, yield: 24.8%, light yellow solid).

MS (ESI): m/z 413.3 [M+H]⁺.

### Step 7: 6-(Cyclopropylcarboxamido)-N-cyclopropyloxy-4-((2-(methoxy-d₃)-3-(1-methyl-1H-1,2,4-triazol-3-yl)phenyl)amino)pyridazine-3-carboxamide (Compound 21)

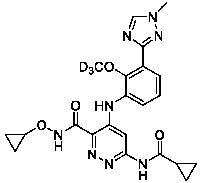

*N*-Methylpyrrolidone (1 mL) and acetonitrile (1 mL) were added to a reaction flask, and then ((6-(cyclopropylcarboxamido)-4-((2-(methoxy-*d*₃)-3-(1-methyl-1*H*-1,2,4-triazol-3-yl)phenyl)amino)pyridazine-3-carbonyl)oxy)zinc (0.5) (75 mg, 0.169 mmol), *O-*cyclopropylhydroxylamine hydrochloride (29 mg, 0.254 mmol) and N-methylimidazole (41 mg, 0.507 mmol) were added. The reaction solution was stirred in an oil bath at 65°C for 15 minutes, and then 1-hydroxybenzotriazole (46 mg, 0.338 mmol) and 1-ethyl-(3-dimethylaminopropyl)carbodiimide hydrochloride (65 mg, 0.338 mmol) were added. The reaction solution continued to be stirred and allowed to react in the oil bath at 65°C for 16 hours. The reaction was stopped, and the reaction solution was filtered. A crude product of the filtrate was separated and purified by high-performance liquid chromatography (elution system: formic acid, water and acetonitrile) to obtain the title compound (20.86 mg, yield: 26.4%, light yellow solid).

MS (ESI): m/z 468.2 [M+H]⁺.

¹H NMR (400 MHz, DMSO-*d*₆) δ 12.46 (s, 1H), 11.35 (s, 1H), 10.55 (s, 1H), 8.59 (s, 1H), 8.12 (s, 1H), 7.66 (dd, *J* = 7.8, 1.5 Hz, 1H), 7.51 (dd, *J* = 8.0, 1.5 Hz, 1H), 7.27 (t, *J* = 7.9 Hz, 1H), 4.09 - 4.08 (m, 1H), 3.94 (s, 3H), 2.11 - 2.03 (m, 1H), 0.91 - 0.86 (m, 2H), 0.85 - 0.78 (m, 4H), 0.61 - 0.54 (m, 2H).

### Example 22

### N-Cyclobutyloxy-6-(cyclopropylcarboxamido)-4-((2-methoxy-3-(1-methyl-1H-1,2,4-triazol-3-yl)phenyl)amino)pyridazine-3-carboxamide (Compound 22)

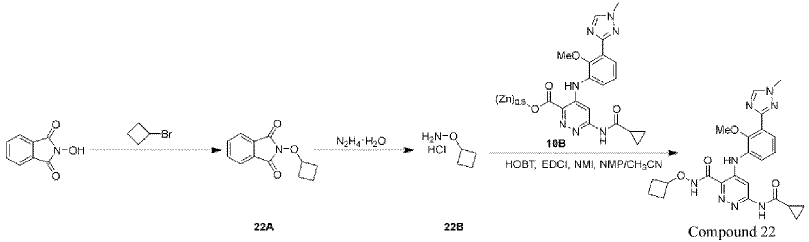

### Step 1: 2-Cyclobutaneoxyisoindoline-1,3-dione

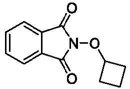

2-Hydroxyisoindoline-1,3-dione (10 g, 61.3 mmol) was dissolved in *N,N-*dimethylformamide (100 mL), and cyclobutyl bromide (12.4 g, 91.85 mmol), 18-crown ether-6 (3.24 g, 12.258 mmol) and potassium carbonate (16.9 g, 122.3 mmol) were added. After replacement with nitrogen, the reaction solution was stirred and allowed to react in an oil bath at 80°C for 16 hours. The reaction was stopped, and the reaction solution was poured into water (80 mL). The aqueous phase was extracted using ethyl acetate (80 mL × 3), and the merged organic phase was washed with saturated brine (80 mL × 3), dried over anhydrous sodium sulfate, and spun in vacuo to remove the solvent to obtain a crude product. The crude product was purified by silica gel column chromatography (petroleum ether:ethyl acetate = 3:1) to obtain the title compound (8.2 g, yield: 61.7%, white solid).

MS (ESI): m/z 218.1 [M+H]⁺.

### Step 2: O-Cyclobutanolamine hydrochloride

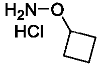

2-Cyclobutaneoxyisoindoline-1,3-dione (500 mg, 2.304 mmol) was dissolved in dichloromethane (8 mL), and hydrazine hydrate (230.7 mg, 4.608 mmol) was added. After replacement with nitrogen, the reaction solution was stirred at room temperature for 16 hours. TLC plate monitoring showed that the starting materials disappeared. The reaction solution was filtered, and the filtrate was poured into water (20 mL). The aqueous phase was extracted using a mixed solvent of chloroform:isopropanol = 3:1 (10 mL × 3), and the merged organic phase was dried over anhydrous sodium sulfate and filtered. A solution (2 mL) of hydrogen chloride in 1,4-dioxane was added to the filtrate. The reaction solution was stirred at room temperature for 1 hour. The reaction solution was concentrated under reduced pressure to obtain the title compound (180 mg, yield: 63.2%, white solid).

### Step 3: N-Cyclobutyloxy-6-(cyclopropylcarboxamido)-4-((2-methoxy-3-(1-methyl-1H-1,2,4-triazol-3-yl)phenyl)amino)pyridazine-3-carboxamide (Compound 22)

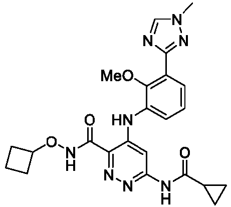

*N*-Methylpyrrolidone (4 mL) and acetonitrile (4 mL) were added to a reaction flask, and the mixed solvent was stirred at room temperature for 10 minutes. Then, ((6-(cyclopropionamido)-4-((3-(1-methyl-1*H*-1,2,4-triazol-3-yl)-2-methoxyphenyl)amino)pyridazine-3-carbonyl)oxy)zinc (0.5) (230 mg, 0.521 mmol), *O*-cyclobutylhydroxylamine hydrochloride (96.6 mg, 0.782 mmol) and *N*-methylimidazole (128.3 mg, 1.563 mmol) were added. The reaction solution was stirred in an oil bath at 65°C for 15 minutes. Then, 1-hydroxybenzotriazole (140.8 mg, 1.042 mmol) and 1-ethyl-(3-dimethylaminopropyl)carbodiimide hydrochloride (199.8 mg, 1.042 mmol) were added, and the reaction solution was stirred in an oil bath at 65°C for 2 hours. The reaction was stopped, and water (30 mL) was added to the reaction solution. The aqueous phase was extracted using ethyl acetate (30 mL × 3). The merged organic phase was washed with saturated brine (30 mL × 3), dried over anhydrous sodium sulfate, and spun in vacuo to remove the solvent to obtain a crude product. The crude product was separated and purified by high-performance liquid chromatography (elution system: formic acid, water and acetonitrile) to obtain the title compound (11.86 mg, yield: 4.7%, white solid).

MS (ESI): m/z 479.0 [M+H]⁺.

¹H NMR (400 MHz, DMSO-*d*₆) δ 12.21 (s, 1H), 11.31 (s, 1H), 10.54 (s, 1H), 8.56 (s, 1H), 8.33 (s, 1H), 7.66 (d, *J =* 7.4 Hz, 1H), 7.51 (d, *J =* 7.6 Hz, 1H), 7.27 (t, *J =* 7.9 Hz, 1H), 4.58 - 4.51 (m, 1H), 3.95 (s, 3H), 3.72 (s, 3H), 2.19 - 2.08 (m, 5H), 1.76 - 1.69 (m, 1H), 1.55 - 1.48 (m, 1H), 0.82 (brs, 4H).

### Biological test evaluation

### Test Example A: In vitro enzyme binding assay of compound TYK2JH2 of the present invention

Purpose: The purpose of this test example was to use a fluorescence resonance energy transfer (TR-FRET) method to test the binding effect of the compound on a TYK2 JH2 pseudokinase, and then evaluate the affinity of the compound for TYK2 JH2.

### Experimental method:

DMSO-dissolved compounds were used to prepare 10 mM stock solutions, then 200X compounds having different gradient concentrations were prepared in a compound dilution plate, and transferred to an Echo plate. 75 nL of the compounds were transferred from the Echo plate to a 384-well assay plate using an Echo instrument, and 5 µL of 3X TYK2 JH2 kinase (Bioduro), 5 µL of 3X Tb antibody (Cisbio) and 5 µL of 3X TRACER (Bioduro) were added to the 384-well assay plate, centrifuged for 30 seconds, and incubated at room temperature for 60 minutes. A 495 nm/520 nm fluorescence signal ratio was read by an Envision microplate reader (PerkinElmer), and data was analyzed using XL-Fit software to calculate the IC₅₀ of the compound.

### Experimental results:

| Compound number | TYK2 JH2 binding assay (IC₅₀, nM) |
|---|---|
| Compound 1 | 0.17 |
| Compound 2 | 0.16 |
| Compound 3 | 0.13 |
| Compound 4 | 0.20 |
| Compound 5 | 0.26 |
| Compound 6 | 0.30 |
| Compound 7 | 0.17 |
| Compound 8 | 0.33 |
| Compound 9 | 0.22 |
| Compound 14 | 0.27 |
| Compound 15 | 0.14 |
| Compound 16 | 0.29 |
| Compound 17 | 0.19 |
| BMS-986165 | 0.25 |

It can be seen from the experimental data that the compound of the present invention had a good binding effect on TYK2 JH2 pseudokinase.

### Test Example B: Determination of the effects of the compound of the present invention on IL-2-induced STATS phosphorylation (JAK1/3) in CD3+ cell subpopulations in human PBMC

Purpose: The purpose of this test example was to test the inhibitory effect of the compound on IL-2-induced STAT5 phosphorylation using protein phosphorylation flow cytometric analysis technology.

Experimental method:

Human PBMC cells were pre-incubated with the compounds, STAT5 was induced to phosphorylate under appropriate stimulation conditions, and corresponding cell subpopulations and targets were stained. Flow cytometry was used to read cell data and analyze the strength of phosphorylated antibody signals under different compound concentrations. PBMCs were resuspended and dispensed into a 96-well plate, 62.5 µL/well. 3.5 µL of 20X compound working solutions were added and incubated at 37°C for 30 minutes. 5 µL of PE Mouse Anti-Human CD3 (BD) was added and incubated at 37°C in the dark for 30 minutes. 4 µL of 20X IL-2 (R&D) was added to each well and incubated at 37°C in the dark for 20 minutes. All cells in the 96-well plate were transferred to a deep-well plate, 400 µL of a fixation working solution (Biolegend) was added to each well, and incubated at room temperature in the dark for 20 minutes. The cells were washed twice with PBS, and 400 µL of Perm Buffer III (BD) was added and incubated at 4°C in the dark for 40 minutes. The cells were washed twice with PBS, and 100 µL of a STAT5 pY694 antibody working solution (BD) was added and incubated at room temperature for 40 minutes. After washing once with PBS, the cells were resuspended in 200 µL of a staining buffer and transferred to a sample plate. The antibody fluorescence intensity was analyzed using FlowJo software, and the IC₅₀ of the compound was calculated using XL-Fit software.

### Experimental results:

| Compound number | IL-2-induced CD3+ pSTAT5 (JAK1/3) in hPBMC (IC₅₀, µM) |
|---|---|
| Compound 7 | >30 |
| Compound 9 | >30 |
| Compound 17 | >30 |
| BMS-986165 | 0.409 |

It can be seen from the experimental data results that the compound of the present invention had lower inhibitory activity on the JAK1/3 signaling pathway and had better selectivity.

### Test Example C: Pharmacokinetic evaluation of the compound of the present invention by intravenous injection or oral gavage in mice

Purpose: The purpose of this test example was to evaluate the pharmacokinetic properties of the compound of the present invention.

The inventors evaluated the pharmacokinetic properties of the compound of the present invention in mice. Animal information is shown in Table 1.

**Table 1: Information table of the test animals of the present invention**

| **Germline** | **Grade** | **Sex** | **Weight** | **Age** | **Source** |
|---|---|---|---|---|---|
| ICR mice | SPF | Male | 26-32 g | 6-8 weeks | Shanghai Xipuer-Bikai Experimental Animal Co., Ltd. |

### Experimental method:

The compound of the present invention was administered to the test animals in the form of 5% DMA + 5% Solutol + 90% saline or 5% ethanol + 5% TPGS + 90% PEG300 solvent. The animals were fasted for 12 hours before administration and had free access to water. For the intravenous administration group, the dose was 1 mg/kg. The drug was administered by intragastric administration at a dose of 10 mg/kg. After the drug administration, blood was intravenously drawn from the animals at the following time points (blood volume was approximately 0.3 mL): 0.083, 0.25, 0.5, 1.0, 2.0, 4.0, 8.0 and 24 h. EDTA-K2 was pre-added to blood collection tubes as an anticoagulant, and the blood samples were centrifuged at 6800 g for 6 min to collect plasma, which was then stored at -80°C.

Sample preparation for LC-MS/MS assay: 15 µL of plasma samples was used for protein precipitation with 300 µL of methanol containing 100 ng/mL IS (IS was tolbutamide). The mixture was vortexed for 1 min and then centrifuged at 18000 g for 10 min. 300 µL of the supernatant was transferred to a 96-well plate. 2 µL of the supernatant was subjected to LC-MS/MS analysis. The analysis results showed that the compound of the present invention had good pharmacokinetic properties in mice.

| Group | Tested substance | Dose (mg/kg) | Tmax (h) | Cmax (ng/mL) | AUClast (h * ng/mL) | T_{1/2} (h) | Cl (mL/min/kg) | Vz (L/kg) | F (%) |
|---|---|---|---|---|---|---|---|---|---|
| Intravenous administration group | Compound 7 | 1 | 0.08 | 3622 | 2700 | 1.51 | 6.13 | 0.81 | \ |
| | Compound 9 | 1 | 0.08 | 3731 | 2778 | 4.30 | 6.09 | 1.80 | \ |
| | Compound 17 | 1 | 0.08 | 3398 | 1855 | 3.61 | 9.13 | 2.77 | \ |
| Intragastric administration group | Compound 7 | 10 | 0.83 | 3460 | 7854 | 3.75 | \ | \ | 29.09 |
| | Compound 9 | 10 | 0.67 | 9217 | 20135 | 4.64 | \ | \ | 72.48 |
| | Compound 17 | 10 | 0.67 | 5636 | 17469 | 5.03 | \ | \ | 94.13 |

## Claims

1. A compound represented by general formula (I), or a pharmaceutically acceptable salt, enantiomer, diastereomer, racemate, solvate, hydrate, polymorph, prodrug or isotopic variant thereof: 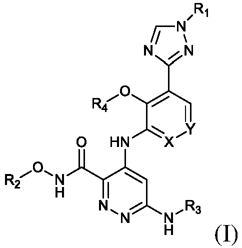 wherein
X is CR' or N;
Y is CR';
R₁ is selected from a C₁₋₆ alkyl, a C₂₋₆ alkenyl, a C₂₋₆ alkynyl, a C₃₋₇ cycloalkyl, a 3- to 7-membered heterocyclyl, a C₆₋₁₀ aryl or a 5- to 10-membered heteroaryl, and the group is optionally substituted with one or more deuteriums until fully deuterated;
R₂ is selected from a C₁₋₆ alkyl, a C₁₋₆ haloalkyl, a C₂₋₆ alkenyl, a C₂₋₆ alkynyl, a C₃₋₇ cycloalkyl, a 3- to 7-membered heterocyclyl, a C₆₋₁₀ aryl or a 5- to 10-membered heteroaryl, and the group is optionally substituted with one or more deuteriums until fully deuterated;
R₃ is selected from an -L-C₁₋₆ alkyl, an -L-C₁₋₆ haloalkyl, an -L-C₃₋₇ cycloalkyl, an -L-3-to 7-membered heterocyclyl, an -L-C₆₋₁₀ aryl or an -L-5- to 10-membered heteroaryl, and the group is optionally substituted with one or more deuteriums until fully deuterated;
R₄ is selected from H, a C₁₋₆ alkyl or a C₁₋₆ haloalkyl, and the group is optionally substituted with one or more deuteriums until fully deuterated;
each group of R1, R2, R3 and R4 is optionally substituted with 1, 2, 3 or 4 R;
wherein L is selected from a bond, -C(O)- or -C(S)-;
R is selected from H, D, a halogen, CN, a C₁₋₆ alkyl or a C₁₋₆ haloalkyl; and
R' is selected from H, D, a halogen or CN.

2. The compound, or the pharmaceutically acceptable salt, enantiomer, diastereomer, racemate, solvate, hydrate, polymorph, prodrug or isotopic variant thereof according to claim 1, wherein
X is CR' or N;
Y is CR';
R₁ is selected from a C₁₋₆ alkyl, a C₃₋₇ cycloalkyl or a 3- to 7-membered heterocyclyl, and the group is optionally substituted with one or more deuteriums until fully deuterated;
R₂ is selected from a C₁₋₆ alkyl, a C₁₋₆ haloalkyl, a C₃₋₇ cycloalkyl or a 3- to 7-membered heterocyclyl, and the group is optionally substituted with one or more deuteriums until fully deuterated;
R₃ is selected from an -L-C₃₋₇ cycloalkyl, an -L-3- to 7-membered heterocyclyl, a C₆₋₁₀ aryl or a 5- to 6-membered heteroaryl, and the group is optionally substituted with one or more deuteriums until fully deuterated;
R4 is selected from H, a C₁₋₆ alkyl or a C₁₋₆ haloalkyl, and the group is optionally substituted with one or more deuteriums until fully deuterated;
each group of R1, R2, R3 and R4 is optionally substituted with 1, 2, 3 or 4 R;
wherein L is selected from -C(O)- or -C(S)-;
R is selected from H, D, a halogen or CN; and
R' is selected from H, D, a halogen or CN.

3. The compound, or the pharmaceutically acceptable salt, enantiomer, diastereomer, racemate, solvate, hydrate, polymorph, prodrug or isotopic variant thereof according to claim 1 or 2, wherein X is CR', preferably CH.

4. The compound, or the pharmaceutically acceptable salt, enantiomer, diastereomer, racemate, solvate, hydrate, polymorph, prodrug or isotopic variant thereof according to any one of claims 1 to 3, wherein Y is CH or CF.

5. The compound, or the pharmaceutically acceptable salt, enantiomer, diastereomer, racemate, solvate, hydrate, polymorph, prodrug or isotopic variant thereof according to any one of claims 1 to 4, wherein R₁ is selected from a C₁₋₆ alkyl, a C₂₋₆ alkenyl, a C₂₋₆ alkynyl, a C₃₋₇ cycloalkyl or a 3- to 7-membered heterocyclyl, and the group is optionally substituted with one or more deuteriums until fully deuterated; preferably, R₁ is selected from a C₁₋₆ alkyl or a C₃₋₇ cycloalkyl, and the group is optionally substituted by one or more deuteriums until fully deuterated; preferably, R₁ is a C₁₋₆ alkyl, which is optionally substituted by one or more deuteriums until fully deuterated; and preferably, R₁ is a C₃₋₇ cycloalkyl.

6. The compound, or the pharmaceutically acceptable salt, enantiomer, diastereomer, racemate, solvate, hydrate, polymorph, prodrug or isotopic variant thereof according to any one of claims 1 to 5, wherein R₂ is selected from a C₁₋₆ alkyl, a C₁₋₆ haloalkyl, a C₂₋₆ alkenyl, a C₂₋₆ alkynyl, a C₃₋₇ cycloalkyl or a 3- to 7-membered heterocyclyl, and the group is optionally substituted with one or more deuteriums until fully deuterated; preferably, R₂ is selected from a C₁₋₆ alkyl, a C₁₋₆ haloalkyl or a C₃₋₇ cycloalkyl, and the group is optionally substituted with one or more deuteriums until fully deuterated; and preferably, R₂ is a C₁₋₆ alkyl, which is optionally substituted by one or more deuteriums until fully deuterated.

7. The compound, or the pharmaceutically acceptable salt, enantiomer, diastereomer, racemate, solvate, hydrate, polymorph, prodrug or isotopic variant thereof according to any one of claims 1 to 6, wherein R₃ is selected from a -C(O)-C₃₋₇ cycloalkyl or a 5- to 6-membered heteroaryl, and the group is optionally substituted with one or more deuteriums until fully deuterated; and preferably, R₃ is a -C(O)-C₃₋₇ cycloalkyl.

8. The compound, or the pharmaceutically acceptable salt, enantiomer, diastereomer, racemate, solvate, hydrate, polymorph, prodrug or isotopic variant thereof according to any one of claims 1 to 7, wherein R₄ is selected from a C₁₋₆ alkyl or a C₁₋₆ haloalkyl, and the group is optionally substituted with one or more deuteriums until fully deuterated; and preferably, R₄ is a C₁₋₆ alkyl, which is optionally substituted by one or more deuteriums until fully deuterated.

9. The compound, or the pharmaceutically acceptable salt, enantiomer, diastereomer, racemate, solvate, hydrate, polymorph, prodrug or isotopic variant thereof according to any one of claims 1 to 8, which has the following general formula: 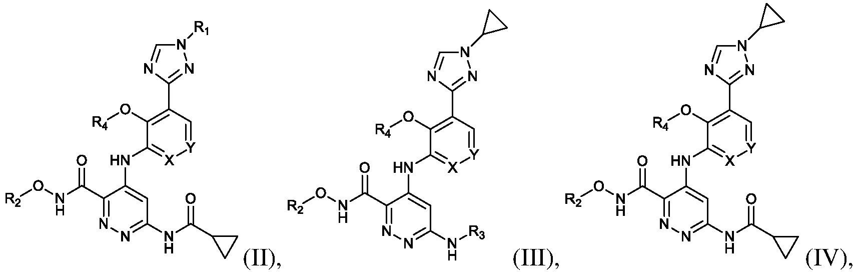 wherein each group is as defined in any one of claims 1 to 8.

10. The compound, or the pharmaceutically acceptable salt, enantiomer, diastereomer, racemate, solvate, hydrate, polymorph, prodrug or isotopic variant thereof according to claim 1, wherein the compound is selected from the following:

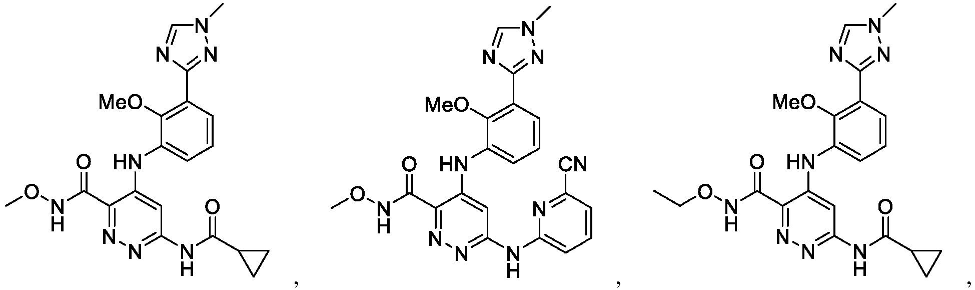

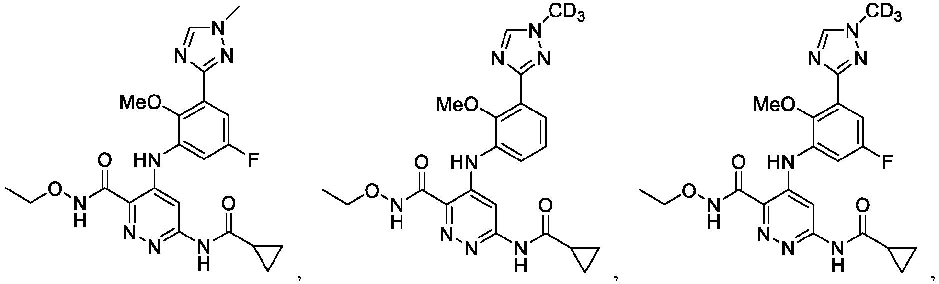

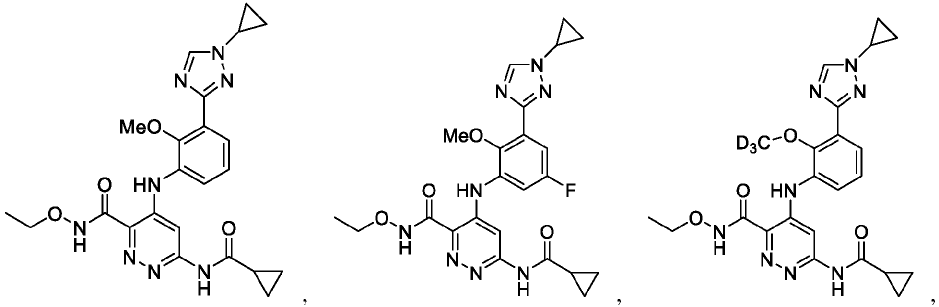

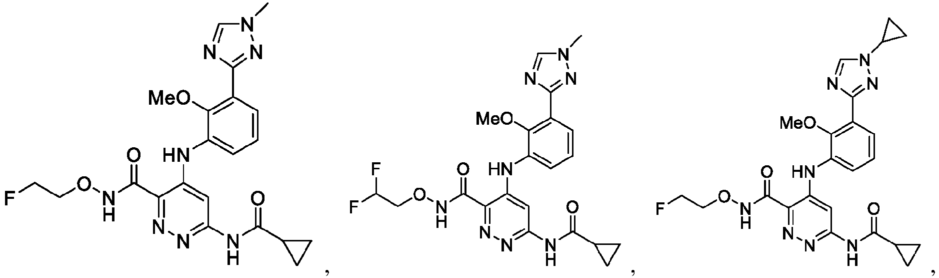

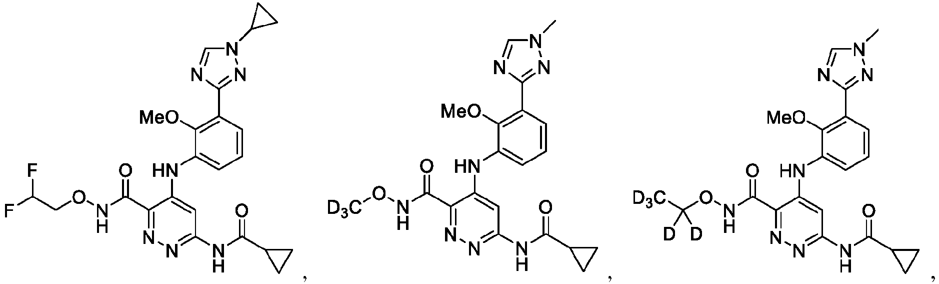

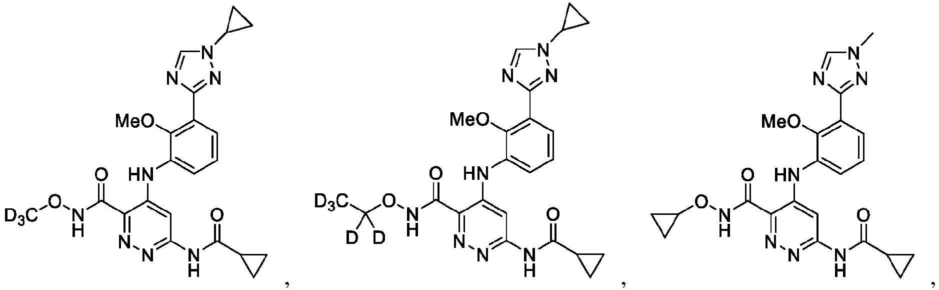

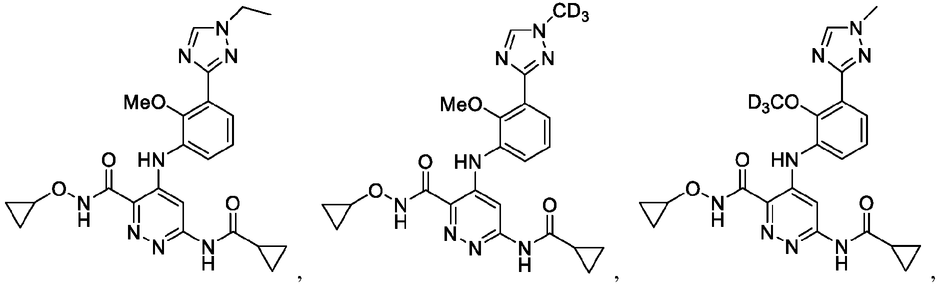

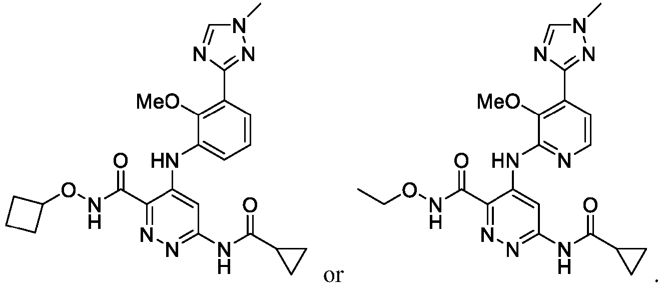

11. A pharmaceutical composition comprising the compound, or the pharmaceutically acceptable salt, enantiomer, diastereomer, racemate, solvate, hydrate, polymorph, prodrug or isotopic variant thereof according to any one of claims 1 to 10, and a mixture thereof, and a pharmaceutically acceptable excipient; wherein preferably, the pharmaceutical composition further comprises other therapeutic agents.

12. A use of the compound or the pharmaceutically acceptable salt, enantiomer, diastereomer, racemate, solvate, hydrate, polymorph, prodrug or isotopic variant thereof according to any one of claims 1 to 10, and a mixture thereof in the preparation of a medicament for treating and/or preventing a TYK2 kinase-mediated disease.

13. A use of the compound or the pharmaceutically acceptable salt, enantiomer, diastereomer, racemate, solvate, hydrate, polymorph, prodrug or isotopic variant thereof according to any one of claims 1 to 10, and a mixture thereof, or the pharmaceutical composition according to claim 11 in the treatment and/or prevention of a TYK2 kinase-mediated disease.

14. A method for treating and/or preventing a TYK2 kinase-mediated disease in a subject, comprising administering to the subject the compound or the pharmaceutically acceptable salt, enantiomer, diastereomer, racemate, solvate, hydrate, polymorph, prodrug or isotopic variant thereof according to any one of claims 1 to 10, and a mixture thereof, or the pharmaceutical composition according to claim 11.

15. The use according to claim 12 or the use of the compound or pharmaceutical composition according to claim 13 or the method according to claim 14, wherein the TYK2 kinase-mediated disease is selected from an autoimmune disease, a skin disease, an allergic disease, organ rejection, cancer, dry eye disease, myelofibrosis and polycythemia vera; preferably, the autoimmune disease is lupus, multiple sclerosis, rheumatoid arthritis, juvenile arthritis, psoriasis, ulcerative colitis, Crohn's disease or autoimmune thyroid disease; the skin disease is psoriasis, a rash or atopic dermatitis; the allergic disease is asthma or rhinitis; the organ transplant rejection is allograft rejection or graft-versus-host disease; and the cancer is renal cancer, liver cancer, pancreatic cancer, gastric cancer, breast cancer, prostate cancer, head and neck cancer, thyroid cancer, lung cancer, glioblastoma, melanoma, lymphoma or leukemia.

16. The use according to claim 12 or the use of the compound or pharmaceutical composition according to claim 13 or the method according to claim 14, wherein the TYK2 kinase-mediated disease is selected from rheumatoid arthritis, psoriasis, ulcerative colitis and Crohn's disease.
